(19) Europäisches Patentamt
European Patent Office
Office européen des brevets

(11) EP 3 505 181 B1

(12) **EUROPEAN PATENT SPECIFICATION**

(45) Date of publication and mention of the grant of the patent:
**07.07.2021 Bulletin 2021/27**

(51) Int Cl.:
***A61K 38/46*** *(2006.01)* ***C12N 9/16*** *(2006.01)*

(21) Application number: **18248241.4**

(22) Date of filing: **28.12.2018**

(54) **PREPARATION OF ENZYME REPLACEMENT THERAPY FOR MUCOPOLYSACCHARIDOSIS IIID**

HERSTELLUNG EINER ENZYMERSATZTHERAPIE FÜR MUCOPOLYSACCHARIDOSE IIID

PRÉPARATION DE THÉRAPIE DE REMPLACEMENT D'ENZYMES POUR MUCOPOLYSACCHARIDOSE IIID

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.12.2017 US 201762611472 P**
**05.04.2018 US 201815946505**

(43) Date of publication of application:
**03.07.2019 Bulletin 2019/27**

(73) Proprietors:
- **Los Angeles Biomedical Research Institute at Harbor-UCLA Medical Center Torrance, CA 90502 (US)**
- **Phoenix Nest Inc. Brooklyn NY 11215 (US)**

(72) Inventors:
- **DICKSON, Patricia Torrance, CA California 90502 (US)**
- **CHOU, Tsui-Fen Torrance, CA California 90502 (US)**
- **EKINS, Sean Brooklyn, NY New York 11215 (US)**
- **KAN, Shih-Hsin Torrance, CA California 90502 (US)**
- **LE, Steven Torrance, CA California 90502 (US)**
- **MOEN, Derek R. Torrance, CA California 90502 (US)**

(74) Representative: **J A Kemp LLP**
**14 South Square**
**Gray's Inn**
**London WC1R 5JJ (GB)**

(56) References cited:
**WO-A2-2017/062666**

- **CARLES ROCA ET AL: "Disease correction by AAV-mediated gene therapy in a new mouse model of mucopolysaccharidosis type IIID", HUMAN MOLECULAR GENETICS, vol. 26, no. 8, 15 April 2017 (2017-04-15) , pages 1535-1551, XP55583612, gb ISSN: 0964-6906, DOI: 10.1093/hmg/ddx058**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 505 181 B1

SEQUENCE LISTING

<110> LOS ANGELES BIOMEDICAL RESEARCH INSTITUTE AT HARBOR-UCLA MEDICAL CENTER PHOENIX NEST INC.

<120> PREPARATION OF ENZYME REPLACEMENT THERAPY FOR MUCOPOLYSACCHARIDOSIS IIID

<130> N415645EP

<150> US 62/611,472
<151> 2017-12-28

<150> US 15/946,505
<151> 2018-04-05

<160> 6

<170> PatentIn version 3.5

<210> 1
<211> 552
<212> PRT
<213> Artificial Sequence

<220>
<223> Synthetic

<400> 1

```
Met Arg Leu Leu Pro Leu Ala Pro Gly Arg Leu Arg Arg Gly Ser Pro
1               5                   10                  15

Arg His Leu Pro Ser Cys Ser Pro Ala Leu Leu Leu Leu Val Leu Gly
            20                  25                  30

Gly Cys Leu Gly Val Phe Gly Val Ala Ala Gly Thr Arg Arg Pro Asn
        35                  40                  45

Val Val Leu Leu Leu Thr Asp Asp Gln Asp Glu Val Leu Gly Gly Met
    50                  55                  60

Thr Pro Leu Lys Lys Thr Lys Ala Leu Ile Gly Glu Met Gly Met Thr
65                  70                  75                  80

Phe Ser Ser Ala Tyr Val Pro Ser Ala Leu Cys Cys Pro Ser Arg Ala
                85                  90                  95

Ser Ile Leu Thr Gly Lys Tyr Pro His Asn His His Val Val Asn Asn
            100                 105                 110

Thr Leu Glu Gly Asn Cys Ser Ser Lys Ser Trp Gln Lys Ile Gln Glu
        115                 120                 125
```

```
Pro Asn Thr Phe Pro Ala Ile Leu Arg Ser Met Cys Gly Tyr Gln Thr
    130                 135                 140

Phe Phe Ala Gly Lys Tyr Leu Asn Glu Tyr Gly Ala Pro Asp Ala Gly
145                 150                 155                 160

Gly Leu Glu His Val Pro Leu Gly Trp Ser Tyr Trp Tyr Ala Leu Glu
                165                 170                 175

Lys Asn Ser Lys Tyr Tyr Asn Tyr Thr Leu Ser Ile Asn Gly Lys Ala
            180                 185                 190

Arg Lys His Gly Glu Asn Tyr Ser Val Asp Tyr Leu Thr Asp Val Leu
        195                 200                 205

Ala Asn Val Ser Leu Asp Phe Leu Asp Tyr Lys Ser Asn Phe Glu Pro
    210                 215                 220

Phe Phe Met Met Ile Ala Thr Pro Ala Pro His Ser Pro Trp Thr Ala
225                 230                 235                 240

Ala Pro Gln Tyr Gln Lys Ala Phe Gln Asn Val Phe Ala Pro Arg Asn
                245                 250                 255

Lys Asn Phe Asn Ile His Gly Thr Asn Lys His Trp Leu Ile Arg Gln
            260                 265                 270

Ala Lys Thr Pro Met Thr Asn Ser Ser Ile Gln Phe Leu Asp Asn Ala
        275                 280                 285

Phe Arg Lys Arg Trp Gln Thr Leu Leu Ser Val Asp Asp Leu Val Glu
    290                 295                 300

Lys Leu Val Lys Arg Leu Glu Phe Thr Gly Glu Leu Asn Asn Thr Tyr
305                 310                 315                 320

Ile Phe Tyr Thr Ser Asp Asn Gly Tyr His Thr Gly Gln Phe Ser Leu
                325                 330                 335

Pro Ile Asp Lys Arg Gln Leu Tyr Glu Phe Asp Ile Lys Val Pro Leu
            340                 345                 350

Leu Val Arg Gly Pro Gly Ile Lys Pro Asn Gln Thr Ser Lys Met Leu
        355                 360                 365

Val Ala Asn Ile Asp Leu Gly Pro Thr Ile Leu Asp Ile Ala Gly Tyr
    370                 375                 380
```

```
Asp Leu Asn Lys Thr Gln Met Asp Gly Met Ser Leu Leu Pro Ile Leu
385                 390                 395                 400

Arg Gly Ala Ser Asn Leu Thr Trp Arg Ser Asp Val Leu Val Glu Tyr
                405                 410                 415

Gln Gly Glu Gly Arg Asn Val Thr Asp Pro Thr Cys Pro Ser Leu Ser
            420                 425                 430

Pro Gly Val Ser Gln Cys Phe Pro Asp Cys Val Cys Glu Asp Ala Tyr
        435                 440                 445

Asn Asn Thr Tyr Ala Cys Val Arg Thr Met Ser Ala Leu Trp Asn Leu
    450                 455                 460

Gln Tyr Cys Glu Phe Asp Asp Gln Glu Val Phe Val Glu Val Tyr Asn
465                 470                 475                 480

Leu Thr Ala Asp Pro Asp Gln Ile Thr Asn Ile Ala Lys Thr Ile Asp
                485                 490                 495

Pro Glu Leu Leu Gly Lys Met Asn Tyr Arg Leu Met Met Leu Gln Ser
            500                 505                 510

Cys Ser Gly Pro Thr Cys Arg Thr Pro Gly Val Phe Asp Pro Gly Tyr
        515                 520                 525

Arg Phe Asp Pro Arg Leu Met Phe Ser Asn Arg Gly Ser Val Arg Thr
    530                 535                 540

Arg Arg Phe Ser Lys His Leu Leu
545                 550
```

<210> 2
<211> 572
<212> PRT
<213> Artificial Sequence

<220>
<223> Synthetic

<400> 2

```
Met Arg Leu Leu Pro Leu Ala Pro Gly Arg Leu Arg Arg Gly Ser Pro
1               5                   10                  15

Arg His Leu Pro Ser Cys Ser Pro Ala Leu Leu Leu Leu Val Leu Gly
            20                  25                  30
```

```
Gly Cys Leu Gly Val Phe Gly Val Ala Ala Gly Thr Arg Arg Pro Asn
        35                  40                  45

Val Val Leu Leu Leu Thr Asp Asp Gln Asp Glu Val Leu Gly Gly Met
    50                  55                  60

Thr Pro Leu Lys Lys Thr Lys Ala Leu Ile Gly Glu Met Gly Met Thr
65                  70                  75                  80

Phe Ser Ser Ala Tyr Val Pro Ser Ala Leu Cys Cys Pro Ser Arg Ala
                85                  90                  95

Ser Ile Leu Thr Gly Lys Tyr Pro His Asn His His Val Val Asn Asn
            100                 105                 110

Thr Leu Glu Gly Asn Cys Ser Ser Lys Ser Trp Gln Lys Ile Gln Glu
        115                 120                 125

Pro Asn Thr Phe Pro Ala Ile Leu Arg Ser Met Cys Gly Tyr Gln Thr
    130                 135                 140

Phe Phe Ala Gly Lys Tyr Leu Asn Glu Tyr Gly Ala Pro Asp Ala Gly
145                 150                 155                 160

Gly Leu Glu His Val Pro Leu Gly Trp Ser Tyr Trp Tyr Ala Leu Glu
                165                 170                 175

Lys Asn Ser Lys Tyr Tyr Asn Tyr Thr Leu Ser Ile Asn Gly Lys Ala
            180                 185                 190

Arg Lys His Gly Glu Asn Tyr Ser Val Asp Tyr Leu Thr Asp Val Leu
        195                 200                 205

Ala Asn Val Ser Leu Asp Phe Leu Asp Tyr Lys Ser Asn Phe Glu Pro
    210                 215                 220

Phe Phe Met Met Ile Ala Thr Pro Ala Pro His Ser Pro Trp Thr Ala
225                 230                 235                 240

Ala Pro Gln Tyr Gln Lys Ala Phe Gln Asn Val Phe Ala Pro Arg Asn
                245                 250                 255

Lys Asn Phe Asn Ile His Gly Thr Asn Lys His Trp Leu Ile Arg Gln
            260                 265                 270

Ala Lys Thr Pro Met Thr Asn Ser Ser Ile Gln Phe Leu Asp Asn Ala
        275                 280                 285
```

```
Phe Arg Lys Arg Trp Gln Thr Leu Leu Ser Val Asp Asp Leu Val Glu
    290                 295                 300

Lys Leu Val Lys Arg Leu Glu Phe Thr Gly Glu Leu Asn Asn Thr Tyr
305                 310                 315                 320

Ile Phe Tyr Thr Ser Asp Asn Gly Tyr His Thr Gly Gln Phe Ser Leu
                325                 330                 335

Pro Ile Asp Lys Arg Gln Leu Tyr Glu Phe Asp Ile Lys Val Pro Leu
            340                 345                 350

Leu Val Arg Gly Pro Gly Ile Lys Pro Asn Gln Thr Ser Lys Met Leu
        355                 360                 365

Val Ala Asn Ile Asp Leu Gly Pro Thr Ile Leu Asp Ile Ala Gly Tyr
    370                 375                 380

Asp Leu Asn Lys Thr Gln Met Asp Gly Met Ser Leu Leu Pro Ile Leu
385                 390                 395                 400

Arg Gly Ala Ser Asn Leu Thr Trp Arg Ser Asp Val Leu Val Glu Tyr
                405                 410                 415

Gln Gly Glu Gly Arg Asn Val Thr Asp Pro Thr Cys Pro Ser Leu Ser
            420                 425                 430

Pro Gly Val Ser Gln Cys Phe Pro Asp Cys Val Cys Glu Asp Ala Tyr
        435                 440                 445

Asn Asn Thr Tyr Ala Cys Val Arg Thr Met Ser Ala Leu Trp Asn Leu
    450                 455                 460

Gln Tyr Cys Glu Phe Asp Asp Gln Glu Val Phe Val Glu Val Tyr Asn
465                 470                 475                 480

Leu Thr Ala Asp Pro Asp Gln Ile Thr Asn Ile Ala Lys Thr Ile Asp
                485                 490                 495

Pro Glu Leu Leu Gly Lys Met Asn Tyr Arg Leu Met Met Leu Gln Ser
            500                 505                 510

Cys Ser Gly Pro Thr Cys Arg Thr Pro Gly Val Phe Asp Pro Gly Tyr
        515                 520                 525

Arg Phe Asp Pro Arg Leu Met Phe Ser Asn Arg Gly Ser Val Arg Thr
    530                 535                 540
```

```
Arg Arg Phe Ser Lys His Leu Leu Gly Gly Gly Gly Ser Gly Gly Gly
545                 550                 555                 560

Gly Ser Glu Gln Lys Leu Ile Ser Glu Glu Asp Leu
                565                 570
```

<210> 3
<211> 1656
<212> DNA
<213> Artificial Sequence

<220>
<223> Synthetic

<400> 3

```
atgcgactgc tgcccctggc tcccggtaga ctgcgaagag gttcaccacg acatctgcca      60
agttgctccc ccgccctgct gctgctggtg ctgggcggat gcctgggagt ctttggagtg     120
gcagctggca cacggaggcc aaacgtggtc ctgctgctga ctgacgatca ggacgaggtg     180
ctgggggggta tgaccccccct gaagaaaaca aaggccctga tcggtgaaat gggcatgaca   240
ttcagctccg catacgtgcc tagcgccctg tgctgtccaa gccgggcttc cattctgact     300
gggaaatatc cacacaatca ccatgtggtc aacaataccc tggagggcaa ctgctctagt     360
aagtcttggc agaaaatcca ggaacctaat acatttccag ccattctgag gagtatgtgc     420
ggttaccaga ctttctttgc tggcaagtac ctgaacgagt atggagctcc agatgcagga     480
ggactggaac acgtgccact gggatggtcc tactggtatg ccctggagaa gaacagcaaa     540
tactataact acaccctgtc tatcaatgga aaggctagaa aacatgggga aaactactct     600
gtggactatc tgacagatgt cctggcaaat gtgagtctgg actttctgga ttacaagtca     660
aacttcgagc ccttctttat gatgattgcc actccagctc ctcactctcc ctggaccgca     720
gcaccacagt atcagaaggc tttccagaac gtgttcgcac ctaggaacaa aaatttcaac     780
atccacggaa ccaacaagca ttggctgatt agacaggcaa aaaccccaat gacaaattca     840
agcatccagt tcctggacaa cgcctttcga aagcggtggc agaccctgct gagcgtggac     900
gatctggtgg agaagctggt caaacgcctg gagtttacag gggaactgaa caacacttac     960
attttctata ccagcgataa cggataccat actgggcagt tttccctgcc catcgacaag    1020
cgacagctgt atgagttcga tatcaaggtg cccctgctgg tccggggacc agggatcaag    1080
cctaatcaga ctagtaaaat gctggtggct aacattgacc tggggcccac catcctggac    1140
attgcaggtt acgatctgaa caagacacag atggatggca tgtccctgct gcctatcctg    1200
cggggagcct caaatctgac ctggaggagc gacgtgctgg tcgagtatca gggtgaaggc    1260
cgcaacgtga ctgatccaac ctgcccctcc ctgtctccag gcgtgtcaca gtgttttcct    1320
```

```
gactgcgtct gtgaggatgc ctacaacaat acctatgctt gcgtgcgaac aatgagcgcc     1380
ctgtggaacc tgcagtactg tgagttcgac gatcaggagg tgtttgtcga agtgtataat     1440
ctgacagcag accctgatca gatcacaaac attgccaaga ctatcgaccc agagctgctg     1500
gggaaaatga attacagact gatgatgctg cagagttgct caggtcctac atgtcgcact     1560
ccaggcgtgt tcgaccccgg ctataggttt gatcctagac tgatgttctc taaccgcgga     1620
agtgtccgaa ccagacgctt ctccaagcat ctgctg                               1656
```

<210> 4
<211> 1656
<212> DNA
<213> Artificial Sequence

<220>
<223> Synthetic

<400> 4

atgcggctcc tgcctctagc cccaggtcgg ctccggcggg gcagcccccg ccacctgccc 60
tcctgcagcc cagcgctgct actgctggtg ctgggcggct gcctgggggt cttcggggtg 120
gctgcgggaa cccggaggcc caacgtggtg ctgctcctca cggacgacca ggacgaagtg 180
ctcggcggca tgacaccgct aaagaaaacc aaagctctca tcggagagat ggggatgact 240
ttttccagtg cttatgtgcc aagtgctctc tgctgcccca gcagagccag tatcctgaca 300
ggaaagtacc cacataatca tcacgttgtg aacaacactc tggaggggaa ctgcagtagt 360
aagtcctggc agaagatcca agaaccaaat actttcccag caattctcag atcaatgtgt 420
ggttatcaga cctttttgc agggaaatat ttaaatgagt acggagcccc agatgcaggt 480
ggactagaac acgttcctct gggttggagt tactggtatg ccttggaaaa gaattctaag 540
tattataatt acaccctgtc tatcaatggg aaggcacgga agcatggtga aaactatagt 600
gtggactacc tgacagatgt tttggctaat gtctccttgg actttctgga ctacaagtcc 660
aactttgagc ccttcttcat gatgatcgcc actccagcgc ctcattcgcc ttggacagct 720
gcacctcagt accagaaggc tttccagaat gtctttgcac caagaaacaa gaacttcaac 780
atccatggaa cgaacaagca ctggttaatt aggcaagcca agactccaat gactaattct 840
tcaatacagt ttttagataa tgcatttagg aaaaggtggc aaactctcct ctcagttgat 900
gaccttgtgg agaaactggt caagaggctg gagttcactg gggagctcaa caacacttac 960
atcttctata cctcagacaa tggctatcac acaggacagt tttccttgcc aatagacaag 1020
agacagctgt atgagtttga tatcaaagtt ccactgttgg ttcgaggacc tgggatcaaa 1080
ccaaatcaga caagcaagat gctggttgcc aacattgact tgggtcctac tattttggac 1140
attgctggct acgacctaaa taagacacag atggatggga tgtccttatt gcccattttg 1200
agaggtgcca gtaacttgac ctggcgatca gatgtcctgg tggaatacca aggagaaggc 1260

cgtaacgtca ctgacccaac atgcccttcc ctgagtcctg gcgtatctca atgcttccca 1320
gactgtgtat gtgaagatgc ttataacaat acctatgcct gtgtgaggac aatgtcagca 1380
ttgtggaatt tgcagtattg cgagtttgat gaccaggagg tgtttgtaga agtctataat 1440
ctgactgcag acccagacca gatcactaac attgctaaaa ccatagaccc agagctttta 1500
ggaaagatga actatcggtt aatgatgtta cagtcctgtt ctgggccaac ctgtcgcact 1560
ccaggggttt ttgaccccgg atacaggttt gacccccgtc tcatgttcag caatcgcggc 1620
agtgtcagga ctcgaagatt ttccaaacat cttctg 1656

<210> 5
<211> 579
<212> PRT
<213> Artificial Sequence

<220>
<223> Synthetic

<400> 5

```
Met Arg Leu Leu Pro Leu Ala Pro Gly Arg Leu Arg Arg Gly Ser Pro
1               5                   10                  15

Arg His Leu Pro Ser Cys Ser Pro Ala Leu Leu Leu Leu Val Leu Gly
            20                  25                  30

Gly Cys Leu Gly Val Phe Gly Val Ala Ala Gly Thr Arg Arg Pro Asn
        35                  40                  45

Val Val Leu Leu Leu Thr Asp Asp Gln Asp Glu Val Leu Gly Gly Met
    50                  55                  60

Thr Pro Leu Lys Lys Thr Lys Ala Leu Ile Gly Glu Met Gly Met Thr
65                  70                  75                  80

Phe Ser Ser Ala Tyr Val Pro Ser Ala Leu Cys Cys Pro Ser Arg Ala
                85                  90                  95

Ser Ile Leu Thr Gly Lys Tyr Pro His Asn His His Val Val Asn Asn
            100                 105                 110

Thr Leu Glu Gly Asn Cys Ser Ser Lys Ser Trp Gln Lys Ile Gln Glu
        115                 120                 125

Pro Asn Thr Phe Pro Ala Ile Leu Arg Ser Met Cys Gly Tyr Gln Thr
    130                 135                 140

Phe Phe Ala Gly Lys Tyr Leu Asn Glu Tyr Gly Ala Pro Asp Ala Gly
```

145 150 155 160

Gly Leu Glu His Val Pro Leu Gly Trp Ser Tyr Trp Tyr Ala Leu Glu
165 170 175

Lys Asn Ser Lys Tyr Tyr Asn Tyr Thr Leu Ser Ile Asn Gly Lys Ala
180 185 190

Arg Lys His Gly Glu Asn Tyr Ser Val Asp Tyr Leu Thr Asp Val Leu
195 200 205

Ala Asn Val Ser Leu Asp Phe Leu Asp Tyr Lys Ser Asn Phe Glu Pro
210 215 220

Phe Phe Met Met Ile Ala Thr Pro Ala Pro His Ser Pro Trp Thr Ala
225 230 235 240

Ala Pro Gln Tyr Gln Lys Ala Phe Gln Asn Val Phe Ala Pro Arg Asn
245 250 255

Lys Asn Phe Asn Ile His Gly Thr Asn Lys His Trp Leu Ile Arg Gln
260 265 270

Ala Lys Thr Pro Met Thr Asn Ser Ser Ile Gln Phe Leu Asp Asn Ala
275 280 285

Phe Arg Lys Arg Trp Gln Thr Leu Leu Ser Val Asp Asp Leu Val Glu
290 295 300

Lys Leu Val Lys Arg Leu Glu Phe Thr Gly Glu Leu Asn Asn Thr Tyr
305 310 315 320

Ile Phe Tyr Thr Ser Asp Asn Gly Tyr His Thr Gly Gln Phe Ser Leu
325 330 335

Pro Ile Asp Lys Arg Gln Leu Tyr Glu Phe Asp Ile Lys Val Pro Leu
340 345 350

Leu Val Arg Gly Pro Gly Ile Lys Pro Asn Gln Thr Ser Lys Met Leu
355 360 365

Val Ala Asn Ile Asp Leu Gly Pro Thr Ile Leu Asp Ile Ala Gly Tyr
370 375 380

Asp Leu Asn Lys Thr Gln Met Asp Gly Met Ser Leu Leu Pro Ile Leu
385 390 395 400

```
Arg Gly Ala Ser Asn Leu Thr Trp Arg Ser Asp Val Leu Val Glu Tyr
                405                 410                 415

Gln Gly Glu Gly Arg Asn Val Thr Asp Pro Thr Cys Pro Ser Leu Ser
            420                 425                 430

Pro Gly Val Ser Gln Cys Phe Pro Asp Cys Val Cys Glu Asp Ala Tyr
        435                 440                 445

Asn Asn Thr Tyr Ala Cys Val Arg Thr Met Ser Ala Leu Trp Asn Leu
    450                 455                 460

Gln Tyr Cys Glu Phe Asp Asp Gln Glu Val Phe Val Glu Val Tyr Asn
465                 470                 475                 480

Leu Thr Ala Asp Pro Asp Gln Ile Thr Asn Ile Ala Lys Thr Ile Asp
                485                 490                 495

Pro Glu Leu Leu Gly Lys Met Asn Tyr Arg Leu Met Met Leu Gln Ser
            500                 505                 510

Cys Ser Gly Pro Thr Cys Arg Thr Pro Gly Val Phe Asp Pro Gly Tyr
        515                 520                 525

Arg Phe Asp Pro Arg Leu Met Phe Ser Asn Arg Gly Ser Val Arg Thr
    530                 535                 540

Arg Arg Phe Ser Lys His Leu Leu Gly Gly Glu Asn Leu Tyr Phe Gln
545                 550                 555                 560

Gly Gly Gly Ser Gly Gly Gly Gly Ser Glu Gln Lys Leu Ile Ser Glu
                565                 570                 575

Glu Asp Leu
```

<210> 6
<211> 560
<212> PRT
<213> Artificial Sequence

<220>
<223> Synthetic

<400> 6

```
Met Arg Leu Leu Pro Leu Ala Pro Gly Arg Leu Arg Arg Gly Ser Pro
1               5                   10                  15

Arg His Leu Pro Ser Cys Ser Pro Ala Leu Leu Leu Leu Val Leu Gly
```

```
            20                  25                  30

Gly Cys Leu Gly Val Phe Gly Val Ala Ala Gly Thr Arg Arg Pro Asn
        35                  40                  45

Val Val Leu Leu Leu Thr Asp Asp Gln Asp Glu Val Leu Gly Gly Met
    50                  55                  60

Thr Pro Leu Lys Lys Thr Lys Ala Leu Ile Gly Glu Met Gly Met Thr
65                  70                  75                  80

Phe Ser Ser Ala Tyr Val Pro Ser Ala Leu Cys Cys Pro Ser Arg Ala
                85                  90                  95

Ser Ile Leu Thr Gly Lys Tyr Pro His Asn His His Val Val Asn Asn
            100                 105                 110

Thr Leu Glu Gly Asn Cys Ser Ser Lys Ser Trp Gln Lys Ile Gln Glu
        115                 120                 125

Pro Asn Thr Phe Pro Ala Ile Leu Arg Ser Met Cys Gly Tyr Gln Thr
    130                 135                 140

Phe Phe Ala Gly Lys Tyr Leu Asn Glu Tyr Gly Ala Pro Asp Ala Gly
145                 150                 155                 160

Gly Leu Glu His Val Pro Leu Gly Trp Ser Tyr Trp Tyr Ala Leu Glu
                165                 170                 175

Lys Asn Ser Lys Tyr Tyr Asn Tyr Thr Leu Ser Ile Asn Gly Lys Ala
            180                 185                 190

Arg Lys His Gly Glu Asn Tyr Ser Val Asp Tyr Leu Thr Asp Val Leu
        195                 200                 205

Ala Asn Val Ser Leu Asp Phe Leu Asp Tyr Lys Ser Asn Phe Glu Pro
    210                 215                 220

Phe Phe Met Met Ile Ala Thr Pro Ala Pro His Ser Pro Trp Thr Ala
225                 230                 235                 240

Ala Pro Gln Tyr Gln Lys Ala Phe Gln Asn Val Phe Ala Pro Arg Asn
                245                 250                 255

Lys Asn Phe Asn Ile His Gly Thr Asn Lys His Trp Leu Ile Arg Gln
            260                 265                 270
```

```
Ala Lys Thr Pro Met Thr Asn Ser Ser Ile Gln Phe Leu Asp Asn Ala
        275                 280                 285

Phe Arg Lys Arg Trp Gln Thr Leu Leu Ser Val Asp Asp Leu Val Glu
    290                 295                 300

Lys Leu Val Lys Arg Leu Glu Phe Thr Gly Glu Leu Asn Asn Thr Tyr
305                 310                 315                 320

Ile Phe Tyr Thr Ser Asp Asn Gly Tyr His Thr Gly Gln Phe Ser Leu
                325                 330                 335

Pro Ile Asp Lys Arg Gln Leu Tyr Glu Phe Asp Ile Lys Val Pro Leu
            340                 345                 350

Leu Val Arg Gly Pro Gly Ile Lys Pro Asn Gln Thr Ser Lys Met Leu
        355                 360                 365

Val Ala Asn Ile Asp Leu Gly Pro Thr Ile Leu Asp Ile Ala Gly Tyr
    370                 375                 380

Asp Leu Asn Lys Thr Gln Met Asp Gly Met Ser Leu Leu Pro Ile Leu
385                 390                 395                 400

Arg Gly Ala Ser Asn Leu Thr Trp Arg Ser Asp Val Leu Val Glu Tyr
                405                 410                 415

Gln Gly Glu Gly Arg Asn Val Thr Asp Pro Thr Cys Pro Ser Leu Ser
            420                 425                 430

Pro Gly Val Ser Gln Cys Phe Pro Asp Cys Val Cys Glu Asp Ala Tyr
        435                 440                 445

Asn Asn Thr Tyr Ala Cys Val Arg Thr Met Ser Ala Leu Trp Asn Leu
    450                 455                 460

Gln Tyr Cys Glu Phe Asp Asp Gln Glu Val Phe Val Glu Val Tyr Asn
465                 470                 475                 480

Leu Thr Ala Asp Pro Asp Gln Ile Thr Asn Ile Ala Lys Thr Ile Asp
                485                 490                 495

Pro Glu Leu Leu Gly Lys Met Asn Tyr Arg Leu Met Met Leu Gln Ser
            500                 505                 510

Cys Ser Gly Pro Thr Cys Arg Thr Pro Gly Val Phe Asp Pro Gly Tyr
        515                 520                 525
```

```
Arg Phe Asp Pro Arg Leu Met Phe Ser Asn Arg Gly Ser Val Arg Thr
    530                 535                 540

Arg Arg Phe Ser Lys His Leu Leu Gly Gly Glu Asn Leu Tyr Phe Gln
545                 550                 555                 560
```

## Description

This invention was made with government support under 1R41NS89061-01, 2R42NS089061-02, and 2R44NS089061-04 awarded by National Institute of Neurological Disorders and Stroke at National Institute of Health. The government has certain rights in the invention.

## BACKGROUND

Sanfilippo disease (mucopolysaccharidosis type III; MPS III) is a devastating neurodegenerative lysosomal storage disorder of childhood. Babies appear normal at birth, learn to walk and talk, but then gradually, progressively, deteriorate to a vegetative state over the span of 10 or 20 years. The central pathologic features of MPS III are neurologic: there is a slowing of development, severe behavioral problems, progressive cognitive decline, dementia, and decline in motor skills that steadily lead to immobility, unresponsiveness, and death.

The fundamental cause of MPS III is an inherited mutation in one of the 4 enzymes required to catabolize heparan sulfate (HS), a glycosaminoglycan which plays important structural and functional roles in the brain and elsewhere. Each type of MPS III (A to D) is due to deficiency of a different enzyme in the HS breakdown pathway. Because MPS III is rare and affects the brain (which is difficult to treat), motivation for pharmaceutical and biotechnology companies to develop new therapies has been limited.

There is no cure or effective treatment available for MPS IIID, and there is therefore an unmet need for developing such a treatment.

## SUMMARY

Thus, the present invention provides a method for preparing a composition suitable for treating mucopolysaccharidosis type IIID (MPS IIID) in a human patient in need thereof, comprising testing the sulfatase activity of a recombinant polypeptide in a testing buffer comprising less than 500 mM sodium, less than 100 mM citrate, less than 100 mM phosphate, less than 100 mM sulfate, and at least 0.005% Triton X-100, and providing the recombinant polypeptide in an artificial cerebrospinal fluid to prepare the composition, wherein the recombinant polypeptide comprises the amino acid sequence of SEQ ID NO: 1 or an amino acid sequence (a) having at least 95% sequence identity to SEQ ID NO: 1 and (b) having the enzymatic activity of human acetylglucosamine-6-sulfatase (GNS).

The present invention further provides a method of testing the sulfatase activity of a recombinant polypeptide, comprising reacting the recombinant polypeptide with para-nitrocatechol sulfate (PNCS) in a testing buffer comprising less than 500 mM sodium, less than 100 mM citrate, less than 100 mM phosphate, less than 100 mM sulfate, and at least 0.005% Triton X-100, wherein the recombinant polypeptide comprises the amino acid sequence of SEQ ID NO: 1 or an amino acid sequence (a) having at least 95% sequence identity to SEQ ID NO: 1 and (b) having the enzymatic activity of human acetylglucosamine-6-sulfatase (GNS).

The method of testing the sulfatase activity of a recombinant peptide may further comprise stopping the reaction with a base. The method of testing the sulfatase activity of a recombinant peptide may further comprise stopping the reaction with a phosphate-citrate buffer. The phosphate-citrate buffer may comprise at least 0.4 M $Na_2HPO_4$ in at least 0.2 M citric acid.

In any of the methods of the present invention described herein, the testing buffer may have a pH of about 5 to about 6. In any of the methods of the present invention described herein, the testing buffer may comprise less than 250 mM sodium, less than 50 mM citrate, less than 50 mM phosphate, less than 50 mM sulfate, and/or at least 0.01% Triton X-100. In any of the methods of the present invention described herein, the testing buffer may comprise about 200 mM sodium acetate and about 0.01% Triton X-100, at about pH 5.6. In any of the methods of the present invention described herein, the testing buffer may have a pH of about 5.6, comprise about 200 mM sodium acetate, and/or comprise about 0.01% Triton X-100.

## BRIEF DESCRIPTION OF THE DRAWINGS

**FIG. 1** shows an example amino acid sequence (SEQ ID NO: 1) useful for treating MPS IIID.

**FIG. 2A** shows the sequence of SEQ ID NO: 2 which, as compared to SEQ ID NO: 1, further includes a glycine-serine linker (underlined) and a c-myc tag (bold and italic) for protein purification.

**FIG. 2B** shows the sequence of SEQ ID NO: 5 which, as compared to SEQ ID NO: 2, further includes a cleavage site (bold and underlined) recognizable by the Tobacco Etch Virus (TEV) protease which is useful for removing the c-myc tag.

**FIG. 2C** shows the sequence of SEQ ID NO: 6, after the c-myc tag is removed from SEQ ID NO: 5 by a (TEV) protease.

**FIG. 3** shows an illustrative cDNA sequence (SEQ ID NO: 3) for encoding a recombinant GNS protein of the present disclosure.

**FIG. 4** shows the wild-type human cDNA sequence (SEQ ID NO: 4) for GNS.

**FIG. 5** presents a sequence alignment between SEQ ID NO: 3 and 4.

**FIG. 6A-D** show that the purified recombinant human alpha-*N*-acetylglucosamine-6-sulfatase (rhGNS) was heavily glycosylated and enzymatically active. **A**) Western blot of rhGNS purification using antibodies against GNS and against the purification tag, myc. Non-clinical-grade rhGNS purchased from R&D systems and alpha-N-acetylglucosaminidase (NAGLU) produced from CHO cells were used as positive controls. **B**) PNGase F and Endo H treatment of purified rhGNS results in a shift in molecular weight, demonstrating that the protein is glycosylated. **C**) Michaelis-Menten Curves of rhGNS. Enzymatic activity of rhGNS was assayed using a fluorogenic substrate (4-MU-GNS) with a 4h second step (squares) vs. 24h second step (circles). $K_m$ was 3.97 mM and $V_{max}$ was 336,359 nmol/24h with the shorter assay. **D**) pH profile of rhGNS activity. Optimal assay conditions occurred within acidic pH range (4-6). Means and S.D. of triplicate experiments.

**FIG. 7A-D** show rhGNS entered human MPS IIID cells, targeted to lysosomes, and reduced GAG storage. **A**) Confocal microscopy of rhGNS uptake into MPS IIID human fibroblasts. Blue: DAPI, Green: rhGNS (anti-myc), Red: Lysotracker. Top row: treated with rhGNS. Bottom row, no rhGNS applied. **B**) rhGNS intracellular uptake and inhibition assay. Cell lysates from MPS IIID human fibroblasts were assayed for GNS activity following 4 hour treatment with rhGNS with or without 5mM mannose-6-phosphate (M6P). C) Heparan sulfate GAG reduction in MPS IIID human fibroblasts treated with 150 ng/ml rhGNS for 72h at 37°C or untreated. Shown is a representative experiment from triplicate experiments. Means and S.D. of triplicate assays. Two wild-type (WT) fibroblast lines are shown as controls. **D**) Radiolabeled GAG accumulation measured at different concentrations of purified rhGNS (0-250 pM) demonstrated an exponential decrease in storage, with storage reduced by half ($EC_{50}$) at 5.5 pM. Radiolabeled GAGs were extracted and measured via scintillation counting, and radioactive counts per minute were normalized to protein concentration. $EC_{50}$ was calculated using exponential decay with a bottom of 40% (equal to WT levels). Means and S.D. of triplicate assays.

**FIG. 8** presents a chart showing that rhGNS is active at body temperature (activity vs temperature, normalized to activity at 24°C). Means and S.D. of triplicate experiments. Each point was assayed in duplicate.

**FIG. 9** demonstrates rhGNS stability in artificial cerebrospinal fluid. Activity is normalized to activity at day=0. Means and S.D. of triplicate experiments. Each point was assayed in duplicate.

**FIG. 10A-B** show the GNS enzyme activity (nmol/hr/mg) in MPS IIID mice or control mice receiving different treatments.

**FIG. 11A and 11B** show the activities of two lysosomal enzymes, alpha-N-acetylglucosaminidase (NAGLU) and β-hexoaminidase (HEX) one day after the MPS IIID or control mice receiving different treatments.

**FIG. 12. Expression and glycosylation of rhGNS.** A) Western blots of rhGNS purification, using antibodies against GNS and c-myc tag. Lanes: Harvested PF CHO Media (Before), media following concentration and buffer exchange (Conc), unbound concentrated media following incubation with c-myc affinity beads (Unbound), c-myc peptide elution of beads (Elution). An enrichment of rhGNS product was observed in the final elution. We purified alpha-N-acetylglucosaminidase (NAGLU) c-myc fusion produced to be used for the GNS activity assay; B) Purity of purified rhGNS and rhNAGLU was shown by SDS-PAGE; C) Purified rhGNS (lane 1) was denatured by glycosidase PNGase F (lane 2) and Endo H (lane 3). A shift in molecular weight from 80 kDa to 58 kDa demonstrated that rhGNS was heavily glycosylated with N-linked mannose residues. (D) Composition analysis of N-glycan isolated from rhGNS sample using HPAEC-PAD analysis. Experimental: 100ug of protein was used for N-glycan isolation using PNGaseF, followed by purification of N-glycans using SPE extraction. Purified N-glycan was hydrolyzed using 2N TFA at 100degC for 4h, acid removed by dry nitrogen flush and finally dissolved in milli-Q water and injected on HPAEC-PAD. Carbo-Pac PA-1 column (4 x 250mm) was used with NaOH-NaOAc gradient as mobile phase for separation. A standard Man-6-P was used to quantify and assign the retention time. Result: The amount of Man-6-P present in 100ug of rhGNS is 0.87ug.

**FIG. 13: Biochemical characterization of purified rhGNS.** A) rhGNS activity towards the fluorogenic substrate 4-MUGNS was measured at increasing substrate concentrations, demonstrating Michaelis-Menten steady-state kinetics with a $k_{cat}$ value of 14,000 nmol/hr/mg and $K_M$ of 4 mM. B) To assess the optimal pH range for the enzymatic activity of GNS, the GNS Assay was performed from pH 3.8-5.8 (Acetate/Acetic Acid Buffer) and 6.2-7.6 (HEPES Buffer) at 37°C for 1 hour. C) Purified rhGNS activity was assessed at different temperatures, exhibiting an increasing of rhGNS enzyme activity with the temperature up to 49 °C, also demonstrating its thermostability at higher temperature up to 65 °C without decrease its enzyme activity. Data is normalized to 24 °C activity. D) Purified rhGNS was stored in artificial CSF at 4°C and activity was measured under standard conditions for 1 hour over a 28-day period; over 80% residual activity remained when normalized to day 0. Means (black circle) and standard deviation of three independent experiments are shown for each Figure.

**FIG. 14: rhGNS enters and reduces GAG accumulation in human MPS IIID fibroblasts.** A) Cellular uptake of rhGNS *in vitro.* IIID fibroblasts GM17495 (Coriell) were seeded in 6-well plates containing at 250,000 cells/well in 2.5 ml culture medium. Purified GNS was applied and incubated with cells for 4h at 37°C. The intracellular enzyme activity of rhGNS increased at the presence of higher amount of rhGNS applied. This cellular uptake of rhGNS is almost abolished at the presence of 5 mM M6P suggesting the cell entry of rhGNS is likely via M6P receptor. B) Two human MPS IIID fibroblasts (GM05093 and GM14795) and two normal human fibroblasts (IMR90 and GM1392) were labeled with 25 μCi/ml $H_2{}^{15}SO_4$ in culture medium without serum for 72 hours at 37°C. In the presence of 250 pM purified rhGNS (2$^{nd}$ and 4$^{th}$ bars from left-hand side), the elevated GAG accumulation (1$^{st}$ and 3$^{rd}$ bars from left-hand side) reduced to normal (WT) levels (white bars; 5$^{th}$ and 6$^{th}$ from left-hand side). Shown is a representative experiment from triplicate experiments. C) Radio-labeled GAG accumulation measured at different concentrations of purified rhGNS (0-250 pM) demonstrated an exponential decrease in storage, with storage reduced by half ($EC_{50}$) at 5.5 pM. Radio-labeled GAGs were extracted and measured via scintillation counting, and radioactive counts per minute were normalized to protein concentration. $EC_{50}$ was calculated using exponential decay with a bottom of 40% (equal to WT levels). Means and S.D. of triplicate assays.

**FIG. 15: rhGNS treated (Gns-/-) mice show increased levels of GNS and decreased hexosamidase.** A) A single dose of 5.3 μg rhGNS administered into the lateral cerebral ventricle at PND 2 of Gns-/- mice under cryo-anesthesia. B) GNS activity in mouse brain lysate after 1 day treatment, C) β -hexosaminidase activity in mouse brain lysate after 3 day treatment and D) C) β -hexosaminidase activity in mouse brain lysate after 7 day treatment. E) β -hexosaminidase activity in mouse brain lysate after 14 day treatment. [GNS+/- (n=4), GNS-/- VEH (vehicle treated, n=5), GNS-/- EZN (rhGNS treatd, n=5).]

**FIG. 16: rhGNS treated (Gns-/-) mice show increased levels of GNS across all sections of the brain.** A) Schematic of neonatal mouse brain with site of injection. rhGNS distribution in 3 sections of the brain as determined by GNS activity in mouse brain lysate after 1 day treatment in 3 sections. B). Section 1; C). Section 2; and D). Section 3. [GNS+/- (n=6), GNS-/- VEH (vehicle treated, n=5), GNS-/- EZN (rhGNS treatd, n=6).

**FIG. 17: Estimated half-life of rhGNS in rhGNS treated (Gns-/-) mice brain.** A single dose of 15.0 μg rhGNS administered into the lateral cerebral ventricle at PND 2 of Gns-/- mice under Cryo-anesthesia. GNS activity in mouse brain lysate after A) 1 day, B) 2 day, C) 3 day treatment. D) Decay of GNS activity is plotted to estimate the half-life of rhGNS in mice brain to be about 1.1 day.

**FIG. 18. rhGNS treated (Gns-/-) mice demonstrate delivery of rhGNS to lysosomes in the mice brain.** A single dose of 15.0 μg rhGNS administered into the lateral cerebral ventricle of 2 day old mice and lysosomes were purified one day post injection of rhGNS using the Lysosome Enrichment Kit for Tissue and Cultured Cells (Thermo). We compare GNS activity (A), β-hexosaminidase activity (B), and LAMP1 level using western blot (C) for the crude lysate and enrich lysosome.

**FIG. 19.** rhGNS secretion into the media was monitored using the GNS assay and western blot daily until full-length GNS expression reached a maximum (day 10-15), at which point the conditioned medium was harvested for rhGNS purification.

**FIG. 20. Expression of rhGNS in individual CHO clone.** Conditioned PF CHO media from isolated rhGNS clones 7 days after confluentce were measured using the GNS assay, and 3 high expression clones (TCB469 B2, TCB470 A1, and TCB470 A5) were identified.

**FIG. 21. Native PAGE of rhGNS.** Purified rhGNS was resolved in a non-denaturing Tris/Bis gel by electrophoresis, exhibiting an estimated molecular weight of 160-200 kDa, suggesting that it forms either a homodimer or trimer conformation.

**FIG. 22. pH Profile of rhGNS using the Sulfatase Assay.** Both the commercially available and our own product demonstrated a pH optimum of approximately 5.6 against the substrate PNCS with overlapping pH profiles.

**FIG. 23. Sulfatase Assay Optimization.** A) Increasing amounts of NaCl lead to decreased rhGNS activity at concentrations greater than 250 mM in a solution containing 200 mM acetate buffer with 0.25% Triton X-100 (pH 5.0). B) The presence of 50 mM citrate or phosphate in the reaction buffer solution completely inhibited rhGNS activity (pH 5.0). C) Acetate buffer concentration did not have a significant effect on overall activity in a solution containing 50 mM NaCl and 0.25% Triton X-100. D) Addition of Triton X-100 increased reproducibility as well as activity with maximum rhGNS activity observed at low levels of the detergent (0.01%).

## DETAILED DESCRIPTION

### I. Definitions

All numerical designations, e.g., pH, temperature, time, concentration, and molecular weight, including ranges, are approximations which are varied (+) or (-) by increments of 0.1. It is to be understood, although not always explicitly stated that all numerical designations are preceded by the term "about". It also is to be understood, although not always explicitly stated, that the reagents described herein are merely exemplary and that equivalents of such are known in the art.

As used in the specification and claims, the singular form "a", "an" and "the" include plural references unless the context clearly dictates otherwise. For example, the term "a pharmaceutically acceptable carrier" includes a plurality of pharmaceutically acceptable carriers, including mixtures thereof.

As used herein, the term "comprising" is intended to mean that the compositions and methods include the recited elements, but do not exclude others. "Consisting essentially of' when used to define compositions and methods, shall mean excluding other elements of any essential significance to the combination for the intended use. Thus, a composition consisting essentially of the elements as defined herein would not exclude trace contaminants from the isolation and purification method and pharmaceutically acceptable carriers, such as phosphate buffered saline, preservatives, and the like. "Consisting of' shall mean excluding more than trace elements of other ingredients and substantial method steps for administering the compositions of this disclosure. Aspects defined by each of these transition terms are within the scope of this disclosure.

The term "protein" and "polypeptide" are used interchangeably and in their broadest sense to refer to a compound of two or more subunit amino acids, amino acid analogs or peptidomimetics. The subunits may be linked by peptide bonds. In another aspects, the subunit may be linked by other bonds, e.g., ester, ether, etc. A protein or peptide must contain at least two amino acids and no limitation is placed on the maximum number of amino acids which may comprise a protein's or peptide's sequence. As used herein the term "amino acid" refers to either natural and/or unnatural or synthetic amino acids, including glycine and both the D and L optical isomers, amino acid analogs and peptidomimetics. Single letter and three letter abbreviations of the naturally occurring amino acids are listed below. A peptide of three or more amino acids is commonly called an oligopeptide if the peptide chain is short. If the peptide chain is long, the peptide is commonly called a polypeptide or a protein.

A "pharmaceutical composition" is intended to include the combination of an active agent with a carrier, inert or active, making the composition suitable for diagnostic or therapeutic use *in vitro, in vivo* or *ex vivo.*

"An effective amount" refers to the amount of derivative sufficient to induce a desired biological and/or therapeutic result. That result can be alleviation of the signs, symptoms, or causes of a disease, or any other desired alteration of a biological system. The effective amount will vary depending upon the specific recombinant GNS protein used, the dosing regimen of the recombinant GNS protein, timing of administration of the recombinant GNS protein, the subject and disease condition being treated, the weight and age of the subject, the severity of the disease condition, the manner of administration and the like, all of which can be determined readily by one of ordinary skill in the art.

As used herein, the terms "treating," "treatment" and the like are used herein to mean obtaining a desired pharmacologic and/or physiologic effect. The effect may be prophylactic in terms of completely or partially preventing a disorder or sign or symptom thereof, and/or may be therapeutic in terms of a partial or complete cure for a disorder and/or adverse effect attributable to the disorder.

"Treating" also covers any treatment of a disorder in a mammal, and includes: (a) preventing a disorder from occurring in a subject that may be predisposed to a disorder, but may have not yet been diagnosed as having it, e.g., prevent MPS IIID symptoms in a patient with the genetic features of the MPS IIID disease.

As used herein, to "treat" further includes systemic amelioration of the symptoms associated with the pathology and/or a delay in onset of symptoms. Clinical and sub-clinical evidence of "treatment" will vary with the pathology, the individual and the treatment.

"Administration" can be effected in one dose, continuously or intermittently throughout the course of treatment. Methods of determining the most effective means and dosage of administration are known to those of skill in the art and will vary with the composition used for therapy, the purpose of the therapy, the target cell being treated, and the subject being treated. Single or multiple administrations can be carried out with the dose level and pattern being selected by the treating physician. Suitable dosage formulations and methods of administering the agents are known in the art. A "subject" of diagnosis or treatment is a cell or a mammal, including a human.

The agents and compositions of the present disclosure can be used in the manufacture of medicaments and for the treatment of humans and other animals by administration in accordance with conventional procedures, such as an active ingredient in pharmaceutical compositions.

An agent of the present disclosure can be administered for therapy by any suitable route, specifically by intrathecal (injection into the spinal fluid), intravenous or intranasal administration.

The terms "polynucleotide" and "oligonucleotide" are used interchangeably and refer to a polymeric form of nucleotides of any length, either deoxyribonucleotides or ribonucleotides or analogs thereof. A polynucleotide can comprise modified nucleotides, such as methylated nucleotides and nucleotide analogs. If present, modifications to the nucleotide structure can be imparted before or after assembly of the polynucleotide. The sequence of nucleotides can be interrupted by non-nucleotide components. A polynucleotide can be further modified after polymerization, such as by conjugation with a labeling component. The term also refers to both double- and single-stranded molecules. Unless otherwise specified or required, any aspects of this disclosure that is a polynucleotide encompasses both the double-stranded form and each of two complementary single-stranded forms known or predicted to make up the double-stranded form.

A polynucleotide is composed of a specific sequence of four nucleotide bases: adenine (A); cytosine (C); guanine (G); thymine (T); and uracil (U) for thymine when the polynucleotide is RNA. Thus, the term "polynucleotide sequence" is the alphabetical representation of a polynucleotide molecule. This alphabetical representation can be input into databases in a computer having a central processing unit and used for bioinformatics applications such as functional genomics and homology searching.

"Homology" or "identity" or "similarity" refers to sequence similarity between two peptides or between two nucleic acid molecules. Homology can be determined by comparing a position in each sequence which may be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares less than 40% identity, or alternatively less than 25% identity, with one of the sequences of the present disclosure.

A polynucleotide or polynucleotide region (or a polypeptide or polypeptide region) has a certain percentage (for example, 60%, 65%, 70%, 75%, 80%, 85%, 90%, 95%, 98% or 99%) of "sequence identity" to another sequence means that, when aligned, that percentage of bases (or amino acids) are the same in comparing the two sequences. This alignment and the percent homology or sequence identity can be determined using software programs known in the art.

For each polynucleotide or polypeptide disclosed in the present disclosure, its biological equivalents are also contemplated. Biologically equivalents are those having the specified percent sequence identity (e.g., at least 75%, 80%, 85%, 90%, 95%, 97%, 98%, 99%) and having the same or similar biological activity as the reference polypeptide or encoding a polypeptide that has the same or similar biological activity as the polypeptide encoded by the reference polynucleotide. In some aspects, a biologically equivalent has one, two, three, four or five addition, deletion or substitution of amino acid resides or nucleotides as compared to the reference polypeptide or polynucleotide.

## II. Methods of Treating MPS IIID

The present disclosure provides, in one aspect, an enzyme replacement treatment (ERT) for MPS IIID that will ameliorate or reverse the catastrophic and fatal neurologic decline caused by this disease. Unlike MPS I, the symptoms of MPS III are largely localized to the brain. Hence, an effective MPS III treatment needs to gain access to the brain. Delivery of large proteins such as the enzymes genetically missing in MPS III will not cross the blood-brain barrier if delivered systemically.

Therefore, in the present technology, a rhGNS is delivered intrathecally (directly into the spinal fluid) to effectively treat the underlying causes of the neurologic symptoms that dominate MPS III pathology.

Experimental data presented herein showed robust expression of rhGNS in Chinese hamster ovary cells enabling effective production of the protein. In a larger scale experiment, rhGNS ~100 μg per 1500 mL media was produced in CHO cells, and was purified to a specific activity of ~100,000 activity units/mg. The data further showed that the expressed rhGNS protein demonstrated maximal enzymatic activity at pH 5.6, demonstrated good enzymatic activity at 37 °C and was stable for over one month at 4 °C in artificial cerebrospinal fluid storage buffer.

Further, experiments showed intracellular enzymatic activity of rhGNS in MPS III fibroblasts when rhGNS was added to the media, and 70 ± 6% rhGNS colocalized with lysosomal markers using confocal microscopy and confirmed that radiolabelled HS diminished 33-65% in MPS III fibroblasts treated with rhGNS (to wild-type levels).

Moreover, in a mouse MPS IIID model, when about 5.3 μg of the rhGNS was administered, GNS enzyme activity recovered to higher than normal levels within 2 hours following the administration, and the activities of two lysosomal enzymes, alpha-N-acetylglucosaminidase (NAGLU) and β-hexoaminidase (HEX) which are overexpressed in MPS IIID patients, were significantly reduced within 1 day after the treatment. Such high and fast-acting *in vivo* efficacy was surprising and unexpected.

In accordance with one aspect of the present disclosure, provided is a method of treating mucopolysaccharidosis type IIID (MPS IIID) in a human patient in need thereof. In one aspect, the method entails injecting to the spinal fluid of the patient an effective amount of a composition comprising a rhGNS protein. In one aspect, the rhGNS includes the amino acid sequence of SEQ ID NO: 1 or an amino acid sequence (a) having a sequence identity (e.g., at least 75%, 80%, 85%, 90%, 95%, 97%, 98%, or 99% sequence identity) to SEQ ID NO: 1 (see **FIG. 1**) and (b) having the enzymatic activity of human GNS protein. In one aspect, the rhGNS includes the amino acid sequence of SEQ ID NO: 2 or an amino acid sequence (a) having a sequence identity (e.g., at least 75%, 80%, 85%, 90%, 95%, 97%, 98%, or 99% sequence identity) to SEQ ID NO: 2 and (b) having the enzymatic activity of human GNS protein. In one aspect, the rhGNS includes the amino acid sequence of SEQ ID NO: 5 or 6, or an amino acid sequence (a) having a sequence identity (e.g., at least 75%, 80%, 85%, 90%, 95%, 97%, 98%, or 99% sequence identity) to SEQ ID NO: 5 or 6 and (b) having the enzymatic activity of human GNS protein.

In some aspects, the rhGNS can be administered once every two weeks to every six months. In one aspect, the rhGNS can be administered once every two weeks, every three weeks, every four weeks, every five weeks, every six weeks, every seven weeks, every eight weeks, every month, every 2 months, every 3 months, every 4 months, every 5 months, or every 6 months. In some aspects, the rhGNS is administered for at least 1 year, 2 years, 5 years, or 10 years. In some aspects, the rhGNS is administered for no more than 2 years, 3 years, 4 year, 5 years, 10 years, 15 years, 20 years or 25 years.

In some aspects, for each administration, the amount of rhGNS is from about 0.05 mg/kg to about 5 mg/kg. In some aspects, for each administration, the amount of rhGNS is from about 0.05 mg/kg to about 4 mg/kg, from about 0.05 mg/kg to about 3 mg/kg, from about 0.05 mg/kg to about 2 mg/kg, from about 0.05 mg/kg to about 1 mg/kg, from about 0.05 mg/kg to about 0.5 mg/kg, from about 0.05 mg/kg to about 0.25 mg/kg, from about 0.1 mg/kg to about 4 mg/kg, from about 0.15 mg/kg to about 4 mg/kg, from about 0.20 mg/kg to about 4 mg/kg, from about 0.25 mg/kg to about 4 mg/kg, from about 0.5 mg/kg to about 4 mg/kg, from about 0.1 mg/kg to about 1 mg/kg, from about 0.1 mg/kg to about 0.9 mg/kg, from about 0.1 mg/kg to about 0.8 mg/kg, from about 0.1 mg/kg to about 0.7 mg/kg, from about 0.1 mg/kg to about 0.6 mg/kg, from about 0.1 mg/kg to about 0.5 mg/kg, from about 0.1 mg/kg to about 0.4 mg/kg, from about 0.1 mg/kg to about 0.3 mg/kg, from about 0.2 mg/kg to about 1 mg/kg, from about 0.2 mg/kg to about 0.9 mg/kg, from about 0.2 mg/kg to about 0.8 mg/kg, from about 0.2 mg/kg to about 0.7 mg/kg, from about 0.2 mg/kg to about 0.6 mg/kg, from about 0.2 mg/kg to about 0.5 mg/kg, from about 0.2 mg/kg to about 0.4 mg/kg. In some aspects, for each administration, the amount of rhGNS is about 0.01 mg/kg, 0.02 mg/kg, 0.03 mg/kg, 0.04 mg/kg, 0.05 mg/kg, 0.06 mg/kg, 0.07 mg/kg, 0.08 mg/kg, 0.09 mg/kg, 0.1 mg/kg, 0.11 mg/kg, 0.12 mg/kg, 0.13 mg/kg, 0.14 mg/kg, 0.15 mg/kg, 0.16 mg/kg, 0.17 mg/kg, 0.18 mg/kg, 0.19 mg/kg, 0.2 mg/kg, 0.21 mg/kg, 0.22 mg/kg, 0.23 mg/kg, 0.24 mg/kg, 0.25 mg/kg, 0.26 mg/kg, 0.27 mg/kg, 0.28 mg/kg, 0.29 mg/kg, 0.3 mg/kg, 0.35 mg/kg, 0.4 mg/kg, 0.45 mg/kg, 0.5 mg/kg, 0.55 mg/kg, 0.6 mg/kg, 0.65 mg/kg, 0.7 mg/kg, 0.75 mg/kg, 0.8 mg/kg, 0.85 mg/kg, 0.9 mg/kg, 0.95 mg/kg, 1 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, or 2 mg/kg.

In some aspects, for each administration, the amount of rhGNS is from about 1 mg to about 100 mg per human patient. In some aspects, for each administration, the amount of rhGNS is from about 1 mg to about 95 mg, from about 1 mg to about 90 mg, from about 1 mg to about 85 mg, from about 1 mg to about 80 mg, from about 1 mg to about 75 mg, from about 1 mg to about 70 mg, from about 1 mg to about 65 mg, from about 1 mg to about 60 mg, from about 1 mg to about 55 mg, from about 1 mg to about 50 mg, from about 1 mg to about 45 mg, from about 1 mg to about 40 mg, from about 1 mg to about 35 mg, from about 1 mg to about 30 mg, from about 1 mg to about 25 mg, from about 1 mg to about 20 mg, from about 1 mg to about 15 mg, from about 1 mg to about 10 mg, from about 5 mg to about 95 mg, from about 5 mg to about 90 mg, from about 5 mg to about 85 mg, from about 5 mg to about 80 mg, from about 5 mg to about 75 mg, from about 5 mg to about 70 mg, from about 5 mg to about 65 mg, from about 5 mg to about 60 mg, from about 5 mg to about 55 mg, from about 5 mg to about 50 mg, from about 5 mg to about 45 mg, from about 5 mg to about 40 mg, from about 5 mg to about 35 mg, from about 5 mg to about 30 mg, from about 5 mg to about 25 mg, from about 5 mg to about 20 mg, from about 5 mg to about 15 mg, from about 5 mg to about 10 mg, from about 10 mg to about 95 mg, from about 10 mg to about 90 mg, from about 10 mg to about 85 mg, from about 10 mg to about 80 mg, from about 10 mg to about 75 mg, from about 10 mg to about 70 mg, from about 10 mg to about 65 mg, from about 10 mg to about 60 mg, from about 10 mg to about 55 mg, from about 10 mg to about 50 mg, from about 10 mg to about 45 mg, from about 10 mg to about 40 mg, from about 10 mg to about 35 mg, from about 10 mg to about 30 mg, from about 10 mg to about 25 mg, from about 10 mg to about 20 mg, from about 10 mg to about 15 mg, from about 15 mg to about 95 mg, from about 15 mg to about 90 mg, from about 15 mg to about 85 mg, from about 15 mg to about 80 mg, from about 15 mg to about 75 mg, from about 15 mg to about 70 mg, from about 15 mg to about 65 mg, from about 15 mg to about 60 mg, from about 15 mg to about 55 mg, from about 15 mg to about 50 mg, from about 15 mg to about 45 mg, from about 15 mg to about 40 mg, from about 15 mg to about 35 mg, from about 15 mg to about 30 mg, from about 15 mg to about 25 mg, or from about 15 mg to about 20 mg.

In some aspects, for each administration, the amount of rhGNS is from about 1 mg to about 5 mg, from about 5 mg to about 10 mg, from about 10 mg to about 15 mg, from about 15 mg to about 20 mg, from about 20 mg to about 25 mg, from about 25 mg to about 30 mg, from about 30 mg to about 35 mg, from about 35 mg to about 40 mg, from about 40 mg to about 45 mg, from about 45 mg to about 50 mg, from about 1mg to about 10 mg, from about 5 mg to about 15 mg, from about 10 mg to about 20 mg, from about 15 mg to about 25 mg, from about 20 mg to about 30 mg, from about 25 mg to about 35 mg, from about 30 mg to about 40 mg, from about 35 mg to about 45 mg, from about 40 mg to about 50 mg, from about 50 mg to about 60 mg, from about 60 mg to about 70 mg, from about 70 mg to about 80, or from about 80 mg to about 90 mg.

In any of the methods described herein, it should be understood, even if not always explicitly stated, that an effective amount of an rhGNS of the present disclosure is administered to the subject. The amount can be empirically determined by the treating physician and will vary with the age, gender, weight and health of the subject. With these variables in mind, one of skill will administer a therapeutically effective amount to the subject to be treated. It is contemplated that a therapeutically effective amount of the rhGNS described herein may contain from about 0.01 milligram of rhGNS per kilogram of a subject's body weight to 1 gram of rhGNS per kilogram of a subject's body weight of rhGNS. In some aspects, a therapeutically effective amount of the rhGNS is from 50 mg to 2000 mg, or from 100 mg to 1000 mg, without limitation.

This GNS enzyme catalyzes the following chemical reaction: hydrolysis of the 6-sulfate groups of the N-acetyl-D-glucosamine 6-sulfate units of heparan sulfate and keratan sulfate. Therefore, in one aspect, the enzymatic activity of human GNS protein refers to the ability to catalyze the hydrolysis of the 6-sulfate groups of the N-acetyl-D-glucosamine 6-sulfate units of heparan sulfate or keratan sulfate. Methods of measuring such an activity are well known in the art. In one aspect, the rhGNS has at least 50% (or at least 60%, 70%, 80%, 85%, 90%, or 95%) activity of the wild-type human GNS in a suitable *in vivo* environment.

In some aspects, the composition is provided in an artificial cerebrospinal fluid. Methods of preparing artificial cerebrospinal fluids (ACSF) are known in the art and ACSF are also commercially available. The artificial cerebrospinal fluid may have a pH that is lower than about 8, lower than about 7.5, from about 5 to 8, from about 5.5 to about 7.5, from about 6 to about 7.5, from about 6 to about 7.

In some aspects, the artificial cerebrospinal fluid comprises about 130-170 mEq/l sodium, about 2.5-5 mEq/l potassium, about 1-3 mEq/l calcium, about 0.5-3 mEq/l magnesium, about 120-180 mEq/l chloride, and about 0.5-2 mEq/l phosphate. In some aspects, the artificial cerebrospinal fluid comprises about 140-160 mEq/l sodium, about 3.5-4.5 mEq/l potassium, about 2.5-3 mEq/l calcium, about 2-3 mEq/l magnesium, about 120-140 mEq/l chloride, and about 1-2 mEq/l phosphate. In some aspects, the artificial cerebrospinal fluid comprises about 140-160 mEq/l sodium, about 3.5-4.5 mEq/l potassium, about 2.5-3 mEq/l calcium, about 2-3 mEq/l magnesium, about 120-140 mEq/l chloride, about 1-2 mEq/l phosphate, about 18-25 mEq/l bicarbonate, and about 2-3 mEq/l sulfate. The osmolarity of the artificial cerebrospinal fluid can be about 250-350 mOsm/l, or about 260-300 mOsm/l. In one aspect, the ACSF contains sodium 149 mEq/l, potassium 4 mEq/l, calcium 2.7 mEq/l, magnesium 2.4 mEq/l, bicarbonate 22.6 mEq/l, chloride 132 mEq/l, sulfate 2.4 mEq/l, phosphate 1.5 mEq/l, pH 6-7.5, 288 mOsm/l but no protein.

In one aspect, the rhGNS exhibits the maximum enzymatic activity at an acidic condition (e.g., pH 5.3 to 5.9, pH 5.4 to 5.8, pH 5.5 to 5.7, pH 5.55 to 5.65, or at about pH 5.6). In one aspect, the rhGNS is at least twice as active at pH 5.6 as at pH 7.0. In another aspect, the rhGNS is at least three times, four times, five times, six times, seven times, eight times, nine time or 10 times as active at pH 5.6 as at pH 7.0.

Method of obtaining rhGNS of high purity and activity are demonstrated in the experimental examples can such rhGNS may be useful for practice of certain aspects as disclosed herein. In one aspect, a rhGNS composition suitable for the treatment includes from about 0.5 mg to about 30 mg of the recombinant protein per ml of the artificial cerebrospinal fluid, or from about 1 mg to about 25 mg, or from about 2 mg to about 20 mg, or from about 5 mg to about 15 mg per ml of the artificial cerebrospinal fluid.

In one aspect, the rhGNS of the present disclosure is able to enter a human fibroblast cell when the recombinant polypeptide is incubated with the human fibroblast cell. In some aspects, such incubation does not involve the use of a cell penetrating peptide, a nanoparticle such as a liposome, and/or the assistant of an agent that induces cell endocytosis (or pinocytosis or phagocytosis).

In some aspects, the rhGNS of the present disclosure is suitably glycosylated. It is readily appreciated that recombinant protein expressed and prepared in an *in vitro* environment undergoes different glycosylation process and/or ends up with different glycosylation than its wild-type counterpart. In one aspect, the rhGNS of the present disclosure adds from 25 kDa to 45 kDa molecular weight to the recombinant polypeptide. In one aspect, the rhGNS of the present disclosure adds at least 20, 25, 30, 35, or 40 kDa molecular weight to the recombinant polypeptide. In one aspect, the rhGNS of the present disclosure adds not more than 30, 35, 40, 45, 50, 55, or 60 kDa molecular weight to the recombinant polypeptide. In one aspect, the rhGNS of the present disclosure adds at from 20 to 60, 25 to 55, 25 to 50, 25 to 45, or 25 to 40, or 25 to 35 kDa molecular weight to the recombinant polypeptide.

In some aspects, the rhGNS is any one of the rhGNS as described above. In some aspects, the rhGNS is conjugated with a moiety capable of extending the circulating half-life of the rhGNS. In some aspects, the moiety is selected from the group consisting of polyethylene glycol, an acyl group, a liposome, a carrier protein, an artificial phospholipid membrane, and a nanoparticle.

A lysosomal targeting moiety can be added or conjugated to the rhGNS, in some aspects, to facilitate delivery. Lysosomal targeting moieties are known in the art. In one aspect, the targeting moiety is a means (e.g. a molecule) for binding the extracellular domain of the human cation-independent M6P receptor in an M6P-independent manner when the receptor is present in the plasma membrane of a target cell. In another aspect, the targeting moiety is an unglycosylated lysosomal targeting domain that binds the extracellular domain of the human cation-independent M6P receptor. In either aspect, the targeting moiety can include, for example, IGF-II; retinoic acid or a derivative thereof; a protein having an amino acid sequence at least 70% identical to a domain of urokinase-type plasminogen activator receptor; an antibody variable domain that recognizes the receptor; or variants thereof.

In another aspect, the targeting moiety is a lysosomal targeting domain that binds the extracellular domain of the human cation-independent M6P receptor but does not bind a mutein of the receptor in which amino acid 1572 is changed from isoleucine to threonine, or binds the mutein with at least ten-fold less affinity (i.e. with at least a ten-fold greater dissociation constant). In another aspect, the targeting moiety is a lysosomal targeting domain capable of binding a receptor domain consisting essentially of repeats 10-15 of the human cation-independent M6P receptor: the lysosomal targeting domain can bind a protein that includes repeats 10-15 even if the protein includes no other moieties that bind the lysosomal targeting domain. Preferably, the lysosomal targeting domain can bind a receptor domain consisting essentially of repeats 10-13 of the human cation-independent M6P receptor.

In some aspects, the lysosomal targeting domain can bind a receptor domain consisting essentially of repeats 11-12, repeat 11, or amino acids 1508-1566 of the human cation-independent M6P receptor. In each of these aspects, the lysosomal targeting domain preferably binds the receptor or receptor domain with a submicromolar dissociation constant at or about pH 7.4. In one preferred aspect, the lysosomal targeting domain binds with an dissociation constant of about 10-7 M. In another preferred aspect, the dissociation constant is less than about 10-7 M.

In another aspect, the targeting moiety is a binding moiety sufficiently duplicative of human IGF-II such that the binding moiety binds the human cation-independent M6P receptor. The binding moiety can be sufficiently duplicative of IGF-II by including an amino acid sequence sufficiently homologous to at least a portion of IGF-II, or by including a molecular structure sufficiently representative of at least a portion of IGF-II, such that the binding moiety binds the cation-independent M6P receptor. The binding moiety can be an organic molecule having a three-dimensional shape representative of at least a portion of IGF-II, such as amino acids 48-55 of human IGF-II, or at least three amino acids selected from the group consisting of amino acids 8, 48, 49, 50, 54, and 55 of human IGF-II. A preferred organic molecule has a hydrophobic moiety at a position representative of amino acid 48 of human IGF-II and a positive charge at or about pH 7.4 at a position representative of amino acid 49 of human IGF-II. In one aspect, the binding moiety is a polypeptide including a polypeptide having antiparallel alpha-helices separated by not more than five amino acids. In another aspect, the binding moiety includes a polypeptide with the amino acid sequence of IGF-I or of a mutein of IGF-I in which amino acids 55-56 are changed and/or amino acids 1-4 are deleted or changed. In a further aspect, the binding moiety includes a polypeptide with an amino acid sequence at least 60% identical to human IGF-II; amino acids at positions corresponding to positions 54 and 55 of human IGF-II are preferably uncharged or negatively charged at or about pH 7.4.

In one aspect, the targeting moiety is a polypeptide comprising the amino acid sequence phenylalanine-arginine-serine. In another aspect, the targeting moiety is a polypeptide including an amino acid sequence at least 75% homologous to amino acids 48-55 of human IGF-II. In another aspect, the targeting moiety includes, on a single polypeptide or on separate polypeptides, amino acids 8-28 and 41-61 of human IGF-II. In another aspect, the targeting moiety includes amino acids 41-61 of human IGF-II and a mutein of amino acids 8-28 of human IGF-II differing from the human sequence at amino acids 9, 19, 26, and/or 27.

### III. Methods of Preparing the Recombinant Human GNS Protein

Polypeptides of this disclosure can be prepared by expressing polynucleotides encoding the polypeptide sequences of this disclosure in an appropriate host cell. This can be accomplished by methods of recombinant DNA technology known to those skilled in the art. The proteins and polypeptides of this disclosure also can be obtained by chemical synthesis using a commercially available automated peptide synthesizer such as those manufactured by Perkin Elmer/Applied Biosystems, Inc., Model 430A or 431A, Foster City, CA, USA. The synthesized protein or polypeptide can be precipitated and further purified, for example by high performance liquid chromatography (HPLC). Accordingly, this disclosure also provides a process for chemically synthesizing the proteins of this disclosure by providing the sequence of the protein and reagents, such as amino acids and enzymes and linking together the amino acids in the proper orientation and linear sequence.

It is known to those skilled in the art that modifications can be made to any peptide to provide it with altered properties. Polypeptides of the disclosure can be modified to include unnatural amino acids. Thus, the peptides may comprise D-amino acids, a combination of D- and L-amino acids, and various "designer" amino acids (*e.g.*, β-methyl amino acids, C-α-methyl amino acids, and N-α-methyl amino acids, etc.) to convey special properties to peptides. Additionally, by assigning specific amino acids at specific coupling steps, peptides with α-helices, β turns, β sheets, α-turns, and cyclic peptides can be generated. Generally, it is believed that α-helical secondary structure or random secondary structure is preferred.

In a further embodiment, subunits of polypeptides that confer useful chemical and structural properties will be chosen. For example, peptides comprising D-amino acids may be resistant to L-amino acid-specific proteases *in vivo*. Modified compounds with D-amino acids may be synthesized with the amino acids aligned in reverse order to produce the peptides of the disclosure as retro-inverso peptides. In addition, the present disclosure envisions preparing peptides that have better defined structural properties, and the use of peptidomimetics, and peptidomimetic bonds, such as ester bonds, to prepare peptides with novel properties. In another embodiment, a peptide may be generated that incorporates a reduced peptide bond, *i.e.,* $R_1$-$CH_2NH$-$R_2$, where $R_1$, and $R_2$ are amino acid residues or sequences. A reduced peptide bond may be introduced as a dipeptide subunit. Such a molecule would be resistant to peptide bond hydrolysis, e.g., protease activity. Such molecules would provide ligands with unique function and activity, such as extended half-lives *in vivo* due to resistance to metabolic breakdown, or protease activity. Furthermore, it is well known that in certain systems constrained peptides show enhanced functional activity (Hruby (1982) Life Sciences 31:189-199 and Hruby et al. (1990) Biochem J. 268:249-262); the present disclosure provides a method to produce a constrained peptide that incorporates random sequences at all other positions.

The following non-classical amino acids may be incorporated in the peptides of the disclosure in order to introduce particular conformational motifs: 1,2,3,4-tetrahydroisoquinoline-3-carboxylate (Kazmierski et al. (1991) J. Am. Chem. Soc. 113:2275-2283); (2S,3S)-methyl-phenylalanine, (2S,3R)- methyl-phenylalanine, (2R,3S)-methyl-phenylalanine and (2R,3R)-methyl-phenylalanine (Kazmierski and Hruby (1991) Tetrahedron Lett. 32(41):5769-5772); 2-aminotetrahydronaphthalene-2- carboxylic acid (Landis (1989) Ph.D. Thesis, University of Arizona); hydroxy-1,2,3,4-tetrahydroisoquinoline-3-carboxylate (Miyake et al. (1989) J. Takeda Res. Labs. 43:53-76) histidine isoquinoline carboxylic acid (Zechel et al. (1991) Int. J. Pep. Protein Res. 38(2):131-138); and HIC (histidine cyclic urea), (Dharanipragada et al. (1993) Int. J. Pep. Protein Res. 42(1):68-77) and (Dharanipragada et al. (1992) Acta. Crystallogr. C. 48:1239-1241).

The following amino acid analogs and peptidomimetics may be incorporated into a peptide to induce or favor specific secondary structures: LL-Acp (LL-3-amino-2-propenidone-6-carboxylic acid), a β-turn inducing dipeptide analog (Kemp et al. (1985) J. Org. Chem. 50:5834-5838); β-sheet inducing analogs (Kemp et al. (1988) Tetrahedron Lett. 29:5081-5082); β-turn inducing analogs (Kemp et al. (1988) Tetrahedron Lett. 29:5057-5060); α-helix inducing analogs (Kemp et al. (1988) Tetrahedron Lett. 29:4935-4938); α-turn inducing analogs (Kemp et al. (1989) J. Org. Chem. 54:109:115); analogs provided by the following references: Nagai and Sato (1985) Tetrahedron Lett. 26:647-650; and DiMaio et al. (1989) J. Chem. Soc. Perkin Trans. p. 1687; a Gly-Ala turn analog (Kahn et al. (1989) Tetrahedron Lett. 30:2317); amide bond isostere (Clones et al. (1988) Tetrahedron Lett. 29:3853-3856); tetrazole (Zabrocki et al. (1988) J. Am. Chem. Soc. 110:5875-5880); DTC (Samanen et al. (1990) Int. J. Protein Pep. Res. 35:501:509); and analogs taught in Olson et al. (1990) J. Am. Chem. Sci. 112:323-333 and Garvey et al. (1990) J. Org. Chem. 56:436. Conformationally restricted mimetics of beta turns and beta bulges, and peptides containing them, are described in U.S. Patent No. 5,440,013, issued August 8, 1995 to Kahn.

It is known to those skilled in the art that modifications can be made to any peptide by substituting one or more amino acids with one or more functionally equivalent amino acids that does not alter the biological function of the peptide. In one aspect, the amino acid that is substituted by an amino acid that possesses similar intrinsic properties including, but not limited to, hydrophobicity, size, or charge. Methods used to determine the appropriate amino acid to be substituted and for which amino acid are known to one of skill in the art. Non-limiting examples include empirical substitution models as described by Dahoff et al. (1978) In Atlas of Protein Sequence and Structure Vol. 5 suppl. 2 (ed. M.O. Dayhoff), pp. 345-352. National Biomedical Research Foundation, Washington DC; PAM matrices including Dayhoff matrices (Dahoff et al. (1978), *supra,* or JTT matrices as described by Jones et al. (1992) Comput. Appl. Biosci. 8:275-282 and Gonnet et al. (1992) Science 256:1443-1145; the empirical model described by Adach and Hasegawa (1996) J. Mol. Evol. 42:459-468; the block substitution matrices (BLOSUM) as described by Henikoff and Henikoff (1992) Proc. Natl. Acad.

Sci. USA 89:1; Poisson models as described by Nei (1987) Molecular Evolutionary Genetics. Columbia University Press, New York.; and the Maximum Likelihood (ML) Method as described by Müller et al. (2002) Mol. Biol. Evol. 19:8-13.

Prior to use of the composition for the therapeutic use, it is desirable to test the composition for the enzymatic activity. It is a discovery of the present disclosure that the enzyme is completely inhibited in the presence of free phosphate, sulfate, or citrate groups. Therefore, purification and other assay buffer conditions should be optimized to avoid addition of these components. Further, addition of the detergent Triton X-100 (Sigma) significantly increased reproducibility and showed maximal enzyme specific activity at low concentrations (0.01%).

Accordingly, in one embodiment, provided is a method of testing the sulfatase activity of a recombinant polypeptide, comprising reacting the recombinant polypeptide with para-nitrocatechol sulfate (PNCS) in a testing buffer having less than 500 mM sodium, less than 100 mM citrate, less than 100 mM phosphate, less than 100 mM sulfate, and at least 0.005% Triton X-100, wherein the recombinant polypeptide is any one of described herein.

In some embodiments, the testing buffer has less than 250 mM sodium, less than 50 mM citrate, less than 50 mM phosphate, less than 50 mM sulfate, and at least 0.01% Triton X-100. In some embodiments, the testing buffer has less than 20 mM citrate, less than 20 mM phosphate, and less than 20 mM sulfate. In some embodiments, the testing buffer has less than 10 mM citrate, less than 10 mM phosphate, and less than 10 mM sulfate. In some embodiments, the testing buffer has less than 5 mM citrate, less than 5 mM phosphate, and less than 5 mM sulfate.

In some embodiments, the testing buffer has pH of about 5 to about 6, preferably at about 5.6. In some embodiments, the testing buffer has about 200 mM sodium acetate and about 0.01% Triton X-100, at about pH 5.6. The testing can be carried out for a few hours, e.g., 1-4 hours, at a temperature such as 37 °C.

The testing reaction can be then stopped by adding a stopping buffer, which preferably comprises a base. In some embodiments, the stopping buffer is a phosphate-citrate buffer, such as one having at least 0.4 M $Na_2HPO_4$ in at least 0.2 M citric acid.

Once the composition is tested, it can be provided is an artificial cerebrospinal fluid to prepare a composition for the therapeutic use. The artificial cerebrospinal fluid, in some embodiments, has a pH of about 6 to 7.5.

### IV. Polynucleotides, Host Cells and Compositions

This disclosure also provides polynucleotides that encode any polypeptide of the present disclosure, and their complements. Complementarity can be determined using traditional hybridization under conditions of moderate or high stringency. As used herein, the term polynucleotide intends DNA and RNA as well as modified nucleotides. For example, this disclosure also provides the anti-sense polynucleotide stand, e.g. antisense RNA to these sequences or their complements.

Further provided, in one aspect, are polynucleotide sequences useful for expressing the rhGNS protein. In one aspect, the polynucleotide sequences are different from the wild-type human cDNA sequence, or any wild-type GNS sequence. In one aspect, the coding sequence of GNS is optimized to achieve high expression efficiency. In one aspect, the coding sequence includes SEQ ID NO: 3.

In some aspects, the polynucleotide includes a nucleic acid sequence (a) having at least 85% (or at least 75%, 80%, 90%, 95%, 97%, 98%, or 99%) sequence identify to SEQ ID NO: 3, (b) encoding the amino acid sequence of SEQ ID NO: 1 (or a biological equivalent of SEQ ID NO: 1 as disclosed herein), and (c) having no more than 95% (or no more than 90%, or 85%) sequence identity to SEQ ID NO: 4. As shown in FIG. 5, SEQ ID NO: 3 and 4 have a sequence identity of about 77.4% (1281 matches over 1656 nucleotides).

Also provided are polynucleotides encoding substantially homologous and biologically equivalent polypeptides to the inventive polypeptides and polypeptide complexes. Substantially homologous and biologically equivalent intends those having varying degrees of homology, such as at least 65%, or alternatively, at least 70 %, or alternatively, at least 75 %, or alternatively at least 80 %, or alternatively, at least 85 %, or alternatively at least 90 %, or alternatively, at least 95 %, or alternatively at least 97 % homologous as defined above and which encode polypeptides having the biological activity of human GNS. It should be understood although not always explicitly stated that aspects to substantially homologous polypeptides and polynucleotides are intended for each aspect of this disclosure, e.g., polypeptides, polynucleotides and antibodies.

The polynucleotides of this disclosure can be replicated using conventional recombinant techniques. Alternatively, the polynucleotides can be replicated using PCR technology. PCR is the subject matter of U.S. Patent Nos. 4,683,195; 4,800,159; 4,754,065; and 4,683,202 and described in PCR: The Polymerase Chain Reaction (Mullis et al. eds, Birkhauser Press, Boston (1994)) and references cited therein. Yet further, one of skill in the art can use the sequences provided herein and a commercial DNA synthesizer to replicate the DNA. Accordingly, this disclosure also provides a process for obtaining the polynucleotides of this disclosure by providing the linear sequence of the polynucleotide, appropriate primer molecules, chemicals such as enzymes and instructions for their replication and chemically replicating or linking the nucleotides in the proper orientation to obtain the polynucleotides. In a separate aspect, these polynucleotides are further isolated. Still further, one of skill in the art can operatively link the polynucleotides to regulatory sequences for their expression in a host cell. The polynucleotides and regulatory sequences are inserted into the host cell (prokaryotic or eukaryotic) for replication and amplification. The DNA so amplified can be isolated from the cell by methods well known to those of skill in the art. A process for obtaining polynucleotides by this method is further provided herein as well as the polynucleotides so obtained.

RNA can be obtained by first inserting a DNA polynucleotide into a suitable prokaryotic or eukaryotic host cell. The DNA can be inserted by any appropriate method, e.g., by the use of an appropriate gene delivery vehicle (*e.g.*, liposome, plasmid or vector) or by electroporation. When the cell replicates and the DNA is transcribed into RNA; the RNA can then be isolated using methods well known to those of skill in the art, for example, as set forth in Sambrook and Russell (2001) *supra*. For instance, mRNA can be isolated using various lytic enzymes or chemical solutions according to the procedures set forth in Sambrook and Russell (2001) *supra* or extracted by nucleic-acid-binding resins following the accompanying instructions provided by manufactures.

In one aspect, provided is a construct comprising the polynucleotide, a protein prepared by expressing the polynucleotide, a cell enclosing the polynucleotide, or a cell stably transfected with the polynucleotide, which is optionally integrated into the cell chromosomes.

Also provided are host cells comprising one or more of the polypeptides or polynucleotides of this disclosure. In one aspect, the polypeptides are expressed and present on the cell surface (extracellularly). Suitable cells containing the disclosed polypeptides include prokaryotic and eukaryotic cells, which include, but are not limited to bacterial cells, yeast cells, insect cells, animal cells, mammalian cells, murine cells, rat cells, sheep cells, simian cells and human cells. Examples of bacterial cells include *Escherichia coli, Salmonella enterica* and *Streptococcus gordonii.* The cells can be purchased from a commercial vendor such as the American Type Culture Collection (ATCC, Rockville Maryland, USA) or cultured from an isolate using methods known in the art. Examples of suitable eukaryotic cells include, but are not limited to 293T HEK cells, as well as the hamster cell line CHO, BHK-21; the murine cell lines designated NIH3T3, NS0, C127, the simian cell lines COS, Vero; and the human cell lines HeLa, PER.C6 (commercially available from Crucell) U-937 and Hep G2. A non-limiting example of insect cells include *Spodoptera frugiperda.* Examples of yeast useful for expression include, but are not limited to *Saccharomyces, Schizosaccharomyces, Hansenula, Candida, Torulopsis, Yarrowia,* or *Pichia*. *See* e.g., U.S. Patent Nos. 4,812,405; 4,818,700; 4,929,555; 5,736,383; 5,955,349; 5,888,768 and 6,258,559.

The present disclosure further provides compositions comprising an rhGNS of the present disclosure and a pharmaceutically acceptable carrier.

"Pharmaceutically acceptable carriers" refers to any diluents, excipients, or carriers that may be used in the compositions of the disclosure. Pharmaceutically acceptable carriers include ion exchangers, alumina, aluminum stearate, lecithin, serum proteins, such as human serum albumin, buffer substances, such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Suitable pharmaceutical carriers are described in Remington's Pharmaceutical Sciences, Mack Publishing Company, a standard reference text in this field. They are preferably selected with respect to the intended form of administration, that is, oral tablets, capsules, elixirs, syrups and the like, and consistent with conventional pharmaceutical practices.

The pharmaceutical compositions of the disclosure can be manufactured by methods well known in the art such as conventional granulating, mixing, dissolving, encapsulating, lyophilizing, or emulsifying processes, among others. Compositions may be produced in various forms, including granules, precipitates, or particulates, powders, including freeze dried, rotary dried or spray dried powders, amorphous powders, injections, emulsions, elixirs, suspensions or solutions. Formulations may optionally contain stabilizers, pH modifiers, surfactants, bioavailability modifiers and combinations of these.

Pharmaceutical formulations may be prepared as liquid suspensions or solutions using a sterile liquid, such as oil, water, alcohol, and combinations thereof. Pharmaceutically suitable surfactants, suspending agents or emulsifying agents, may be added for oral or parenteral administration. Suspensions may include oils, such as peanut oil, sesame oil, cottonseed oil, corn oil and olive oil. Suspension preparation may also contain esters of fatty acids, such as ethyl oleate, isopropyl myristate, fatty acid glycerides and acetylated fatty acid glycerides. Suspension formulations may include alcohols, such as ethanol, isopropyl alcohol, hexadecyl alcohol, glycerol and propylene glycol. Ethers, such as poly(ethyleneglycol), petroleum hydrocarbons, such as mineral oil and petrolatum, and water may also be used in suspension formulations.

The compositions of this disclosure are formulated for pharmaceutical administration to a mammal, preferably a human being. Such pharmaceutical compositions of the disclosure may be administered in a variety of ways, preferably intrathecally. Other routes, such as intravenous and intranasal are contemplated as well.

Sterile injectable forms of the compositions of this disclosure may be aqueous or oleaginous suspension. These suspensions may be formulated according to techniques known in the art using suitable dispersing or wetting agents and suspending agents. The sterile injectable preparation may also be a sterile injectable solution or suspension in a nontoxic parenterally acceptable diluent or solvent, for example as a solution in 1,3-butanediol. Among the acceptable vehicles and solvents that may be employed is an artificial cerebrospinal fluid. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation. Compounds may be formulated for parenteral administration by injection such as by bolus injection or continuous infusion. A unit dosage form for injection may be in ampoules or in multi-dose containers.

In addition to dosage forms described above, pharmaceutically acceptable excipients and carriers and dosage forms are generally known to those skilled in the art and are included in the disclosure. It should be understood that a specific dosage and treatment regimen for any particular patient will depend upon a variety of factors, including the activity of the specific rhGNS employed, the age, body weight, general health, sex and diet, renal and hepatic function of the patient, and the time of administration, rate of excretion, drug combination, judgment of the treating physician or veterinarian and severity of the particular disease being treated.

Also provided by this disclosure are pharmaceutical compositions containing one or more of the rhGNS of the present disclosure and a pharmaceutically acceptable carrier. The compositions are administered to a subject in need thereof in an amount that will provide the desired benefit. The compositions can be co-administered with any suitable agent or therapy that complements or enhances the activity of the rhGNS. An example of such is a second agent capable of extending the plasma half-life of the rhGNS. Examples of suitable second agents include but are not limited to an anti-rhGNS antibody recognizing the exosite of the rhGNS.

Combination therapies are also provided. In addition to the injection, the patient can further receive a therapy such as bone marrow replacement, or administration of genistein or a chaperone. Genistein is a compound with the chemical name of 5,7-Dihydroxy-3-(4-hydroxyphenyl)chromen-4-one. Suitable chaperones can be screened by high throughput screening and computational screening for a particular patient.

## EXAMPLES

The disclosure is further understood by reference to the following examples, which are intended to be purely exemplary of the invention.

### Example 1. Preparation and *in vitro* Testing of rhGNS

In this example, a stably-transfected Chinese hamster ovarian (CHO) cell line was used to produce pre-clinical levels of recombinant human GNS (rhGNS) protein. rhGNS has been purified and enzymatically characterized. Suitable storage conditions have been identified for both longevity and safe administration. Using MPS IIID fibroblasts, this example has evaluated its cellular uptake, mediated via the M6P receptor, and further demonstrated localization within the lysosome and the ability to reduce glycosaminoglycan (GAG) storage.

The coding sequence (cDNA) of rhGNS (SEQ ID NO: 3, **FIG. 3;** for comparison, see the wild-type sequence of SEQ ID NO: 4, **FIG. 4,** and their sequence alignment in **FIG. 5)** was inserted into a mammalian expression plasmid using restriction enzyme digestion and ligation. The rhGNS can contain a c-myc moiety (as illustrated in **FIG. 2A**) or contains a C-terminal TEV protease cleavage site between the protein and the c-myc moiety (see **FIG. 2B** and **2C** for pre- and post-cleavage sequences) for ease of purification, and expression is driven by a CMV promoter. Chinese hamster ovary (CHO) cells were stably transfected, isolated, and screened for high expressing clones. Cells were grown in roller bottles and media harvested to obtain the secreted rhGNS. Following concentration, media was loaded into a c-myc affinity column, washed, and then eluted using soluble c-myc peptide in artificial cerebrospinal fluid (Elliotts B Solution, USP). Eluted rhGNS was then concentrated to a final concentration of 1 mg/ml. This example achieved a yield of >100 μg rhGNS per 1500 mL media, reaching a specific activity >200,000 units/mg (**Table 1**).

**Table 1: Purification of secreted rhGNS from CHO cell line using c-myc affinity column.**

| Step | Volume (ml) | Protein (μg) | Total activity (units)* | Specific Activity (units/mg protein) |
|---|---|---|---|---|
| PF CHO Media | 1,450 | 330,165 | 108,998 | 330 |

(continued)

| Step | Volume (ml) | Protein (μg) | Total activity (units)* | Specific Activity (units/mg protein) |
|---|---|---|---|---|
| Concentrated Media | 110 | 304,920 | 307,826 | 1,010 |
| Flow through | 110 | 302,878 | 273,667 | 904 |
| myc Elution | 3 | 186 | 26,408 | 142,263 |
| Elution Concentration | 0.14 | 119 | 28,593 | 241,058 |

Western Blot analysis of the purification steps (**FIG. 6A**) and glycosidase digestion revealed that purified rhGNS is highly glycosylated (**FIG. 6B**), which is vital for both intracellular uptake and lysosomal targeting. Using the fluorogenic substrate 4-Methylumbelliferyl 6-Sulfo-2-acetamido-2-deoxy-a-D-glucopyranoside (4-MU-GNS), purified rhGNS was shown to be both enzymatically active and stable following storage in artificial cerebral spinal fluid (**FIG. 6C**). Further biochemical characterization of the enzyme showed optimal reaction conditions within the lysosomal pH range (4-5.6), with 10-fold lower activity at neutral pH (**FIG. 6D**).

Enzymatically-active rhGNS was taken up by MPSIIID fibroblasts and co-localized with lysosomal markers (**FIG. 7A**). Both uptake and lysosomal targeting were shown to be decrease significantly in the presence of free M6P (**FIG. 7B**), suggesting that our rhGNS is rich in M6P glycosylation and uptake is M6P receptor dependent. We demonstrated a minimum of 33% and a maximum of 65% reduction in heparan sulfate in three independent experiments in two human MPS IIID cell lines treated with rhGNS, reaching wild-type levels of heparan sulfate (**FIG. 7C**). Further, radiolabeled GAG accumulation measured at different concentrations of purified rhGNS (0-250 pM) demonstrated an exponential decrease in storage, with storage reduced by half ($EC_{50}$) at 5.5 pM. Radiolabeled GAGs were extracted and measured via scintillation counting, and radioactive counts per minute were normalized to protein concentration (**FIG. 7D**). This result indicates that rhGNS produced in this example was not only active against the artificial substrate, but was also able to catabolize the primary substrate that is responsible for MPS IIID neuropathology.

Rare childhood neurodegenerative disorders like MPS IIID are some of the most heartbreaking and devastating diseases imaginable. To date, there is no approved treatment or cure for MPS IIID. Using a CHO stable cell line, this example was able to produce and purify enzymatically active and highly-glycosylated rhGNS. The product was mannose-6-phosphorylated, entered into human MPS IIID cells, targets to the lysosomal compartment, and is stable in the ideal vehicle for intrathecal delivery. Furthermore, we have shown that it is able to decrease accumulation of GAG in MPS IIID patient fibroblasts to WT levels, thereby correcting the primary physiological effects of the disease.

### Example 2. Additional Testing of rhGNS

As shown in **FIG. 8,** this example assayed rhGNS over a range of temperature and found good enzymatic activity at body temperature.

The example also developed a storage buffer that will enable at least 1 month of storage. Purified rhGNS was tested in a variety of buffers, and was found to be stable for over one month at 4 °C in artificial cerebrospinal fluid (**FIG. 9**). Artificial cerebrospinal fluid is formulated to mimic the electrolyte composition of natural cerebrospinal fluid but contains no protein (sodium 149 mEq/l, potassium 4 mEq/l, calcium 2.7 mEq/l, magnesium 2.4 mEq/l, bicarbonate 22.6 mEq/l, chloride 132 mEq/l, sulfate 2.4 mEq/l, phosphate 1.5 mEq/l, pH 6-7.5, 288 mOsm/l). Thus not only did this example demonstrate stability of rhGNS for >1 month in a storage buffer, it was able to show stability in the *ideal* storage buffer for intrathecal administration to patients.

### Example *3. In vivo* Testing of rhGNS

This example will perform *in vivo* proof-of-concept studies in MPS IIID mice. *In vivo* effectiveness of the intrathecal delivery of rhGNS can be demonstrated such as the alleviation of neurologic, cognitive, and/or neurobehavioral pathologies caused by MPS IIID. This example will scale up our production of rhGNS so that the enzyme activity, lysosomal storage reduction, neuropathology and half-life estimation can be studied in the recently characterized MPS IIID knock out mouse. It is contemplated that this example will produce sufficient rhGNS, demonstrate increased enzyme activity, leading to lysosomal storage reduction and improved neuropathology and a half-life in the MPS IIID knock out mouse that would ultimately be suggestive of favorable kinetics in humans.

This example will also perform process development and product characterization. Eventual production of rhGNS for preclinical studies, clinical trials, and human patients will require a scalable process that can be readily adapted to a current good manufacturing practice (CGMP) facility. This example will develop a production and purification process that is scalable to preclinical and clinical needs, and perform product characterization (protein interaction, aggregation, glycosylation, etc.) and assessment of batch-to-batch variability.

**Example 4. Testing of rhGNS in MPS IIID Mice**

This example tested the efficacy of rhGNS in MPS IIID knock out mice, with non-diseased mice as control. Recombinant human GNS (rhGNS) were produced as described above, using cerebrospinal fluid (CSF) as the vehicle which alone served as control. The treatment groups are shown in the table below.

| Group No. (Name) | Mice | Treatment | n |
|---|---|---|---|
| 1 (Carrier) | Non-diseased | CSF | 3 |
| 2 (MPS 3D) | MPS IIID | CSF | 3 |
| 3 (MPS 3D ERT) | MPS IIID | rhGNS (5.3μg) | 2 |

As shown in **FIG. 10A,** only two hours after dosing, the GNS enzyme activity (nmol/hr/mg) in group 3 was already higher than in the control group 1 (carrier). By contrast, group 2 which did not receive the treatment had hardly detectable GNS activity. Similar results were also observed at 4 hours (**FIG. 10B**).

The activities of two lysosomal enzymes, alpha-N-acetylglucosaminidase (NAGLU) and β-hexoaminidase (HEX), were measured one day after dosing. The results are presented in **FIG. 11A and 11B,** respectively. For both enzymes, the activity levels were higher in MPS IIID mice, but the activities were reduced by the treatment, demonstrating the effectiveness of the treatment.

**Example 5. Enzyme Replacement Therapy for Mucopolysaccharidosis IIID using Recombinant Human alpha-N-acetylglucosamine-6-sulfatase Demonstrates reduction of Lysosomal Storage Markers In vitro and In vivo**

There is currently no cure or effective treatment available for Mucopolysaccharidosis type IIID (MPS IIID, Sanfilippo syndrome type D) which is a lysosomal storage disorder (LSD) caused by the deficiency of α-*N*-acetylglucosamine-6-sulfatase (GNS). The clinical symptoms of MPS IIID, like other subtypes of Sanfilippo syndromes, are largely localized to the central nervous system (CNS) and any treatment aiming to ameliorate or reverse the catastrophic and fatal neurologic decline caused by this disease will be required to be delivered across the blood brain barrier. We now describe an enzyme replacement treatment (ERT) for MPS IIID using recombinant human α-*N*-acetylglucosamine-6-sulfatase (rhGNS) via intracerebroventricular delivery. We produced rhGNS in CHO cells with specific activity of ~100,000 activity units/mg protein, with maximal enzymatic activity at lysosomal pH (pH 5.6), and stable enzymatic activity at 4°C over one month in artificial cerebrospinal fluid. Additionally, we have demonstrated rhGNS was taken up in MPSIIID fibroblasts and rhGNS can clear intracellular glycosaminoglycans (GAGs). Our *in vivo* study by delivery of rhGNS in the cerebroventricles of neonatal MPS IIID mice demonstrated the recovery of the GNS enzyme activity level and a significant reduction of lysosomal storage markers throughout the brain. The *in vivo* half-life of rhGNS was estimated to be ~1.1 day and rhGNS was found to localize in lysosomes in treated mice. Our results therefore demonstrate the potential of using rhGNS as a potential ERT for MPS IIID in preparation for further IND enabling studies.

In the present study, recombinant human GNS (rhGNS) was expressed and purified from CHO cells, characterized to determine glycosylation and mannose 6-phosphate content, and shown to be enzymatically active using two optimized *in vitro* assays. Delivery of rhGNS to MPS IIID patient fibroblasts demonstrated intracellular uptake, reduction of GAG storage *in vitro.* Using an MPS IIID knock out mouse model we have also demonstrated normalization of brain GNS and reduction of lysosomal storage markers throughout the brain. These results may indicate that this rhGNS may be a viable clinical candidate for ERT to treat MPS IIID and is worthy of further exploration.

**Methods**

**Molecular cloning of rhGNS-** An expression cassette containing the full-length of human *GNS* cDNA coding for the 552 amino acids (NM_002076.3) and c-myc moiety (EQKLISEED) with a short unstructured GGS linker in between was synthesized using codon optimization for expression in Chinese hamster ovary (CHO) cells by Genscript USA Inc. (Piscataway, NJ). It was further cloned in an expression vector pCI-Neo driven by a CMV promoter (Promega Corporation, Madison, WI). For purification purposes, a TEV protease cleavage site was further introduced in the C-terminal of this construct using QuikChange II XL site-directed mutagenesis kit (Agilent Technologies, Inc., Santa Clara, CA). The final cDNA sequence was confirmed by Sanger-based sequencing (Laragen, Inc., Culver City, CA).

**Cell culture and CHO cell expression lines-** CHO-K1 (American Type Culture Collection [ATCC], Manassas, VA) cells were cultured in Ham's F12/DMEM containg glutamine (Corning Incorporated, Corning, NY) supplemented with 10% fetal bovine serum, 100 units/mL Penicillin and 100ug/mL Streptomycin (Lonza) at 37°C in a 5% $CO_2$ tissue culture incubator. The rhGNS expression vector was transfected into CHO-K1 cells using BioT Transfection Reagent (Bioland Scientific, Paramount, CA) and transfected cells were selected by 700 μg/ml G-418 (Sigma-Aldrich, St. Louis, MO) for 14 days, at which point control cells without the vector died. Transfected cells were dissociated by trypsin and stained by propidium iodide (PI; Thermo Scientific, Waltham, MA) for viability and sorted by FACSAria III (BD Biosciences, Franklin Lakes, NJ) with gating based on cell viability (negative for PI staining). Single cells were isolated into a 96-well plate and cultured with medium containing 700 μg/ml G-418 until colonies were formed.

An initial screening was performed by GNS enzyme activity and western blotting with anti c-myc antibody (Thermo Scientific, Waltham, MA) using HyClone serum-free PF CHO media (GE Healthcare Life Sciences, Logan, UT) supplemented with 4 mM L-glutamine, 200 μg/ml G418, from each single colony formed. Stable CHO cell clones were maintained and expanded in culture medium with 300 μg/ml G-418.

For protein production, stable clones were inoculated into roller bottles (Thermo Scientific, Waltham, MA) and grown until confluence, at which time, the medium was replaced to HyClone serum-free PF CHO media (GE Healthcare Life Sciences, Logan, UT) supplemented with 4 mM L-glutamine, 200 μg/ml G418, and nucleosides (10 mg/L each of adenosine, cytosine, guanosine, hypoxanthine, thymidine, and uridine), and secreted rhGNS was daily monitored by both GNS assay and western blot until full-length GNS expression reached a plateau (day 10-15) before harvested for rhGNS purification (FIG. 19).

**Purification of rhGNS-** Following harvest, conditioned media was concentrated using Vivaflow® Crossflow devices with a molecular weight cut-off of 50 kDa (Sartorius Corporation, Bohemia, NY) to 10 % of original volume and the concentrated media was exchanged to artificial cerebrospinal fluid (CSF; 125 mM NaCl, 22.6 mM $NaHCO_3$, 4.4mM dextrose, 1.2 mM $MgSO_4$, 4 mM KCl, 1.4 mM $CaCl_2$, 0.75 mM $Na_2HPO_4$) at 4°C. Approximately 50 mL concentrated media was mixed with 500 μl of c-myc affinity beads (50% slurry; Medical & Biological Laboratories Co., Ltd., Nagoya, Japan) and incubated overnight at 4°C under rotation.

The slurry beads were then loaded onto a 25 mL chromatography column for rhGNS purification. The unbound flow-through was collected and loaded back into the column one more time. The column was washed with artificial CSF (10mL x 3 times). Bound rhGNS was eluted using 3 mL of 0.2 mg/mL c-myc peptide (Medical & Biological Laboratories Co., Ltd., Nagoya, Japan), and concentrated to a final concentration of approximately 1 mg/ml protein by Microcon 50,000 MWCO Centrifugal Filter (Merck Millipore, Billerica, MA).

**Enzymatic activity assays for rhGNS-** A two-step fluorometric measurement of GNS activity was performed. 2.5 μl of media or purified enzyme was incubated with 2.5 μl of 10 mM 4-methylumbelliferyl alpha-*N*-acetylglucosaminide-6-sulfate (4-MU-GNS; Toronto Research Chemicals, Toronto, Canada) in reaction buffer (0.2 M sodium acetate, pH 5.6; 20 mM lead acetate, 0.01% Triton X-100) at 37°C for 1 hour, and the reaction was then stopped by addition of 10 μl of phosphate-citrate buffer (0.4 M $Na_2HPO_4$ / 0.2 M citric-acid buffer, pH 4.7). To release 4-MU fluorophore from the reaction mixture, 5 μl of concentrated rhNAGLU-IGFII conditioned medium (specific activity > 100 nmol/hr/μl; Kan et al 2014) media or 5 μl of purified myc tagged rhNAGLU-IGFII was supplied and incubated for 2h at 37°C. Reactions were quenched by the addition of 50 μl of glycine carbonate buffer, pH 10.5. Fluorescence measurements were obtained using a SpectraMax Paradigm Multi-Mode Microplate Reader (Molecular Devices, Sunnyvale, CA) at excitation and emission wavelengths of 360 nm and 450 nm, respectively. One activity unit of GNS was defined as 1 nmol of 4-MU converted substrate per 24 hour (first step) at 37°C. Protein concentration was determined using Bradford Reagent (Bio-Rad Laboratories, Inc, Irvine, CA). Commercial rhGNS (R&D Systems; Minneapolis, MN) was used as a positive control and 4-MU standard curves were present on all readings to account for differences in read conditions between assays. Detailed assay optimization is described in the Supplemental method sections below.

**SDS-PAGE and Western blotting-** Samples were resolved onto Mini-Protean™ TGX™ 4-16% gels along with Kaleidoscope™ protein standard (Bio-Rad Laboratories, Inc, Irvine, CA) as a molecular weight marker. Protein bands were visualized by staining with Imperial™ protein stain (Thermo Scientific, Waltham, MA) or transferred onto nitrocellulose membranes (Bio-Rad Laboratories, Irvine, CA) for western blotting. After blocking with 5% milk in TBST, membranes were probed with either goat anti-GNS antibody (R&D Systems, Minneapolis, MN) or mouse anti-c-myc antibody (Thermo Scientific, Waltham, MA) overnight at 4°C. After washing, membranes were incubated for 2 hours at room temperature with HRP-conjugated secondary antibodies (Santa Cruz Biotechnology, Inc., Santa Cruz, CA), and developed with the Immobilin™ chemiluminescence kit (Merck Millipore, Billerica, MA) and imaged using the Chemi-Doc™ Imaging System (Bio-Rad Laboratories, Inc, Irvine, CA).

**Glycosylation analysis-** Deglycosylation of rhGNS was performed with two different enzymes: PNGase F and Endo H (New England BioLabs, Ipswich, MA). Approximately 3.5 μg of purified rhGNS underwent glycosidase digestion with either enzyme for one hour at 37°C according to manufacturer instructions. The digestion was then visualized via SDS-PAGE followed by staining with Imperial™ Protein Stain or further by immunostaining with anti-c-myc antibody as described above.

**Composition analysis of N-glycan isolated from rhGNS sample using HPAEC-PAD analysis.** 100ug of purified rhGNS was used for N-glycan isolation using PNGaseF, followed by purification of N-glycans using SPE extraction. Purified N-glycan was hydrolyzed using 2N TFA at 100°C for 4h, acid removed by dry nitrogen flush and finally dissolved in milli-Q water and injected on HPAEC-PAD. Carbo-Pac PA-1 column (4 x 250 mM) was used with NaOH-NaOAc gradient as mobile phase for separation. A standard Man-6-P was used to quantify and assign the retention time.

**Steady-state kinetic analysis -** A Michaelis-Menten kinetics curve was generated when 3 ng/ml rhGNS was incubated for 1 hour at 37°C at 8 different concentrations of 4-MUGNS ranging from 0.2 to 25mM with a 4 hour 4-MU release step under standard reaction conditions. All calculations of $V_{max}$, $k_{cat}$, and $K_m$ were made using GraphPad Prism 6.0 (GraphPad, Inc., La Jolla, CA) by fitting data to Equation 1.

$$\text{Equation (1)} \qquad v = V_{max}*[\text{4-MUGNS}]/(K_m + [\text{4-MUGNS}])$$

In order to determine the optimal GNS activity over a range of pH, we used three buffer conditions to cover the range of pH values (pH 3.8 to 5.8 using acetate/acetic acid buffer (pKa=4.76), and (pH 6.2 to 7.6 using HEPES buffer (pKa=7.5). 2.5 $\mu$L of rhGNS (3 ng/uL) was incubated with 2.5 $\mu$L of 4-MU-GNS (10 mM made with the desired buffer) at 37 °C for 1h. The rest of steps are same as described in Enzymatic activity assays for rhGNS.

**Enzyme Uptake in MPS IIID fibroblasts -** Intracellular rhGNS enzyme uptake was determined using fully-confluent MPS IIID human skin fibroblasts (GM17495, Coriell Institute, Camden, NJ) in DMEM supplement with 1% L-Glutamine, 1% Penicillin Streptomycin solution, and 10% FBS treated with purified rhGNS at 37°C for 4 hours. Cells were then washed with PBS and harvested using TrypLE Select (Thermo Scientific, Waltham, MA) and cell pellets were further washed with HEPES buffer (0.2M, pH 7.2; Sigma-Aldrich, St. Louis, MO) before lysing in GNS Lysis buffer (GNS reaction buffer supplemented with 0.1% Triton X-100 and 0.1mM DTT) by vigorous pipetting followed by incubation on ice for 20 minutes. Samples were then centrifuged at 10,000 x g for 15 minutes, and supernatants were recovered for GNS activity assay as described above. Intracellular GNS enzyme activity is normalized with the protein concentration from the lysed pellet.

rhGNS uptake inhibition was performed by pre-incubating 10 mM mannose 6-phosphate (M6P; Sigma-Aldrich, St. Louis, MO) with MPS IIID human skin fibroblasts (GM17495) for 10 minutes before applying an equal volume of medium containing 40 nM purified rhGNS with the cells and further incubated for 4 hours at 37°C, reaching a final concentration of 20 nM for rhGNS and 5 mM for M6P. Following treatment, intracellular GNS activity in treated cells was measured as described above.

**GAG storage reduction-** GAG storage in cultured MPS IIID human skin fibroblasts (GM05093 and GM17495) and control skin fibroblasts (IMR90 and GM01392) was measured by labeling newly synthesized GAG with $H_2{}^{35}SO_4$. In brief, cells were grown to confluence in 6-well plates supplemented with 25 $\mu$Ci/ml $H_2{}^{35}SO_4$ (Perkin-Elmer, Waltham, MA) in the presence of purified 150 ng/mL rhGNS at 37°C and 5% $CO_2$. After 72 hours of labeling, cells were washed and then harvested by trypsinization and centrifugation. Intracellular GAG was extracted from cell pellets by resuspending and boiling in 85% ethanol supplemented with 1$\mu$g/ml glycogen. The extracted GAG was centrifuged and dissolved in 10% sodium hydroxide and neutralized with 2 M acetic acid and radiolabeled GAG was measured via scintillation counting (Tri-Carb 2800 TR, Perkin-Elmer, Waltham, MA). Radioactive counts per minute were normalized to protein concentration. The estimate of $K_{correction}$ for GAG reduction was further performed at the presence of different concentrations (0, 0.032, 0.16, 0.8, 4 and 20 ng/mL) of rhGNS with MPS IIID human skin fibroblasts (GM17495) as described above. Data were normalized and presented as percentage of cpm/mg protein compared to untreated MPS IIID cells and were best fit to an exponential decay using GraphPad Prism (GraphPad, Inc., La Jolla, CA). Half-maximal concentration for correction was calculated using the pharmacokinetics module ($IC_{50}$) in GraphPad Prism (GraphPad, Inc., La Jolla, CA).

***In vivo* Study using MPS IIID constitutive knockout mouse**

Animal experiments were approved by the Institutional Animal Care and Use Committee at the Los Angeles Biomedical Research Institute at Harbor-UCLA Medical Center, which is accredited by the Association for Assessment and Accreditation of Laboratory Animal Care (AAALAC).

A MPS IIID constitutive knockout mouse model was generated by Taconic Biosciences (Rensselaer, NY) and maintained on an inbred background (C57BL/6), which has been shown to display deficiency of GNS activity and accumulates lysosomal storage[22]. Mutant male MPS IIID mice (*Gns*$^{-/-}$) were mated with heterozygous females (*Gns*$^{+/-}$) females to obtained homozygous affected mice and heterozygous controls. Genotype was determined with primers: MPS IIID F: 5'-GTACTCATTCTTGCAGAAGACCC-3'; MPS IIID R1: 5'-CTTCAGTGGCGTCTACAAAGC -3'; and MPS IIID R2: 5'-AGTGCCACCAAGCAGAACTC -3'.

Neonatal mice in both genders were toe clipped and genotyped at birth. 5 μl sterile-filtered (0.2 μm), purified rhGNS (1 μg/μl) or vehicle was injected in the left. The injection site for intracerebroventricular (ICV) injection was ~2.5 mm between bregma and eyes; 2 mm away laterally from sagittal suture, and 2.5 mm depth.

**Biochemical evaluations in MPS IIID mice with rhGNS ERT**

Tissues were homogenized using a bullet blender (Next Advance, Inc., Troy, NY) with GNS assay buffer containing 0.1% TritonX-100). Homogenized tissues were assayed for GNS, and β-hexosaminidase activity, a secondary marker of lysosomal storage marker by 4-methylumbelliferyl assays. Hexosaminidase is in MPS disease. The catalytic activity of b-hexosaminidase (combined A and B isoforms) was determined by hydrolysis of 4-methylumbelliferyl-N-acetyl-β-glucosaminide (EMD Millipore, Burlington, MA) using 1.25 mM substrate in the incubation mixture. A unit of activity is defined as the release of 1 nmol of 4-methylumbelliferone (4MU) per hour or 24 hour at 37°C.

**Lysosome Purification**

Lysosome fraction from the brains harvested from MPS IIID mutant (*Gns*$^{-/-}$) mice treated with or without rhGNS along with aged matched carrier control (*Gns*$^{-/-}$) mice were enriched using the Lysosome Enrichment Kit for Tissue and Cultured Cells (Thermo Scientific, Waltham, MA) following manufacturer's instruction. Isolated lysosomes were lysed GNS lysis buffer before enzyme activity assays and westen blots.

**Statistical analysis**

Statistical analysis for the level of significance was done by paired t-test. Figures were made using GraphPad Prism 7.0 (GraphPad, Inc., La Jolla, CA)

**Supplemental Methods**

**Sulfatase Assay-** An absorbance-based measurement of GNS sulfatase activity was adapted from the protocol provided by R&D systems with modifications using a 96-well plate format. Each well containing 10μl of 2 μg/ml rhGNS in reaction buffer 1 (200 mM sodium acetate, pH 5.6, 0.01% Triton X-100) was incubated with 40μl of 2.5mM para-nitrocatechol sulfate (PNCS) for 1-4 hours at 37°C. Following incubation, 50 μl of 0.2M NaOH was added to stop reactions, and plates were then centrifuged at 1000x g for lmin. Absorbance was measured at 510nm using the Synergy Neo Multi-Mode microplate reader (BioTek). Each reaction condition was carried out in triplicate.

**Assay Optimization Sulfatase Assay**

Two assays were used to assess GNS activity, the first of which is a colorimetric assay utilized para-nitrocatechol-sulfate (PNCS), an artificial substrate of non-specific sulfatase activity. Cleavage of the sulfate group occurs in a one-step reaction producing para-nitrocatechol (PNC), yielding maximum absorbance at 510nm under basic conditions and referred to as the 'Sulfatase Assay'. Individual buffer components were optimized in independent experiments (FIG. 23). Enzyme activity remained relatively stable up to levels of 250 mM NaCl, whereas acetate concentration showed very minor effects. Experiments with other common biological buffers demonstrated that the enzyme is completely inhibited in the presence of free phosphate, sulfate, or citrate groups. Therefore, purification and other assay buffer conditions were similarly optimized to avoid addition of these components. Addition of the detergent Triton X-100 (Sigma) significantly increased reproducibility and showed maximal enzyme specific activity at low concentrations (0.01%). In order to test activity over a range of pH, we use three buffer conditions to cover the range of pH values: acetate (pKa=4.76; range=3.2-5.6), MES (pKa=6.16; range=5.5-6.7), and HEPES (pKa=7.55; range=6.8-8.2).

**Assay Optimization GNS Assay**

While the Sulfatase Assay provided a cheap and direct measurement of sulfatase activity, it is worth noting that PNCS is a common substrate of *several* sulfatases and therefore lacks substrate specificity. To address this, we utilized a coupled, fluorometric assay using 4-MU-GNS, and more closely mimics the natural substrate of the GNS enzyme, which we term the "GNS Assay". The GNS activity assay was completed in two parts, where the first step involved incubation with 4-MUGNS, producing 4-methylumbelliferyl-2-acetamido-2-deoxy-alpha-D-glucopyranoside, and the second step involved addition of alpha-*N*-acetylglucosaminidase (NAGLU) to release 4-MU, which exhibits an excitation/emission profile of 360/450nm. The GNS assay was first developed over twenty years ago primarily for the diagnosis of MPSIIID patients; therefore the assay was carried out over a prolonged 24-48 hour incubation period due to the low concentration of the enzyme present in cell lysates of healthy individuals.

Because the assay requires two sequential reaction steps, it was essential that the second step enzyme was not limiting for the amount of product produced from the first (GNS) reaction. Therefore, highly concentrated stocks of recombinant human NAGLU conditioned media were produced. We then used this concentrated media for optimization. We found that using 5μL of the 20X concentrated rhNAGLU stock after 30 minutes we were able to achieve complete turnover of 4-methylumbelliferyl-2-acetamido-2-deoxy-alpha-D-glucopyranoside produced by 20 ng of rhGNS after one hour reaction time (>250 nmol 4-MU).

**Results**

**Characterization of rhGNS expressed in a CHO cell line**

In this study, a total of 50 clones were initially isolated and 12 were positive for rhGNS secretion confirmed by GNS assay (FIG. 19). Three highest expression clones (TCB469 B2, TCB470 A1, and TCB470 A5) were identified, and clone TCB469 B2 was used for subsequent purification in this study. rhGNS secretion into the media was monitored using the GNS assay and western blot daily until full-length GNS expression reached a maximum (day 10-15, FIG. 19), at which point the conditioned medium was harvested for rhGNS purification. The purification Table is shown in Table 2. The full-length of the engineered rhGNS fusion protein in this study is 579 amino acids with a deduced molecular weight of 64.8 kDa with pI of 8.60 and the mature rhGNS is 535 amino acid with the first 44 a.a be cleaved. The purified protein detected by western blot and SDS-PAGE appeared be approximately 80 kDa (FIG. 12A and 12B), likely due to *N*-glycosylation at one or more of the 13 potential N-glycosylation sites, however, which sites were really been used is still unclear.

**Table 2: Purification of rhGNS produced in CHO cells.**

| | Volume (ml) | Protein (μg) | Total activity (units)* | Specific Activity (units/mg protein) | Yield (%) |
|---|---|---|---|---|---|
| PF CHO Media | 1,450 | 330,165 | 108,998 | 330 | - |
| Concentrated Media | 110 | 304,920 | 307,826 | 1,010 | 100 |
| myc Elution | 3 | 186 | 26,408 | 142,263 | 8.1 |
| *1 unit = 1 nmol converted substrate per 24 h at 37°C. The substrate is 4-metylumbelliferyl *N*-acetyl-glucosamine-6-sulfate. | | | | | |

To further understand the glycosylation pattern of rhGNS, the purified enzyme was treated with endoglycosidases PNGase F or Endo H to remove attached carbohydrate molecules. PNGase F hydrolyzes almost all types of N-linked oligosaccharides, whereas Endo H cleaves mannose rich N-linked oligosaccharides within the chitobiose core. The rhGNS was sensitive to digestion with both PNGase F and Endo H, making molecular weight shifts from 80 kDa t0 58 kDa, most likely correspond to the mature GNS without the first 44 a.a. It suggests the protein was N-glycosylated with high-mannose residues. (FIG. 12C). We have used high performance anion exchange chromatography with pulsed amperometric detection (HPAEC-PAD) to determine that our rhGNS is modified by mannonse-6-phosphate and the amount of Man-6-P present in 100ug of protein is 0.87ug (FIG. 12D).

A Native polyacrylamide gel electrophoresis was performed to resolve rhGNS in a non-denaturing environment; the molecular weight was estimated to be ~160-200 kDa, indicating a dimeric structure (FIG. 21). LC/MS/MS analysis of trypsin digested rhGNS further confirmed the identity of rhGNS (Tables 3-4).

**Table 3:** LC/MS/MS analysis of trypsin digested rhGNS further confirmed the identity of rhGNS. Analysis of rhGNS from myc affinity purification by LC/MS/MS analysis determined two major contaminant proteins are Histone H4 and Isoform 3 of T-complex protein 1 subunit eta.

| Description | Area | Normalized to GNS intensity (%) | Coverage | # PSM | # AAs | MW [kDa] |
|---|---|---|---|---|---|---|
| glucosamine (N-acetyl)-6-sulfatase (GNS) | 5.48E10 | 100 | 49.64 | 154 | 552 | 62.1 |
| Histone H4 OS=Homo sapiens | 2.12E9 | 4 | 51.46 | 21 | 103 | 11.4 |

(continued)

| Description | Area | Normalized to GNS intensity (%) | Coverage | # PSM | # AAs | MW [kDa] |
|---|---|---|---|---|---|---|
| Isoform 3 of T-complex protein 1 subunit eta | 2.80E9 | 5 | 30.06 | 33 | 499 | 54.8 |

**Table 4:** Analysis of rhNAGLU from myc affinity purification by LC/MS/MS analysis determined the major contaminant protein is Histone H4.

| Description | Area | Normalized to GNS intensity (%) | Coverage | # PSM | # AAs | MW [kDa] |
|---|---|---|---|---|---|---|
| Alpha-N-acetylgluco saminidase OS=Homo sapiens GN=NAGLU | 2.9E10 | 100 | 50.87 | 246 | 743 | 82.2 |
| Histone H4 OS=Homo sapiens | 4.21E8 | 1.4 | 50.49 | 15 | 103 | 11.4 |

**Steady State kinetic analysis of rhGNS using 4-MU-GNS substrate.** To determine the steady state kinetic constants of rhGNS, we perform the GNS activity assay with increasing amounts of 4-MU-GNS and generated a Michaelis-Menten kinetics curve (FIG. 13A). The calculated $K_m$ was 4.0 mM, and the estimated $k_{cat}$ was determined to be 336,000 units/mg. The optimal pH for lysosomal hydrolase activity is typically observed in the acidic range. rhGNS purified in this study exhibited a peak activity at pH 5.6 (FIG. 13B), with activity at neutral pH (pH 7) approximately 10-fold lower across both assays (FIG. 22).

**Temperature effect on rhGNS activity and stability of rhGNS at 4°C.** To determine how sensitive rhGNS to the temperature, we assayed rhGNS over a range of temperatures (23 °C to 65 °C) and found rhGNS has a very wide optimum temperature range up to 65°C. GNS activity is 1.5 fold at 37 °C comparing to room temperature (23 °C) and is almost double at 49°C comparing to room temperature. Overall, GNS has good enzymatic activity at body temperature (37 °C), with doubled enzyme activity up to 49°C and remained active up to 65°C (FIG. 13C). The enzyme stability assays also showed that purified rhGNS was also found to be stable for over one month at 4°C in artificial CSF and still had greater than 80% of initial enzyme activity (FIG. 13D).

Interestingly, while generating biological buffers, we identified that both citrate and phosphate completely inhibited rhGNS enzyme activity (FIG. 23).

**rhGNS uptake into MPS IIID fibroblasts via M6P receptors and cleared accumulated GAGs**

MPS IIID human fibroblasts were incubated for 4 hours with varying concentrations of rhGNS (concentration) and the intracellular rhGNS activity was measured (FIG. 14A). Maximal uptake reached ~30% of GNS activity in wild-type fibroblasts. The uptake of rhGNS into MPS IIID human fibroblasts was inhibited by the addition of mannose 6-phosphate (M6P) at the final concentration of 5 mM, thus suggesting both that rhGNS is mannose 6-phosphorylated and that intracellular rhGNS uptake is M6P receptor-mediated.

We next assessed whether the rhGNS taken up by the MPS IIID fibroblasts was able to restore their GAG degradation using a $^{35}S$ incorporation assay. Two different MPS IIID fibroblast cell lines, GM17495 and GM17495 accumulated consistently higher levels of $^{35}S$-labeled GAGs after 72-hour labeling compared to wild type cell lines, IMR90 and GM01392. At the presence of 150 ng/ml purified rhGNS during labeling, the total intracellular $^{35}S$-labeled GAGs in these MPS IIID lines were restored to the wild type level (FIG. 14B). This result suggests that rhGNS is able to restore the GAG degradation pathway, the primary storage substrate that accumulates in MPS IIID disease. We further titrated rhGNS in the labeling media and observed as low as 0.8 ng/mL (10 pM) rhGNS was sufficient to reduce GAG storage to levels similar to WT. The calculated concentration required for half-maximal correction was 0.44 ng/mL (5.5 pM). (FIG. 14C)

**Intracerebroventricular enzyme replacement in MPS IIID mice with rhGNS**

We next performed Intracerebroventricular (ICV) injection of rhGNS into newborn GNS knockout model (*Gns*$^{-/-}$) to assess its distribution along with efficacy to lower the secondary marker *in vivo.* The injection site indicates by X and circle as shown in FIG. 15A. We were able to detect statistically significant return of GNS activity toward to wild-type levels 24 hours after dosing with 5 μg purified rhGNS (FIG. 15B). Deficiency of GNS in MPS IIID mouse brains is accompanied by increased activity of other lysosomal enzymes, including NAGLU and β-hexosaminidase. (FIG. 15C-E) showed the activity of β-hexosaminidase in the whole brain homogenate of the mutant brain to be more than 30% that in the age-matched heterozygote controls. After the treatment of ICV ERT with rhGNS, we observed significant β-hexosaminidase activity reduction after 3 days and 7 days in the mouse brain treated with from birth, single dose, ICV ERT (FIG. 15C, D), and this reduction effect was diminished after 14 days of initial treatment (FIG. 15E).

We further looked into the distribution of rhGNS by cutting the treated brain into 3 sections, where the injection site is in section 2 (FIG. 16A). One day post-injection, we were able to detect statistically significant increase of GNS activity throughout the entire neonatal brain in all 3 sections (FIG. 16B-D). We also looked into the *in vivo* half-life of rhGNS in MPS IIID mice brain by assaying ICV-ERT post-treated brain from 1-3 days and measured the GNS enzyme activity. The estimate the *in vivo* half-life of rhGNS in our study was found it to be ~1.1 day (FIG. 17). Lysosomal fractioning from 1-day post ICV ERT brains was enriched by a discontinuous density gradient centrifugation. The GNS enzyme activity from the ICV ERT treated. rhGNS was shown to target to lysosomes in post one day injection into MPS IIID mice. GNS returned to carrier levels in knock out mice lysosomes (FIG. 18A) and western blot show that the lysosome enriched fraction has higher level of LAMP1, which is a lysosomal marker (FIG. 18B).

In the current study we report the production of rhGNS and characterize the activity *in vitro* and *in vivo.*

We found the yield of active purified recombinant protein was low at 3.3% and was likely N-glycosylated with high-mannose residues (FIG. 12C). LC/MS/MS of trypsin digested rhGNS confirmed the identity of the protein. We characterized the kinetics (Km 4mM, $k_{cat}$ 14,000nmol) and the optimal pH (5.6), found citrate and phosphate inhibited rhGNS activity, thermostability up to 49°C and stability for a month at 4°C in artificial CSF. These properties bode well for creating a treatment. Our studies also demonstrated that rhGNS is taken up by fibroblasts *in vitro* (FIG. 15), catabolizes GAG with the concentration for half maximal correction of 0.44ng/mL (FIG. 15B). In this very first *in vivo* ERT study in MPS IIID mouse model, we demonstrated that GNS activity could be returned and lysosomal storage markers reduced (FIG. 16) while this effect is comparable across the brain (FIG. 17). While the half-life of the rhGNS is approximately 24h the β-hexosaminidase was still normalized at 7 days (FIG. 18). rhGNS was definitively shown to reach the lysosomes *in vivo.*

We have now described the initial development and characterization of an ERT for MPS IIID in which rhGNS is generated in CHO cells. We have produced rhGNS ~100 μg per 1500 mL media in CHO cells (specific activity of ~100,000 activity units/mg), demonstrated maximal enzymatic activity at pH 5.6 (low activity at neutral pH), which is closer to lysosomal pH, demonstrated good enzymatic activity at 37°C and showed it was stable for over one month at 4°C in artificial cerebrospinal fluid storage buffer. Additionally, we have demonstrated cellular uptake of rhGNS in MPS IIID fibroblasts, and confirmed GAG clearance (33-65%) in MPS IIID fibroblasts treated with rhGNS. Intracerebroventricular injection of rhGNS in neonatal mice at day 1 demonstrated return of GNS activity and areduction of lysosomal storage markers NAGLU and β-hexosaminidase throughout the brain. The calculated half-life of rhGNS was found to be 1.1 day although hexosaminidase remained at normal levels upto 7 days. We ultimately confirmed that rhGNS was found to localize in lysosomes of treated mice which had normalized levels of lysosomal storage markers. Our results therefore demonstrate the clear potential of rhGNS as an ERT for MPS IIID in preparation for further IND enabling studies.

## Claims

1. A method for preparing a composition suitable for treating mucopolysaccharidosis type IIID (MPS IIID) in a human patient in need thereof, comprising testing the sulfatase activity of a recombinant polypeptide in a testing buffer comprising less than 500 mM sodium, less than 100 mM citrate, less than 100 mM phosphate, less than 100 mM sulfate, and at least 0.005% Triton X-100, and providing the recombinant polypeptide in an artificial cerebrospinal fluid to prepare the composition, wherein the recombinant polypeptide comprises the amino acid sequence of SEQ ID NO: 1 or an amino acid sequence (a) having at least 95% sequence identity to SEQ ID NO: 1 and (b) having the enzymatic activity of human acetylglucosamine-6-sulfatase (GNS).

2. A method of testing the sulfatase activity of a recombinant polypeptide, comprising reacting the recombinant polypeptide with para-nitrocatechol sulfate (PNCS) in a testing buffer comprising less than 500 mM sodium, less than 100 mM citrate, less than 100 mM phosphate, less than 100 mM sulfate, and at least 0.005% Triton X-100, wherein the recombinant polypeptide comprises the amino acid sequence of SEQ ID NO: 1 or an amino acid sequence (a) having at least 95% sequence identity to SEQ ID NO: 1 and (b) having the enzymatic activity of human acetylglucosamine-6-sulfatase (GNS).

3. The method of claim 2, further comprising stopping the reaction with a base.

4. The method of claim 2, further comprising stopping the reaction with a phosphate-citrate buffer.

5. The method of claim 4, wherein the phosphate-citrate buffer comprises at least 0.4 M $Na_2HPO_4$ in at least 0.2 M citric acid.

6. The method of any one of the preceding claims, wherein
the testing buffer has a pH of about 5 to about 6.

7. The method of any one of the preceding claims, wherein the testing buffer comprises less than 250 mM sodium, less than 50 mM citrate, less than 50 mM phosphate, less than 50 mM sulfate, and at least 0.01% Triton X-100.

8. The method of any one of the preceding claims, wherein the testing buffer comprises about 200 mM sodium acetate and about 0.01% Triton X-100, at about pH 5.6.

9. The method of any one of the preceding claims, wherein the artificial cerebrospinal fluid has a pH of about 6 to 7.5.

10. The method of any one of the preceding claims, wherein the recombinant polypeptide comprises the amino acid sequence of SEQ ID NO: 2, 5 or 6.

## Patentansprüche

1. Verfahren zur Herstellung einer Zusammensetzung, die zur Behandlung von Mucopolysaccharidose Typ IIID (MPS IIID) in einem menschlichen Patienten, der diese benötigt, geeignet ist, umfassend das Testen der Sulfatase-Aktivität eines rekombinanten Polypeptids in einem Testpuffer, der weniger als 500 mM Natrium, weniger als 100 mM Citrat, weniger als 100 mM Phosphat, weniger als 100 mM Sulfat und mindestens 0,005 % Triton X-100 umfasst, und das Bereitstellen des rekombinanten Polypeptids in einer künstlichen zerebrospinalen Flüssigkeit, um die Zusammensetzung herzustellen, wobei das rekombinante Polypeptid die Aminosäuresequenz von SEQ ID NO: 1 oder eine Aminosäuresequenz umfasst, die (a) mindestens 95 % Sequenzidentität mit SEQ ID NO: 1 aufweist und (b) die enzymatische Aktivität von humaner Acetylglucosamin-6-Sulfatase (GNS) besitzt.

2. Verfahren zum Testen der Sulfatase-Aktivität eines rekombinanten Polypeptids, umfassend das Reagieren des rekombinanten Polypeptids mit para-Nitrocatecholsulfat (PNCS) in einem Testpuffer, der weniger als 500 mM Natrium, weniger als 100 mM Citrat, weniger als 100 mM Phosphat, weniger als 100 mM Sulfat und mindestens 0,005 % Triton X-100 umfasst, wobei das rekombinante Polypeptid die Aminosäuresequenz von SEQ ID NO: 1 oder eine Aminosäuresequenz umfasst, die (a) mindestens 95 % Sequenzidentität mit SEQ ID NO: 1 aufweist und (b) die enzymatische Aktivität von humaner Acetylglucosamin-6-Sulfatase (GNS) besitzt.

3. Verfahren nach Anspruch 2, ferner umfassend das Anhalten der Reaktion mit einer Base.

**4.** Verfahren nach Anspruch 2, ferner umfassend das Anhalten der Reaktion mit einem Phosphat-Citrat-Puffer.

**5.** Verfahren nach Anspruch 4, wobei der Phosphat-Citrat-Puffer mindestens 0,4 M $Na_2HPO_4$ in mindestens 0,2 M Zitronensäure umfasst.

**6.** Verfahren nach einem der vorstehenden Ansprüche, wobei der Testpuffer einen pH-Wert von etwa 5 bis etwa 6 aufweist.

**7.** Verfahren nach einem der vorstehenden Ansprüche, wobei der Testpuffer weniger als 250 mM Natrium, weniger als 50 mM Citrat, weniger als 50 mM Phosphat, weniger als 50 mM Sulfat und mindestens 0,01 % Triton X-100 umfasst.

**8.** Verfahren nach einem der vorstehenden Ansprüche, wobei der Testpuffer etwa 200 mM Natriumacetat und etwa 0,01 % Triton X-100 bei einem pH-Wert von etwa 5,6 umfasst.

**9.** Verfahren nach einem der vorstehenden Ansprüche, wobei die künstliche zerebrospinale Flüssigkeit einen pH-Wert von etwa 6 bis 7,5 aufweist.

**10.** Verfahren nach einem der vorstehenden Ansprüche, wobei das rekombinante Polypeptid die Aminosäuresequenz von SEQ ID NO: 2, 5 oder 6 umfasst.

**Revendications**

**1.** Procédé de préparation d'une composition appropriée pour traiter la mucopolysaccharidose de type IIID (MPS IIID) chez un patient humain en ayant besoin, comprenant le test de l'activité sulfatase d'un polypeptide recombinant dans un tampon de test comprenant moins de 500 mM de sodium, moins de 100 mM de citrate, moins de 100 mM de phosphate, moins de 100 mM de sulfate et au moins 0,005% de Triton X-100, et la fourniture du polypeptide recombinant dans un liquide céphalo-rachidien artificiel pour préparer la composition, dans lequel le polypeptide recombinant comprend la séquence d'acides aminés de SEQ ID NO: 1 ou une séquence d'acides aminés (a) ayant au moins 95% d'identité de séquence avec SEQ ID NO: 1 et (b) ayant l'activité enzymatique de l'acétylglucosamine-6-sulfatase humaine (GNS).

**2.** Procédé de test de l'activité sulfatase d'un polypeptide recombinant, comprenant la réaction du polypeptide recombinant avec du sulfate de para-nitrocatéchol (PNCS) dans un tampon de test comprenant moins de 500 mM de sodium, moins de 100 mM de citrate, moins de 100 mM de phosphate, moins de 100 mM de sulfate et au moins 0,005% de Triton X-100, dans lequel le polypeptide recombinant comprend la séquence d'acides aminés de SEQ ID NO: 1 ou une séquence d'acides aminés (a) ayant au moins 95% d'identité de séquence avec SEQ ID NO: 1 et (b) ayant l'activité enzymatique de l'acétylglucosamine-6-sulfatase humaine (GNS).

**3.** Procédé selon la revendication 2, comprenant en outre l'arrêt de la réaction avec une base.

**4.** Procédé selon la revendication 2, comprenant en outre l'arrêt de la réaction avec un tampon phosphate-citrate.

**5.** Procédé selon la revendication 4, dans lequel le tampon phosphate-citrate comprend au moins 0,4 M de $Na_2HPO_4$ dans au moins 0,2 M d'acide citrique.

**6.** Procédé selon l'une quelconque des revendications précédentes, dans lequel le tampon de test a un pH d'environ 5 à environ 6.

**7.** Procédé selon l'une quelconque des revendications précédentes, dans lequel le tampon de test comprend moins de 250 mM de sodium, moins de 50 mM de citrate, moins de 50 mM de phosphate, moins de 50 mM de sulfate et au moins 0,01% de Triton X-100.

**8.** Procédé selon l'une quelconque des revendications précédentes, dans lequel le tampon de test comprend environ 200 mM d'acétate de sodium et environ 0,01% de Triton X-100, à un pH d'environ 5,6.

**9.** Procédé selon l'une quelconque des revendications précédentes, dans lequel le liquide céphalo-rachidien artificiel a un pH d'environ 6 à 7,5.

**10.** Procédé selon l'une quelconque des revendications précédentes, dans lequel le polypeptide recombinant comprend la séquence d'acides aminés de SEQ ID NO: 2, 5 ou 6.

```
  1 MRLLPLAPGR LRRGSPRHLP SCSPALLLLV LGGCLGVFGV AAGTRRPNVV
 51 LLLTDDQDEV LGGMTPLKKT KALIGEMGMT FSSAYVPSAL CCPSRASILT
101 GKYPHNHHVV NNTLEGNCSS KSWQKIQEPN TFPAILRSMC GYQTFFAGKY
151 LNEYGAPDAG GLEHVPLGWS YWYALEKNSK YYNYTLSING KARKHGENYS
201 VDYLTDVLAN VSLDFLDYKS NFEPFFMMIA TPAPHSPWTA APQYQKAFQN
251 VFAPRNKNFN IHGTNKHWLI RQAKTPMTNS SIQFLDNAFR KRWQTLLSVD
301 DLVEKLVKRL EFTGELNNTY IFYTSDNGYH TGQFSLPIDK RQLYEFDIKV
351 PLLVRGPGIK PNQTSKMLVA NIDLGPTILD IAGYDLNKTQ MDGMSLLPIL
401 RGASNLTWRS DVLVEYQGEG RNVTDPTCPS LSPGVSQCFP DCVCEDAYNN
451 TYACVRTMSA LWNLQYCEFD DQEVFVEVYN LTADPDQITN IAKTIDPELL
501 GKMNYRLMML QSCSGPTCRT PGVFDPGYRF DPRLMFSNRG SVRTRRFSKH
551 LL
```

Sequence of GNS (SEQ ID NO: 1)

**FIG. 1**

```
  1 MRLLPLAPGR LRRGSPRHLP SCSPALLLLV LGGCLGVFGV AAGTRRPNVV
 51 LLLTDDQDEV LGGMTPLKKT KALIGEMGMT FSSAYVPSAL CCPSRASILT
101 GKYPHNHHVV NNTLEGNCSS KSWQKIQEPN TFPAILRSMC GYQTFFAGKY
151 LNEYGAPDAG GLEHVPLGWS YWYALEKNSK YYNYTLSING KARKHGENYS
201 VDYLTDVLAN VSLDFLDYKS NFEPFFMMIA TPAPHSPWTA APQYQKAFQN
251 VFAPRNKNFN IHGTNKHWLI RQAKTPMTNS SIQFLDNAFR KRWQTLLSVD
301 DLVEKLVKRL EFTGELNNTY IFYTSDNGYH TGQFSLPIDK RQLYEFDIKV
351 PLLVRGPGIK PNQTSKMLVA NIDLGPTILD IAGYDLNKTQ MDGMSLLPIL
401 RGASNLTWRS DVLVEYQGEG RNVTDPTCPS LSPGVSQCFP DCVCEDAYNN
451 TYACVRTMSA LWNLQYCEFD DQEVFVEVYN LTADPDQITN IAKTIDPELL
501 GKMNYRLMML QSCSGPTCRT PGVFDPGYRF DPRLMFSNRG SVRTRRFSKH
551 LLGGGGSGGG GSEQKLISEE DL
```

Sequence of rhGNS (SEQ ID NO: 2)

# FIG. 2A

1 MRLLPLAPGR LRRGSPRHLP SCSPALLLLV LGGCLGVFGV AAGTRRPNVV

51 LLLTDDQDEV LGGMTPLKKT KALIGEMGMT FSSAYVPSAL CCPSRASILT

101 GKYPHNHHVV NNTLEGNCSS KSWQKIQEPN TFPAILRSMC GYQTFFAGKY

151 LNEYGAPDAG GLEHVPLGWS YWYALEKNSK YYNYTLSING KARKHGENYS

201 VDYLTDVLAN VSLDFLDYKS NFEPFFMMIA TPAPHSPWTA APQYQKAFQN

251 VFAPRNKNFN IHGTNKHWLI RQAKTPMTNS SIQFLDNAFR KRWQTLLSVD

301 DLVEKLVKRL EFTGELNNTY IFYTSDNGYH TGQFSLPIDK RQLYEFDIKV

351 PLLVRGPGIK PNQTSKMLVA NIDLGPTILD IAGYDLNKTQ MDGMSLLPIL

401 RGASNLTWRS DVLVEYQGEG RNVTDPTCPS LSPGVSQCFP DCVCEDAYNN

451 TYACVRTMSA LWNLQYCEFD DQEVFVEVYN LTADPDQITN IAKTIDPELL

501 GKMNYRLMML QSCSGPTCRT PGVFDPGYRF DPRLMFSNRG SVRTRRFSKH

551 LLGG**ENLYFQ** GGGSGGG GS***EQKLISEE DL***

Sequence of a rhGNS variant (SEQ ID NO: 5)

# FIG. 2B

```
  1 MRLLPLAPGR LRRGSPRHLP SCSPALLLLV LGGCLGVFGV AAGTRRPNVV

 51 LLLTDDQDEV LGGMTPLKKT KALIGEMGMT FSSAYVPSAL CCPSRASILT

101 GKYPHNHHVV NNTLEGNCSS KSWQKIQEPN TFPAILRSMC GYQTFFAGKY

151 LNEYGAPDAG GLEHVPLGWS YWYALEKNSK YYNYTLSING KARKHGENYS

201 VDYLTDVLAN VSLDFLDYKS NFEPFFMMIA TPAPHSPWTA APQYQKAFQN

251 VFAPRNKNFN IHGTNKHWLI RQAKTPMTNS SIQFLDNAFR KRWQTLLSVD

301 DLVEKLVKRL EFTGELNNTY IFYTSDNGYH TGQFSLPIDK RQLYEFDIKV

351 PLLVRGPGIK PNQTSKMLVA NIDLGPTILD IAGYDLNKTQ MDGMSLLPIL

401 RGASNLTWRS DVLVEYQGEG RNVTDPTCPS LSPGVSQCFP DCVCEDAYNN

451 TYACVRTMSA LWNLQYCEFD DQEVFVEVYN LTADPDQITN IAKTIDPELL

501 GKMNYRLMML QSCSGPTCRT PGVFDPGYRF DPRLMFSNRG SVRTRRFSKH

551 LLGGENLYFQ
```

Sequence of rhGNS variant after removal of c-myc tag (SEQ ID NO: 6)

# FIG. 2C

```
   1 ATGCGACTGC TGCCCCTGGC TCCCGGTAGA CTGCGAAGAG GTTCACCACG ACATCTGCCA
  61 AGTTGCTCCC CCGCCCTGCT GCTGCTGGTG CTGGGCGGAT GCCTGGGAGT CTTTGGAGTG
 121 GCAGCTGGCA CACGGAGGCC AAACGTGGTC CTGCTGCTGA CTGACGATCA GGACGAGGTG
 181 CTGGGGGGTA TGACCCCCCT GAAGAAAACA AAGGCCCTGA TCGGTGAAAT GGGCATGACA
 241 TTCAGCTCCG CATACGTGCC TAGCGCCCTG TGCTGTCCAA GCCGGGCTTC CATTCTGACT
 301 GGGAAATATC CACACAATCA CCATGTGGTC AACAATACCC TGGAGGGCAA CTGCTCTAGT
 361 AAGTCTTGGC AGAAAATCCA GGAACCTAAT ACATTTCCAG CCATTCTGAG GAGTATGTGC
 421 GGTTACCAGA CTTTCTTTGC TGGCAAGTAC CTGAACGAGT ATGGAGCTCC AGATGCAGGA
 481 GGACTGGAAC ACGTGCCACT GGGATGGTCC TACTGGTATG CCCTGGAGAA GAACAGCAAA
 541 TACTATAACT ACACCCTGTC TATCAATGGA AAGGCTAGAA AACATGGGGA AAACTACTCT
 601 GTGGACTATC TGACAGATGT CCTGGCAAAT GTGAGTCTGG ACTTTCTGGA TTACAAGTCA
 661 AACTTCGAGC CCTTCTTTAT GATGATTGCC ACTCCAGCTC CTCACTCTCC CTGGACCGCA
 721 GCACCACAGT ATCAGAAGGC TTTCCAGAAC GTGTTCGCAC CTAGGAACAA AAATTTCAAC
 781 ATCCACGGAA CCAACAAGCA TTGGCTGATT AGACAGGCAA AAACCCCAAT GACAAATTCA
 841 AGCATCCAGT TCCTGGACAA CGCCTTTCGA AAGCGGTGGC AGACCCTGCT GAGCGTGGAC
 901 GATCTGGTGG AGAAGCTGGT CAAACGCCTG GAGTTTACAG GGGAACTGAA CAACACTTAC
 961 ATTTTCTATA CCAGCGATAA CGGATACCAT ACTGGGCAGT TTTCCCTGCC CATCGACAAG
1021 CGACAGCTGT ATGAGTTCGA TATCAAGGTG CCCCTGCTGG TCCGGGGACC AGGGATCAAG
1081 CCTAATCAGA CTAGTAAAAT GCTGGTGGCT AACATTGACC TGGGGCCCAC CATCCTGGAC
1141 ATTGCAGGTT ACGATCTGAA CAAGACACAG ATGGATGGCA TGTCCCTGCT GCCTATCCTG
1201 CGGGGAGCCT CAAATCTGAC CTGGAGGAGC GACGTGCTGG TCGAGTATCA GGGTGAAGGC
1261 CGCAACGTGA CTGATCCAAC CTGCCCCTCC CTGTCTCCAG GCGTGTCACA GTGTTTTCCT
1321 GACTGCGTCT GTGAGGATGC CTACAACAAT ACCTATGCTT GCGTGCGAAC AATGAGCGCC
1381 CTGTGGAACC TGCAGTACTG TGAGTTCGAC GATCAGGAGG TGTTTGTCGA AGTGTATAAT
1441 CTGACAGCAG ACCCTGATCA GATCACAAAC ATTGCCAAGA CTATCGACCC AGAGCTGCTG
1501 GGGAAAATGA ATTACAGACT GATGATGCTG CAGAGTTGCT CAGGTCCTAC ATGTCGCACT
1561 CCAGGCGTGT TCGACCCCGG CTATAGGTTT GATCCTAGAC TGATGTTCTC TAACCGCGGA
1621 AGTGTCCGAA CCAGACGCTT CTCCAAGCAT CTGCTG
```

Sequence of rhGNS cDNA (SEQ ID NO: 3)

# FIG. 3

```
   1 ATGCGGCTCC TGCCTCTAGC CCCAGGTCGG CTCCGGCGGG GCAGCCCCCG CCACCTGCCC
  61 TCCTGCAGCC CAGCGCTGCT ACTGCTGGTG CTGGGCGGCT GCCTGGGGGT CTTCGGGGTG
 121 GCTGCGGGAA CCCGGAGGCC CAACGTGGTG CTGCTCCTCA CGGACGACCA GGACGAAGTG
 181 CTCGGCGGCA TGACACCGCT AAAGAAAACC AAAGCTCTCA TCGGAGAGAT GGGGATGACT
 241 TTTTCCAGTG CTTATGTGCC AAGTGCTCTC TGCTGCCCCA GCAGAGCCAG TATCCTGACA
 301 GGAAAGTACC CACATAATCA TCACGTTGTG AACAACACTC TGGAGGGGAA CTGCAGTAGT
 361 AAGTCCTGGC AGAAGATCCA AGAACCAAAT ACTTTCCCAG CAATTCTCAG ATCAATGTGT
 421 GGTTATCAGA CCTTTTTTGC AGGGAAATAT TTAAATGAGT ACGGAGCCCC AGATGCAGGT
 481 GGACTAGAAC ACGTTCCTCT GGGTTGGAGT TACTGGTATG CCTTGGAAAA GAATTCTAAG
 541 TATTATAATT ACACCCTGTC TATCAATGGG AAGGCACGGA AGCATGGTGA AAACTATAGT
 601 GTGGACTACC TGACAGATGT TTTGGCTAAT GTCTCCTTGG ACTTTCTGGA CTACAAGTCC
 661 AACTTTGAGC CCTTCTTCAT GATGATCGCC ACTCCAGCGC CTCATTCGCC TTGGACAGCT
 721 GCACCTCAGT ACCAGAAGGC TTTCCAGAAT GTCTTTGCAC CAAGAAACAA GAACTTCAAC
 781 ATCCATGGAA CGAACAAGCA CTGGTTAATT AGGCAAGCCA AGACTCCAAT GACTAATTCT
 841 TCAATACAGT TTTTAGATAA TGCATTTAGG AAAAGGTGGC AAACTCTCCT CTCAGTTGAT
 901 GACCTTGTGG AGAAACTGGT CAAGAGGCTG GAGTTCACTG GGGAGCTCAA CAACACTTAC
 961 ATCTTCTATA CCTCAGACAA TGGCTATCAC ACAGGACAGT TTTCCTTGCC AATAGACAAG
1021 AGACAGCTGT ATGAGTTTGA TATCAAAGTT CCACTGTTGG TTCGAGGACC TGGGATCAAA
1081 CCAAATCAGA CAAGCAAGAT GCTGGTTGCC AACATTGACT TGGGTCCTAC TATTTTGGAC
1141 ATTGCTGGCT ACGACCTAAA TAAGACACAG ATGGATGGGA TGTCCTTATT GCCCATTTTG
1201 AGAGGTGCCA GTAACTTGAC CTGGCGATCA GATGTCCTGG TGGAATACCA AGGAGAAGGC
1261 CGTAACGTCA CTGACCCAAC ATGCCCTTCC CTGAGTCCTG GCGTATCTCA ATGCTTCCCA
1321 GACTGTGTAT GTGAAGATGC TTATAACAAT ACCTATGCCT GTGTGAGGAC AATGTCAGCA
1381 TTGTGGAATT TGCAGTATTG CGAGTTTGAT GACCAGGAGG TGTTTGTAGA AGTCTATAAT
1441 CTGACTGCAG ACCCAGACCA GATCACTAAC ATTGCTAAAA CCATAGACCC AGAGCTTTTA
1501 GGAAAGATGA ACTATCGGTT AATGATGTTA CAGTCCTGTT CTGGGCCAAC CTGTCGCACT
1561 CCAGGGGTTT TTGACCCCGG ATACAGGTTT GACCCCCGTC TCATGTTCAG CAATCGCGGC
1621 AGTGTCAGGA CTCGAAGATT TTCCAAACAT CTTCTG
```

Human wild-type GNS coding sequence (SEQ ID NO: 4)

# FIG. 4

```
SEQ ID NO:3 1     ATGCGACTGCTGCCCCTGGCTCCCGGTAGACTGCGAAGAGGTTCACCACGACATCTGCCA  60
                  ||||| || ||||| || || || ||| | || ||  | ||    || || || |||||
SEQ ID NO:4 1     ATGCGGCTCCTGCCTCTAGCCCCAGGTCGGCTCCGGCGGGGCAGCCCCCGCCACCTGCCC  60

SEQ ID NO:3 61    AGTTGCTCCCCCGCCCTGCTGCTGCTGGTGCTGGGCGGATGCCTGGGAGTCTTTGGAGTG  120
                     |||  ||| || ||||| |||||||||||||||||| |||||||| ||||| || |||
SEQ ID NO:4 61    TCCTGCAGCCCAGCGCTGCTACTGCTGGTGCTGGGCGGCTGCCTGGGGGTCTTCGGGGTG  120

SEQ ID NO:3 121   GCAGCTGGCACACGGAGGCCAAACGTGGTCCTGCTGCTGACTGACGATCAGGACGAGGTG  180
                  || || || || ||||||||| |||||||| ||||| || || ||||| ||||||||| |||
SEQ ID NO:4 121   GCTGCGGGAACCCGGAGGCCCAACGTGGTGCTGCTCCTCACGGACGACCAGGACGAAGTG  180

SEQ ID NO:3 181   CTGGGGGGTATGACCCCCCTGAAGAAAACAAAGGCCCTGATCGGTGAAATGGGCATGACA  240
                  || || || ||||| || || |||||||| || || || ||||| || ||||| ||||| 
SEQ ID NO:4 181   CTCGGCGGCATGACACCGCTAAAGAAAACCAAAGCTCTCATCGGAGAGATGGGGATGACT  240

SEQ ID NO:3 241   TTCAGCTCCGCATACGTGCCTAGCGCCCTGTGCTGTCCAAGCCGGGCTTCCATTCTGACT  300
                  ||   |   || || ||||| || || || ||||| || ||| | ||    || ||||| 
SEQ ID NO:4 241   TTTTCCAGTGCTTATGTGCCAAGTGCTCTCTGCTGCCCCAGCAGAGCCAGTATCCTGACA  300

SEQ ID NO:3 301   GGGAAATATCCACACAATCACCATGTGGTCAACAATACCCTGGAGGGCAACTGCTCTAGT  360
                  || || || ||||| ||||| || || || ||||| || |||||||| ||||||  ||||
SEQ ID NO:4 301   GGAAAGTACCCACATAATCATCACGTTGTGAACAACACTCTGGAGGGGAACTGCAGTAGT  360

SEQ ID NO:3 361   AAGTCTTGGCAGAAAATCCAGGAACCTAATACATTTCCAGCCATTCTGAGGAGTATGTGC  420
                  ||||| |||||||| ||||| ||||| |||||| || ||||| ||||| ||     ||||| 
SEQ ID NO:4 361   AAGTCCTGGCAGAAGATCCAAGAACCAAATACTTTCCCAGCAATTCTCAGATCAATGTGT  420

SEQ ID NO:3 421   GGTTACCAGACTTTCTTTGCTGGCAAGTACCTGAACGAGTATGGAGCTCCAGATGCAGGA  480
                  ||||| ||||| || ||||| || || ||  | || ||||| ||||| ||||||||||| 
SEQ ID NO:4 421   GGTTATCAGACCTTTTTTGCAGGGAAATATTTAAATGAGTACGGAGCCCCAGATGCAGGT  480

SEQ ID NO:3 481   GGACTGGAACACGTGCCACTGGGATGGTCCTACTGGTATGCCCTGGAGAAGAACAGCAAA  540
                  ||||| ||||||||| || ||||| |||   ||||||||||||| |||| |||||    || 
SEQ ID NO:4 481   GGACTAGAACACGTTCCTCTGGGTTGGAGTTACTGGTATGCCTTGGAAAAGAATTCTAAG  540

SEQ ID NO:3 541   TACTATAACTACACCCTGTCTATCAATGGAAAGGCTAGAAAACATGGGGAAAACTACTCT  600
                  || ||||| ||||||||||||||||||||| |||||  | || ||||| ||||||||   |
SEQ ID NO:4 541   TATTATAATTACACCCTGTCTATCAATGGGAAGGCACGGAAGCATGGTGAAAACTATAGT  600

SEQ ID NO:3 601   GTGGACTATCTGACAGATGTCCTGGCAAATGTGAGTCTGGACTTTCTGGATTACAAGTCA  660
                  |||||||| |||||||||||  |||| |||||     ||||||||||||| |||||||| 
SEQ ID NO:4 601   GTGGACTACCTGACAGATGTTTTGGCTAATGTCTCCTTGGACTTTCTGGACTACAAGTCC  660

SEQ ID NO:3 661   AACTTCGAGCCCTTCTTTATGATGATTGCCACTCCAGCTCCTCACTCTCCCTGGACCGCA  720
                  ||||| ||||||||||| |||||||| |||||||||||| ||||| || || ||||| || 
SEQ ID NO:4 661   AACTTTGAGCCCTTCTTCATGATGATCGCCACTCCAGCGCCTCATTCGCCTTGGACAGCT  720

SEQ ID NO:3 721   GCACCACAGTATCAGAAGGCTTTCCAGAACGTGTTCGCACCTAGGAACAAAAATTTCAAC  780
                  ||||| ||||| ||||||||||||||||| || || ||||| || ||||| || ||||||
SEQ ID NO:4 721   GCACCTCAGTACCAGAAGGCTTTCCAGAATGTCTTTGCACCAAGAAACAAGAACTTCAAC  780

SEQ ID NO:3 781   ATCCACGGAACCAACAAGCATTGGCTGATTAGACAGGCAAAAACCCCAATGACAAATTCA  840
                  ||||| ||||| ||||||||| ||| | ||||| || || || || ||||||||| ||||| 
SEQ ID NO:4 781   ATCCATGGAACGAACAAGCACTGGTTAATTAGGCAAGCCAAGACTCCAATGACTAATTCT  840

SEQ ID NO:3 841   AGCATCCAGTTCCTGGACAACGCCTTTCGAAAGCGGTGGCAGACCCTGCTGAGCGTGGAC  900
                     || |||||  | || || || ||| | ||  ||||||| || || ||    || || 
SEQ ID NO:4 841   TCAATACAGTTTTTAGATAATGCATTTAGGAAAAGGTGGCAAACTCTCCTCTCAGTTGAT  900
```

**FIG. 5**

```
SEQ ID NO:3 901    GATCTGGTGGAGAAGCTGGTCAAACGCCTGGAGTTTACAGGGGAACTGAACAACACTTAC  960
                   || || |||||||| ||||||||  | |||||||| || ||||| || ||||||||||||
SEQ ID NO:4 901    GACCTTGTGGAGAAACTGGTCAAGAGGCTGGAGTTCACTGGGGAGCTCAACAACACTTAC  960

SEQ ID NO:3 961    ATTTTCTATACCAGCGATAACGGATACCATACTGGGCAGTTTTCCCTGCCCATCGACAAG  1020
                   || |||||||||   || || || || || || || ||||||||| |||| || ||||||
SEQ ID NO:4 961    ATCTTCTATACCTCAGACAATGGCTATCACACAGGACAGTTTTCCTTGCCAATAGACAAG  1020

SEQ ID NO:3 1021   CGACAGCTGTATGAGTTCGATATCAAGGTGCCCCTGCTGGTCCGGGGACCAGGGATCAAG  1080
                    |||||||||||||||| |||||||| || || ||| |||| || ||||| ||||||||
SEQ ID NO:4 1021   AGACAGCTGTATGAGTTTGATATCAAAGTTCCACTGTTGGTTCGAGGACCTGGGATCAAA  1080

SEQ ID NO:3 1081   CCTAATCAGACTAGTAAAATGCTGGTGGCTAACATTGACCTGGGGCCCACCATCCTGGAC  1140
                   || |||||||| || || |||||||| || ||||||||| |||| || || ||  |||||
SEQ ID NO:4 1081   CCAAATCAGACAAGCAAGATGCTGGTTGCCAACATTGACTTGGGTCCTACTATTTTGGAC  1140

SEQ ID NO:3 1141   ATTGCAGGTTACGATCTGAACAAGACACAGATGGATGGCATGTCCCTGCTGCCTATCCTG  1200
                   ||||| || ||||| || || ||||||||||||||||| |||||| |  |||| ||  ||
SEQ ID NO:4 1141   ATTGCTGGCTACGACCTAAATAAGACACAGATGGATGGGATGTCCTTATTGCCCATTTTG  1200

SEQ ID NO:3 1201   CGGGGAGCCTCAAATCTGACCTGGAGGAGCGACGTGCTGGTCGAGTATCAGGGTGAAGGC  1260
                    | || |||   ||  |||||||| |    || || ||||| || || || || ||||||
SEQ ID NO:4 1201   AGAGGTGCCAGTAACTTGACCTGGCGATCAGATGTCCTGGTGGAATACCAAGGAGAAGGC  1260

SEQ ID NO:3 1261   CGCAACGTGACTGATCCAACCTGCCCCTCCCTGTCTCCAGGCGTGTCACAGTGTTTTCCT  1320
                   || ||||| ||||| ||||| ||||| ||||||  ||| ||||| || || || || ||
SEQ ID NO:4 1261   CGTAACGTCACTGACCCAACATGCCCTTCCCTGAGTCCTGGCGTATCTCAATGCTTCCCA  1320

SEQ ID NO:3 1321   GACTGCGTCTGTGAGGATGCCTACAACAATACCTATGCTTGCGTGCGAACAATGAGCGCC  1380
                   ||||| || ||||| ||||| || |||||||||||||| || ||| | ||||||   ||
SEQ ID NO:4 1321   GACTGTGTATGTGAAGATGCTTATAACAATACCTATGCCTGTGTGAGGACAATGTCAGCA  1380

SEQ ID NO:3 1381   CTGTGGAACCTGCAGTACTGTGAGTTCGACGATCAGGAGGTGTTTGTCGAAGTGTATAAT  1440
                    |||||||  ||||||| || ||||| || || |||||||||||||| ||||| ||||||
SEQ ID NO:4 1381   TTGTGGAATTTGCAGTATTGCGAGTTTGATGACCAGGAGGTGTTTGTAGAAGTCTATAAT  1440

SEQ ID NO:3 1441   CTGACAGCAGACCCTGATCAGATCACAAACATTGCCAAGACTATCGACCCAGAGCTGCTG  1500
                   ||||| |||||||| || |||||||| |||||||| || || || |||||||||||  |
SEQ ID NO:4 1441   CTGACTGCAGACCCAGACCAGATCACTAACATTGCTAAAACCATAGACCCAGAGCTTTTA  1500

SEQ ID NO:3 1501   GGGAAAATGAATTACAGACTGATGATGCTGCAGAGTTGCTCAGGTCCTACATGTCGCACT  1560
                   || || ||||| ||  |  | |||||| | |||   || || || || || |||||||||
SEQ ID NO:4 1501   GGAAAGATGAACTATCGGTTAATGATGTTACAGTCCTGTTCTGGGCCAACCTGTCGCACT  1560

SEQ ID NO:3 1561   CCAGGCGTGTTCGACCCCGGCTATAGGTTTGATCCTAGACTGATGTTCTCTAACCGCGGA  1620
                   ||||| || || |||||||| || |||||||| ||  | || ||||||   || |||||
SEQ ID NO:4 1561   CCAGGGGTTTTTGACCCCGGATACAGGTTTGACCCCCGTCTCATGTTCAGCAATCGCGGC  1620

SEQ ID NO:3 1621   AGTGTCCGAACCAGACGCTTCTCCAAGCATCTGCTG  1656
                   |||||| | ||  || | || ||||| ||||| |||
SEQ ID NO:4 1621   AGTGTCAGGACTCGAAGATTTTCCAAACATCTTCTG  1656
```

# FIG. 5 (continued)

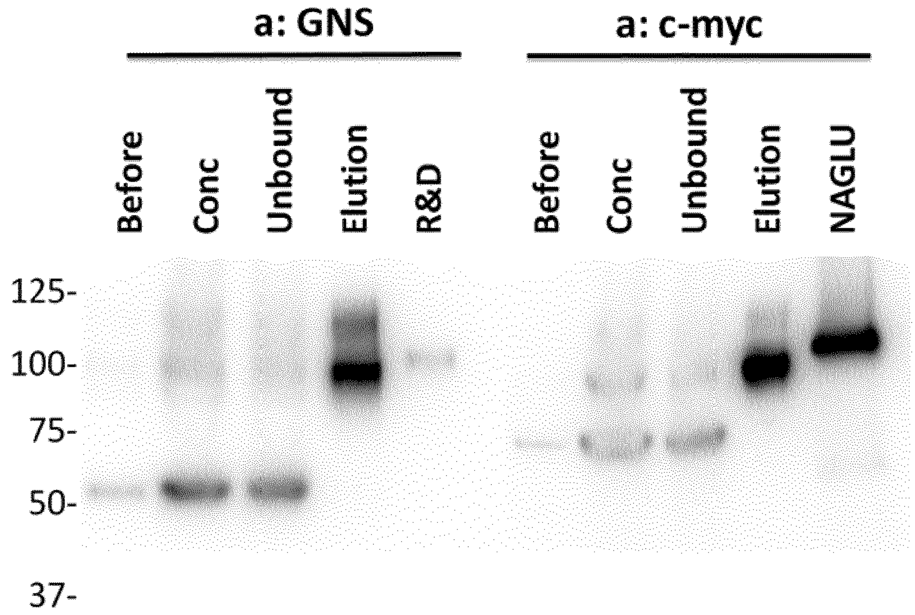
a: GNS
a: c-myc
Before
Conc
Unbound
Elution
R&D
Before
Conc
Unbound
Elution
NAGLU
125-
100-
75-
50-
37-

FIG. 6A

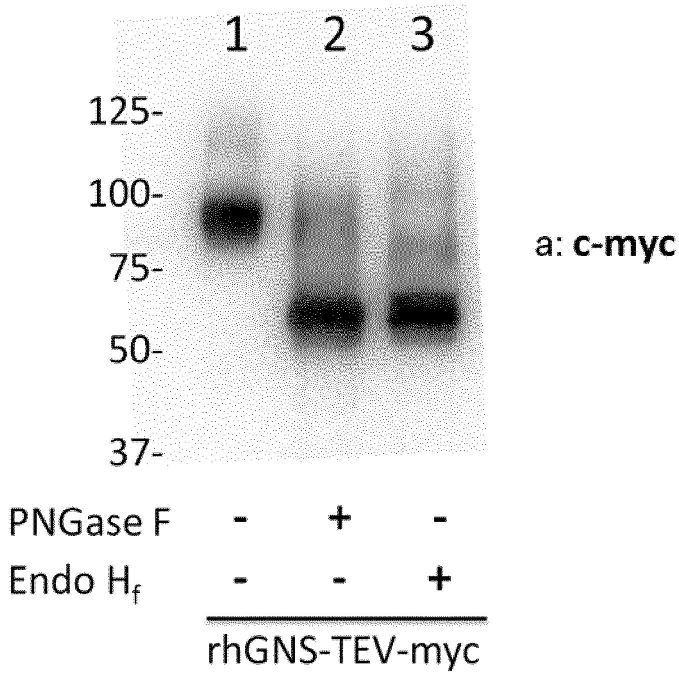
1
2
3
125-
100-
75-
50-
37-
a: c-myc
PNGase F - + -
Endo Hf - - +
rhGNS-TEV-myc

FIG. 6B

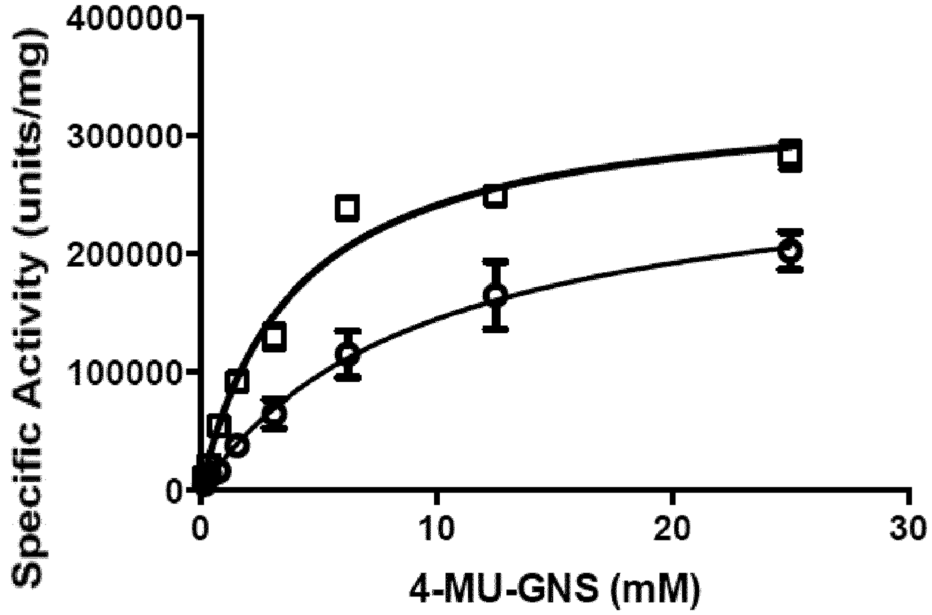
400000
300000
200000
100000
0
Specific Activity (units/mg)
0
10
20
30
4-MU-GNS (mM)

FIG. 6C

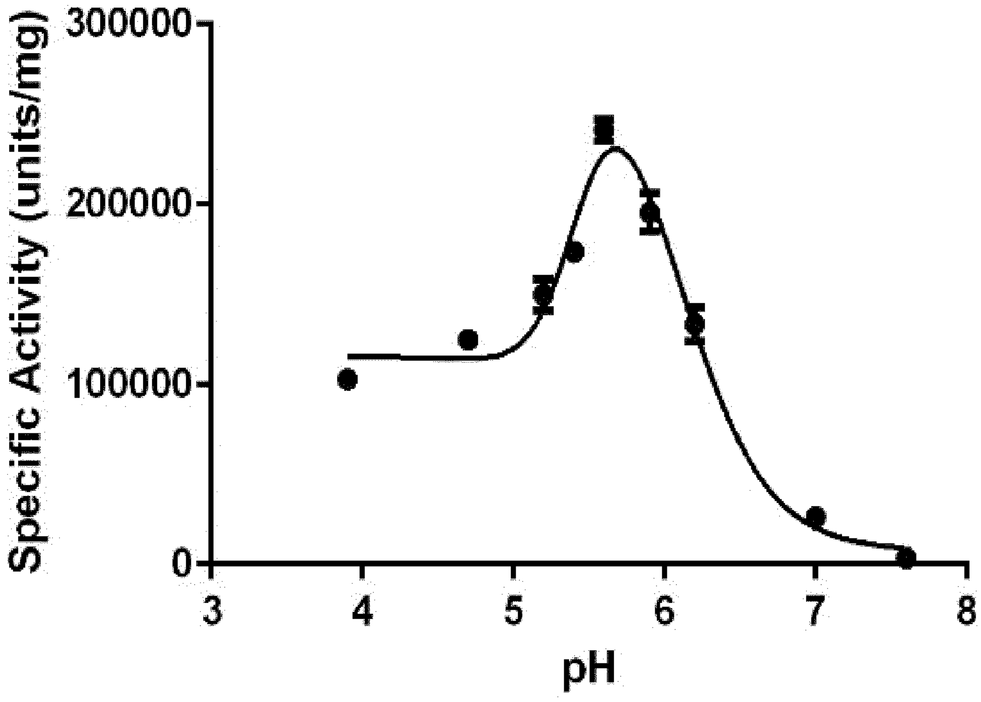
300000
200000
100000
0
Specific Activity (units/mg)
3
4
5
6
7
8
pH

FIG. 6D

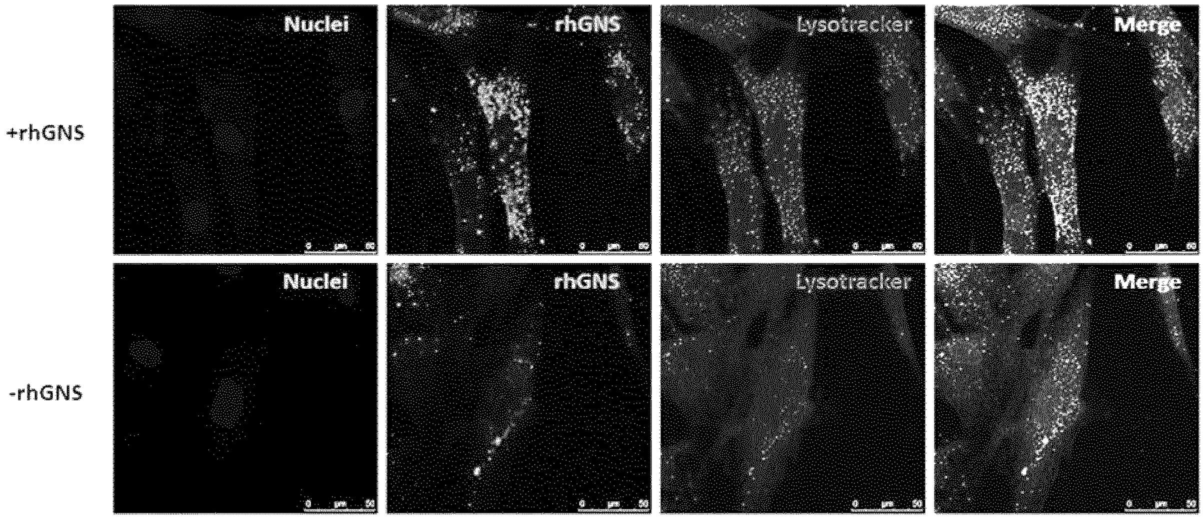
+rhGNS
-rhGNS
Nuclei
rhGNS
Lysotracker
Merge

FIG. 7A

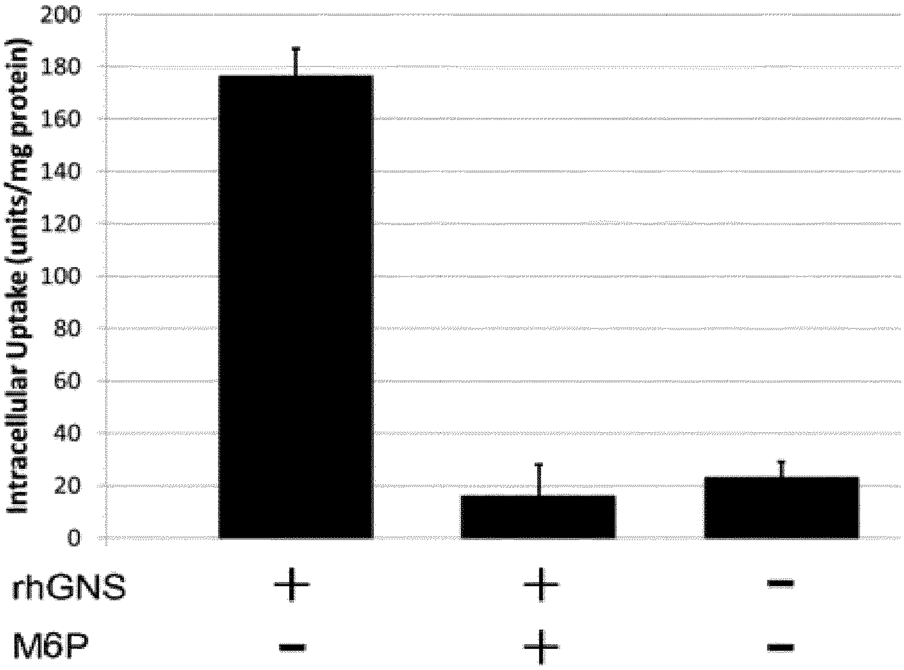
Intracellular Uptake (units/mg protein)
200
180
160
140
120
100
80
60
40
20
0
rhGNS + + −
M6P − + −

FIG. 7B

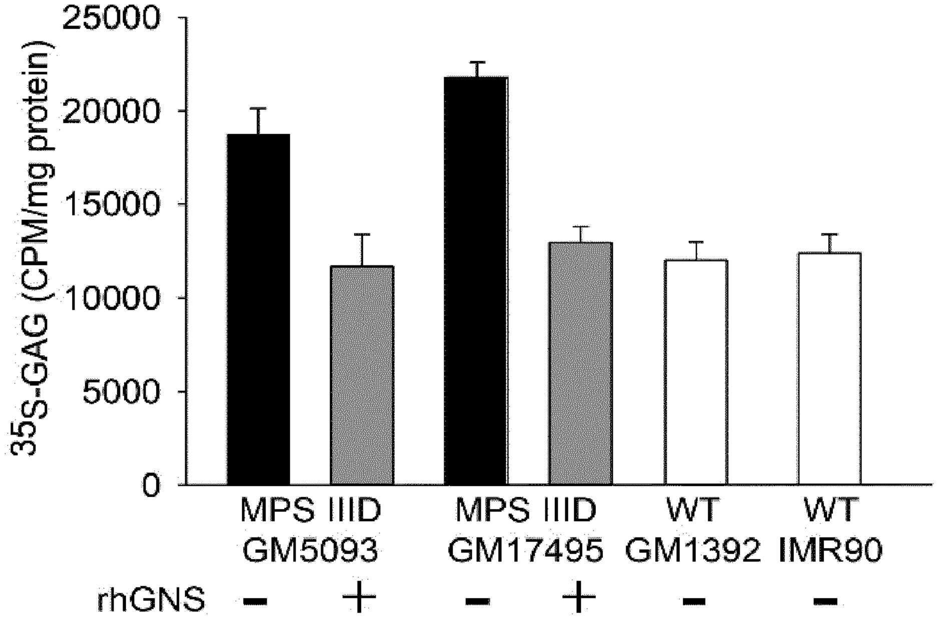
25000
20000
15000
10000
5000
0
35S-GAG (CPM/mg protein)
MPS IIID GM5093
MPS IIID GM17495
WT GM1392
WT IMR90
rhGNS - + - + - -

FIG. 7C

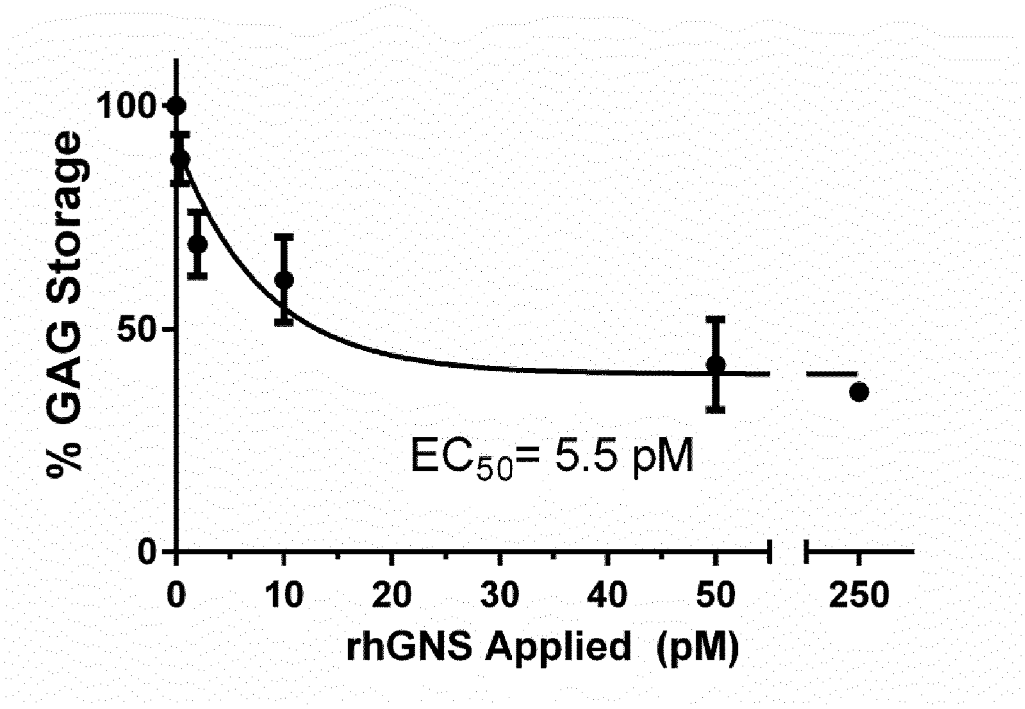
100
50
0
% GAG Storage
EC50= 5.5 pM
0 10 20 30 40 50 250
rhGNS Applied (pM)

FIG. 7D

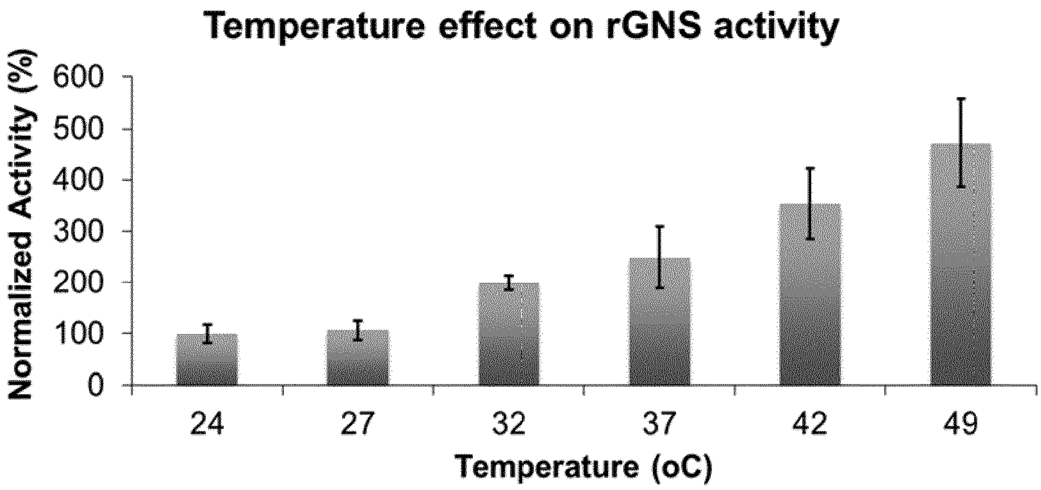
Temperature effect on rGNS activity
Normalized Activity (%)
600
500
400
300
200
100
0
24
27
32
37
42
49
Temperature (oC)

FIG. 8

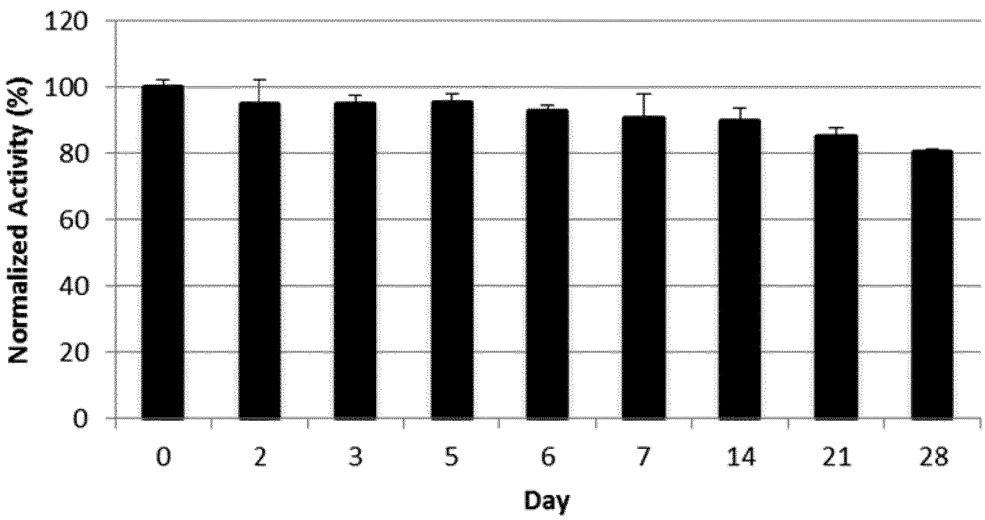
120
100
80
60
40
20
0
Normalized Activity (%)
0
2
3
5
6
7
14
21
28
Day

FIG. 9

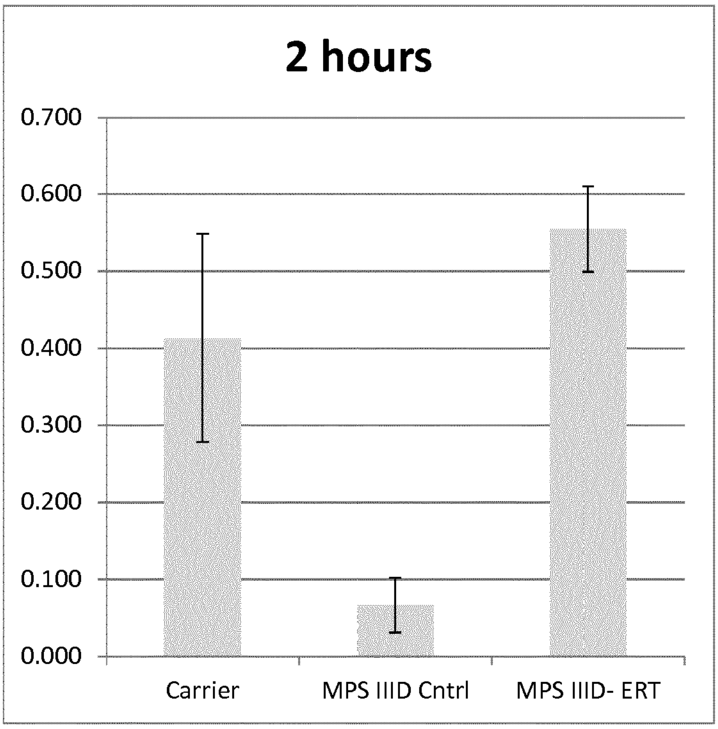


FIG. 10A

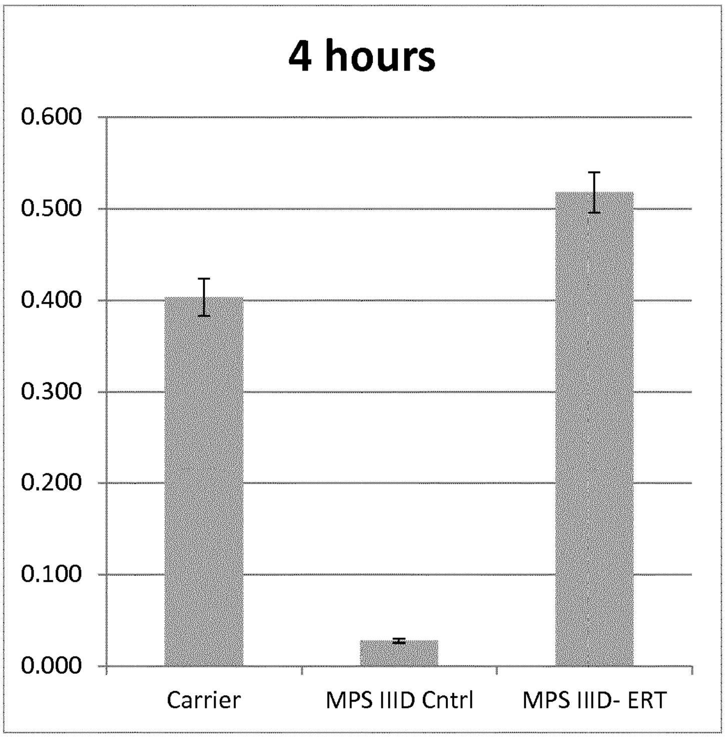


FIG. 10B

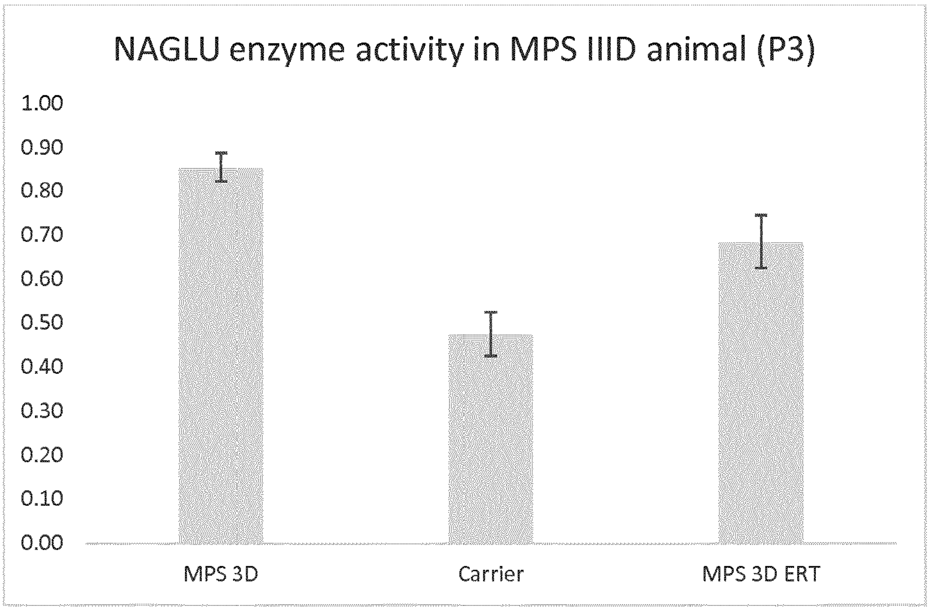


FIG. 11A

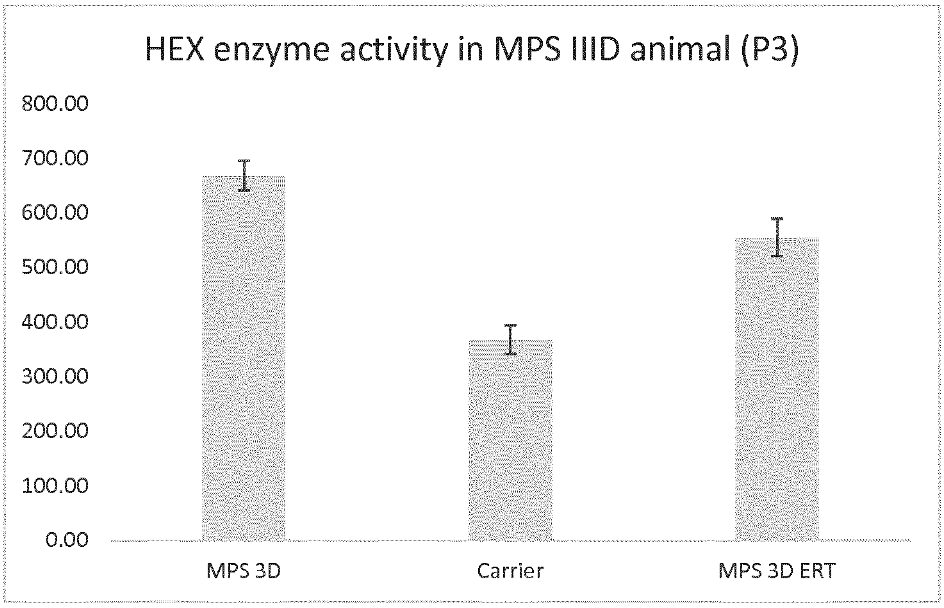


FIG. 11B

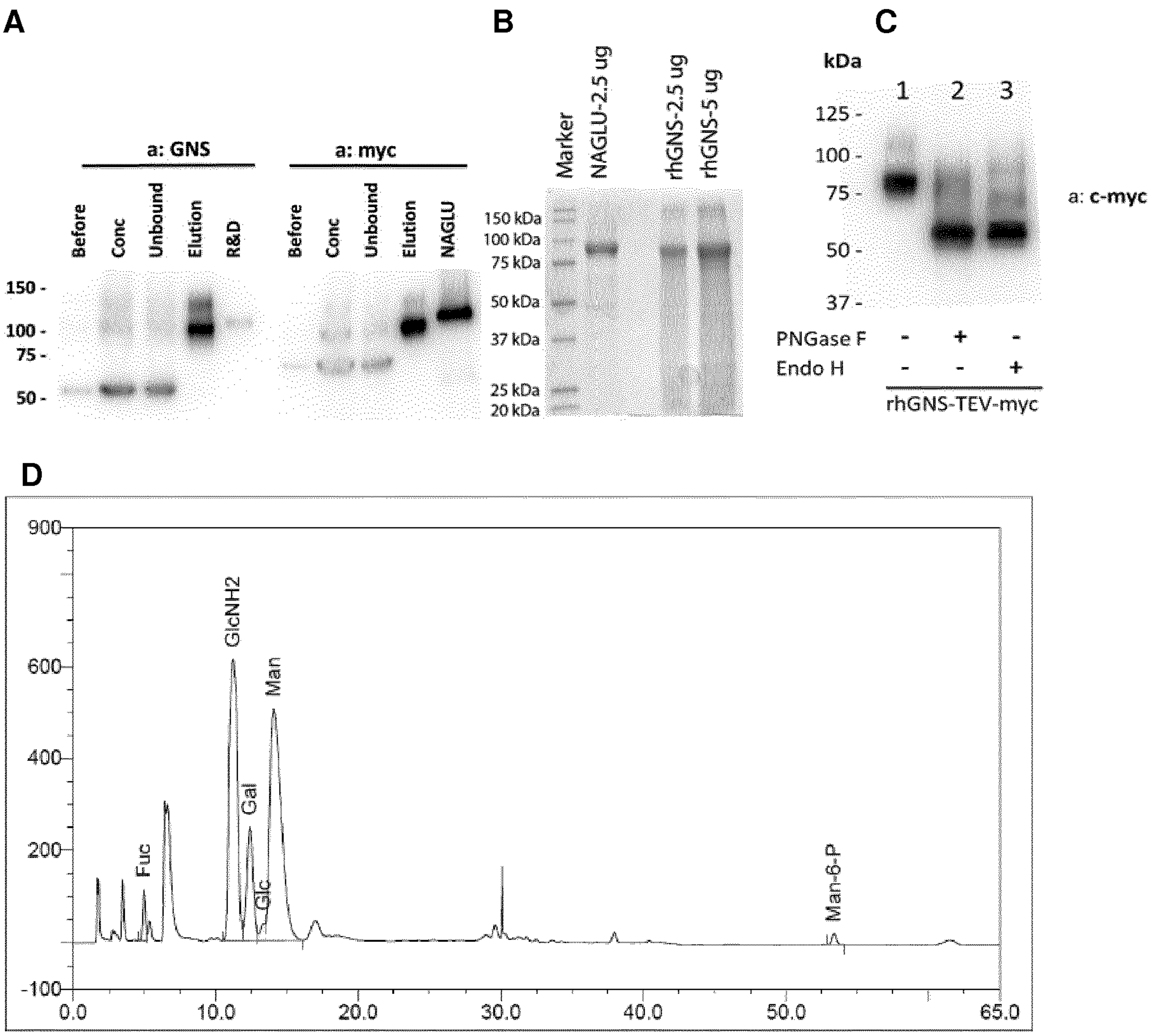
A
a: GNS
a: myc
Before
Conc
Unbound
Elution
R&D
Before
Conc
Unbound
Elution
NAGLU
150 -
100 -
75 -
50 -
B
Marker
NAGLU-2.5 ug
rhGNS-2.5 ug
rhGNS-5 ug
150 kDa
100 kDa
75 kDa
50 kDa
37 kDa
25 kDa
20 kDa
C
kDa
1
2
3
125 -
100 -
75 -
50 -
37 -
a: c-myc
PNGase F
Endo H
rhGNS-TEV-myc
D
900
600
400
200
-100
0.0
10.0
20.0
30.0
40.0
50.0
65.0
Fuc
GlcNH2
Gal
Glc
Man
Man-6-P

FIG. 12

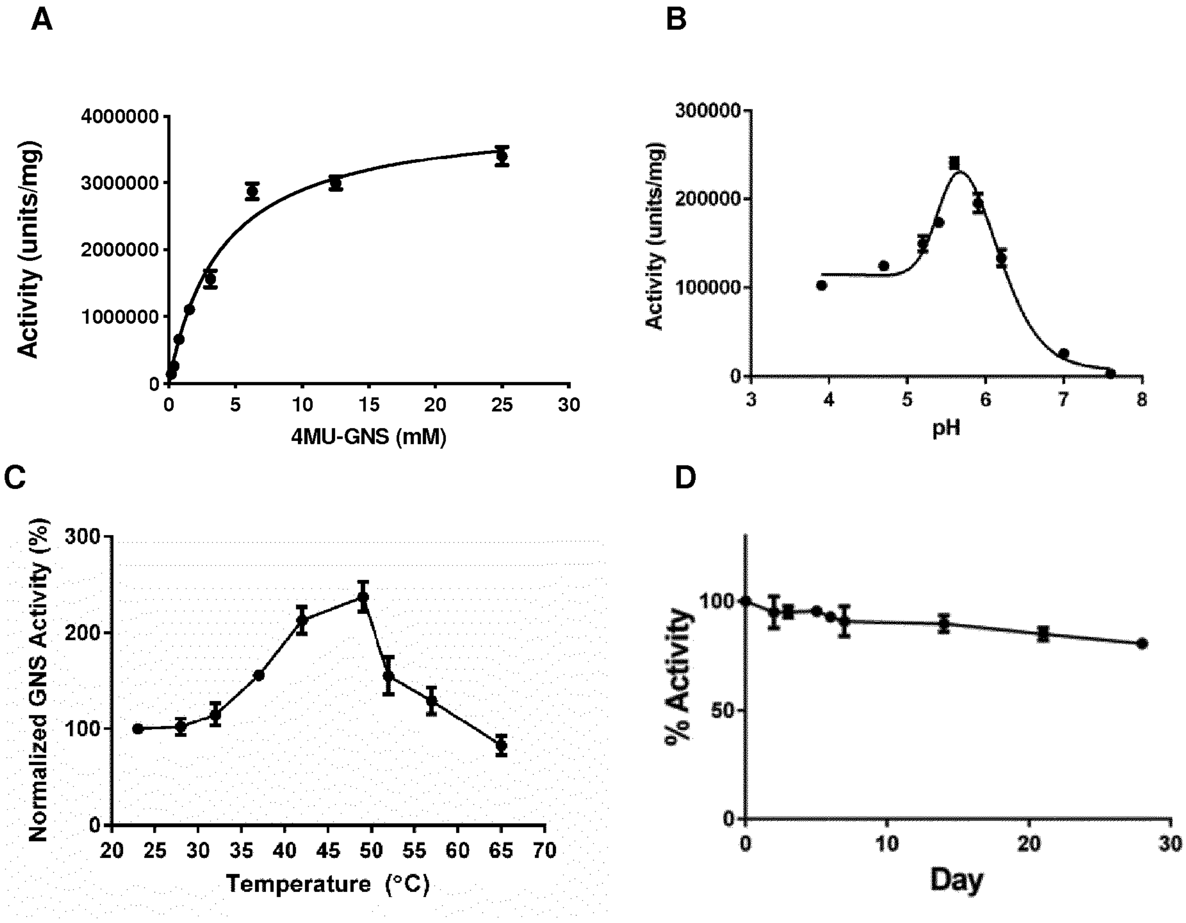
A
4000000
3000000
2000000
1000000
0
Activity (units/mg)
0 5 10 15 20 25 30
4MU-GNS (mM)
B
300000
200000
100000
0
Activity (units/mg)
3 4 5 6 7 8
pH
C
300
200
100
0
Normalized GNS Activity (%)
20 25 30 35 40 45 50 55 60 65 70
Temperature (°C)
D
100
50
0
% Activity
0 10 20 30
Day

FIG. 13

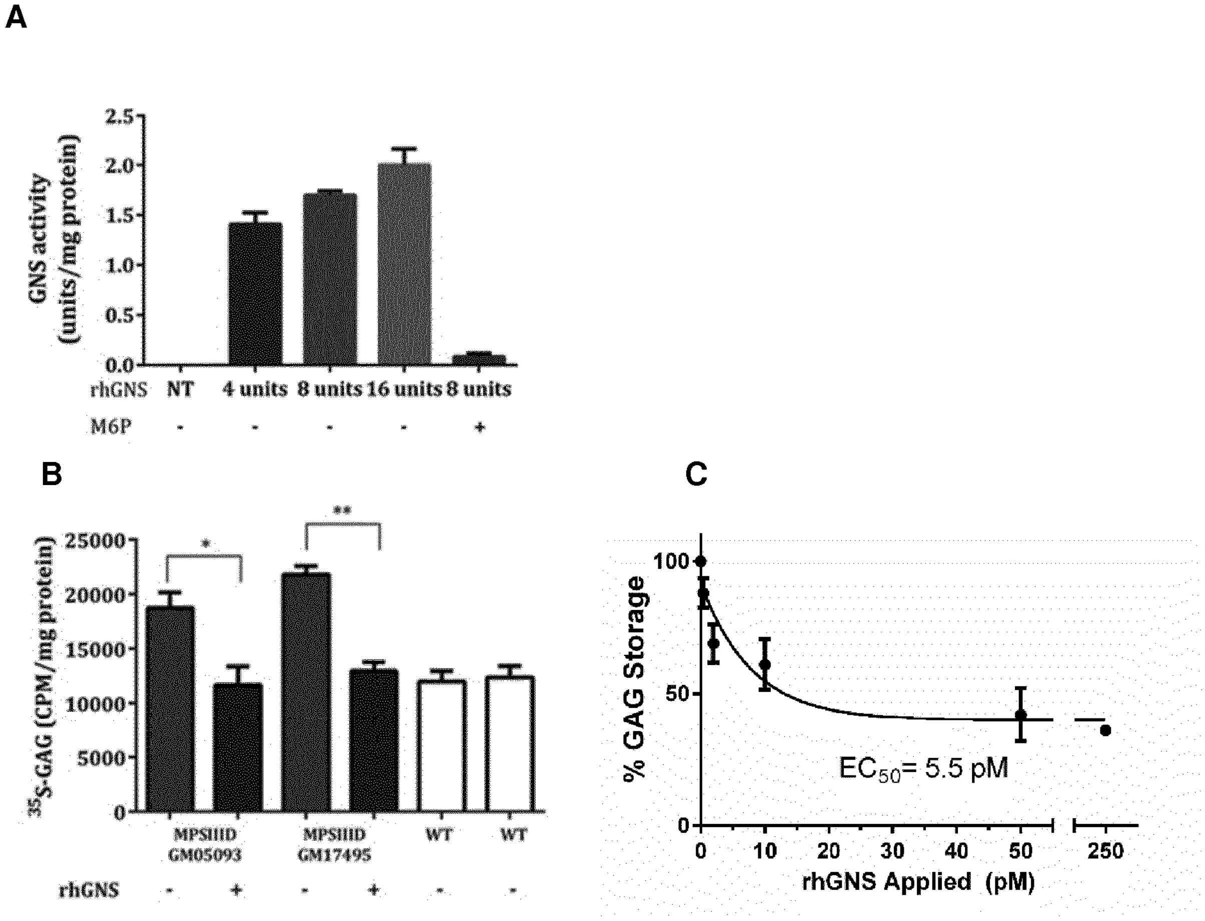
A
GNS activity
(units/mg protein)
2.5
2.0
1.5
1.0
0.5
0.0
rhGNS NT 4 units 8 units 16 units 8 units
M6P - - - - +
B
$^{35}$S-GAG (CPM/mg protein)
25000
20000
15000
10000
5000
0
*
**
MPSIIID GM05093
MPSIIID GM17495
WT
WT
rhGNS - + - + - -
C
% GAG Storage
100
50
0
EC$_{50}$= 5.5 pM
0 10 20 30 40 50 250
rhGNS Applied (pM)

FIG. 14

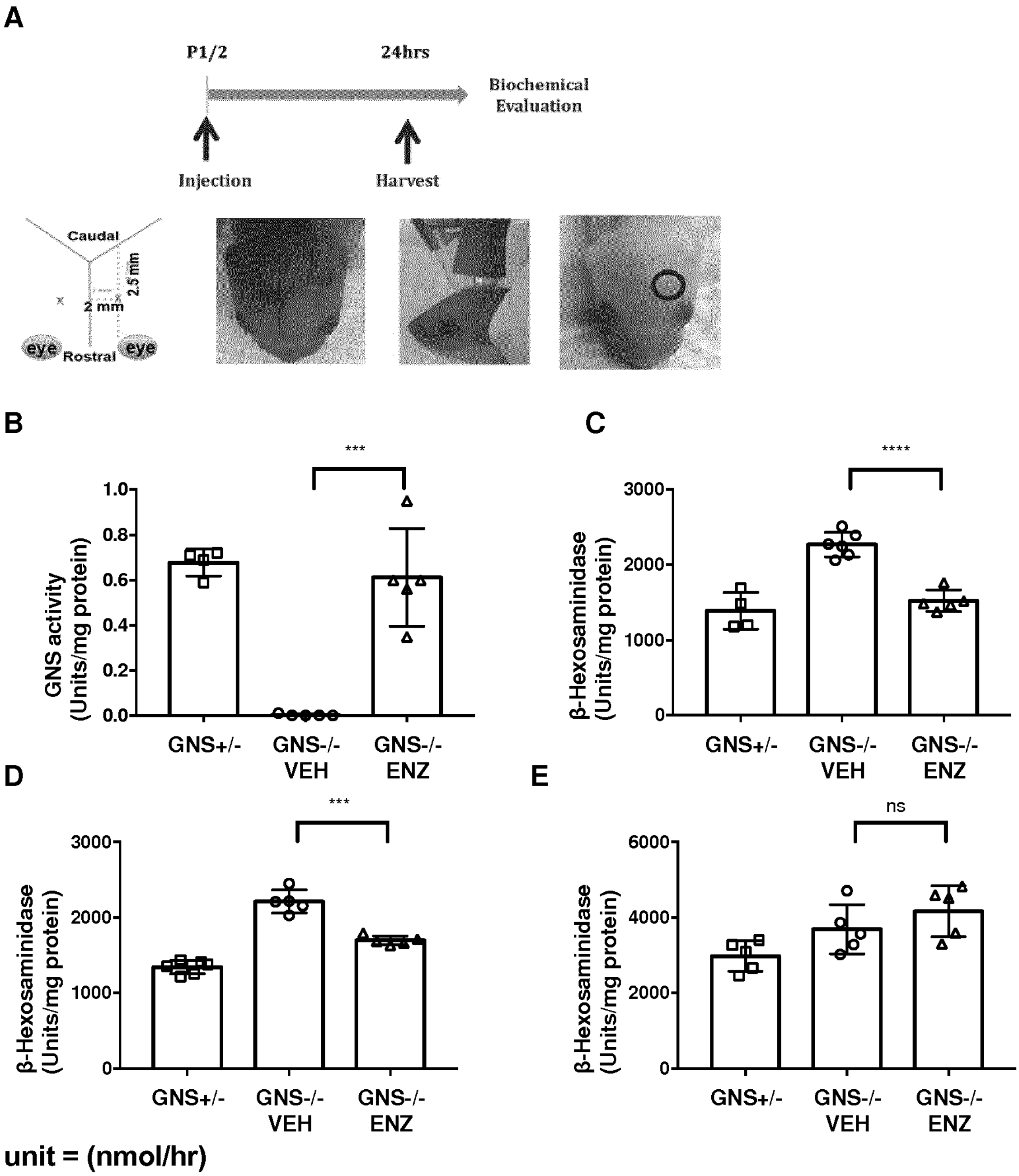
A
P1/2
24hrs
Biochemical Evaluation
Injection
Harvest
Caudal
2.5 mm
2 mm
eye
Rostral
eye
B
***
GNS activity (Units/mg protein)
1.0
0.8
0.6
0.4
0.2
0.0
GNS+/-
GNS-/- VEH
GNS-/- ENZ
C
****
β-Hexosaminidase (Units/mg protein)
3000
2000
1000
0
GNS+/-
GNS-/- VEH
GNS-/- ENZ
D
***
β-Hexosaminidase (Units/mg protein)
3000
2000
1000
0
GNS+/-
GNS-/- VEH
GNS-/- ENZ
E
ns
β-Hexosaminidase (Units/mg protein)
6000
4000
2000
0
GNS+/-
GNS-/- VEH
GNS-/- ENZ
unit = (nmol/hr)

**FIG. 15**

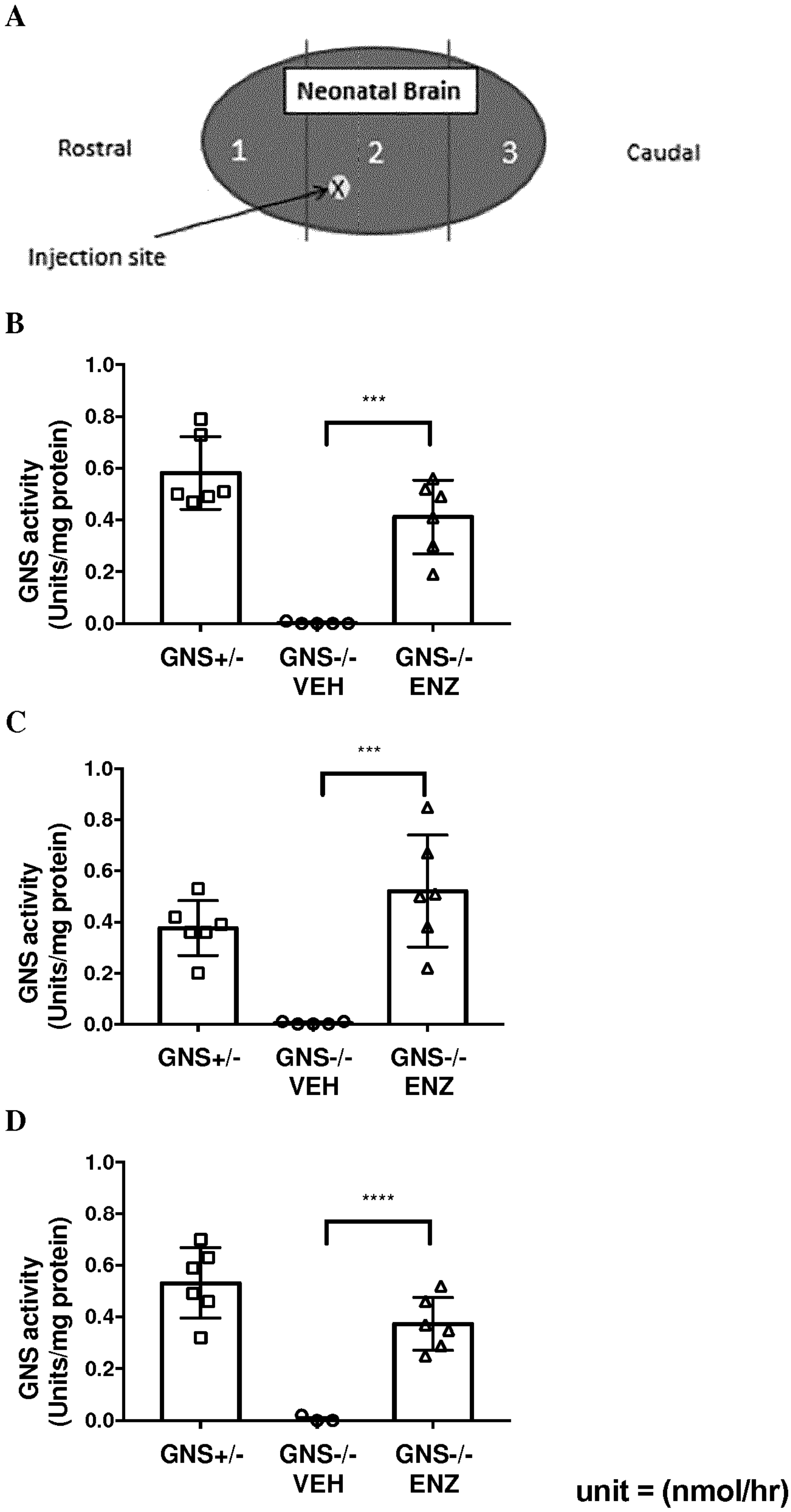
A
Neonatal Brain
Rostral
1
2
3
Caudal
Injection site
B
GNS activity
(Units/mg protein)
***
GNS+/-
GNS-/-
VEH
GNS-/-
ENZ
C
GNS activity
(Units/mg protein)
***
GNS+/-
GNS-/-
VEH
GNS-/-
ENZ
D
GNS activity
(Units/mg protein)
****
GNS+/-
GNS-/-
VEH
GNS-/-
ENZ
unit = (nmol/hr)

FIG. 16

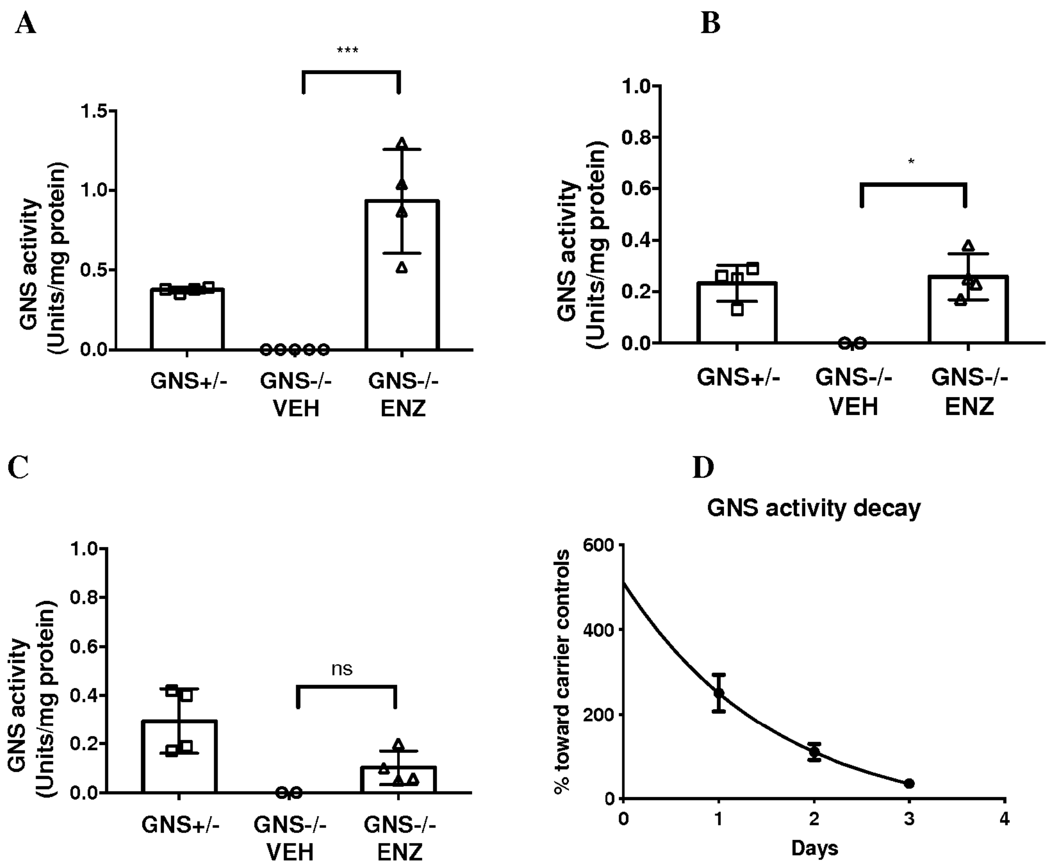


unit = (nmol/hr)

**FIG. 17**

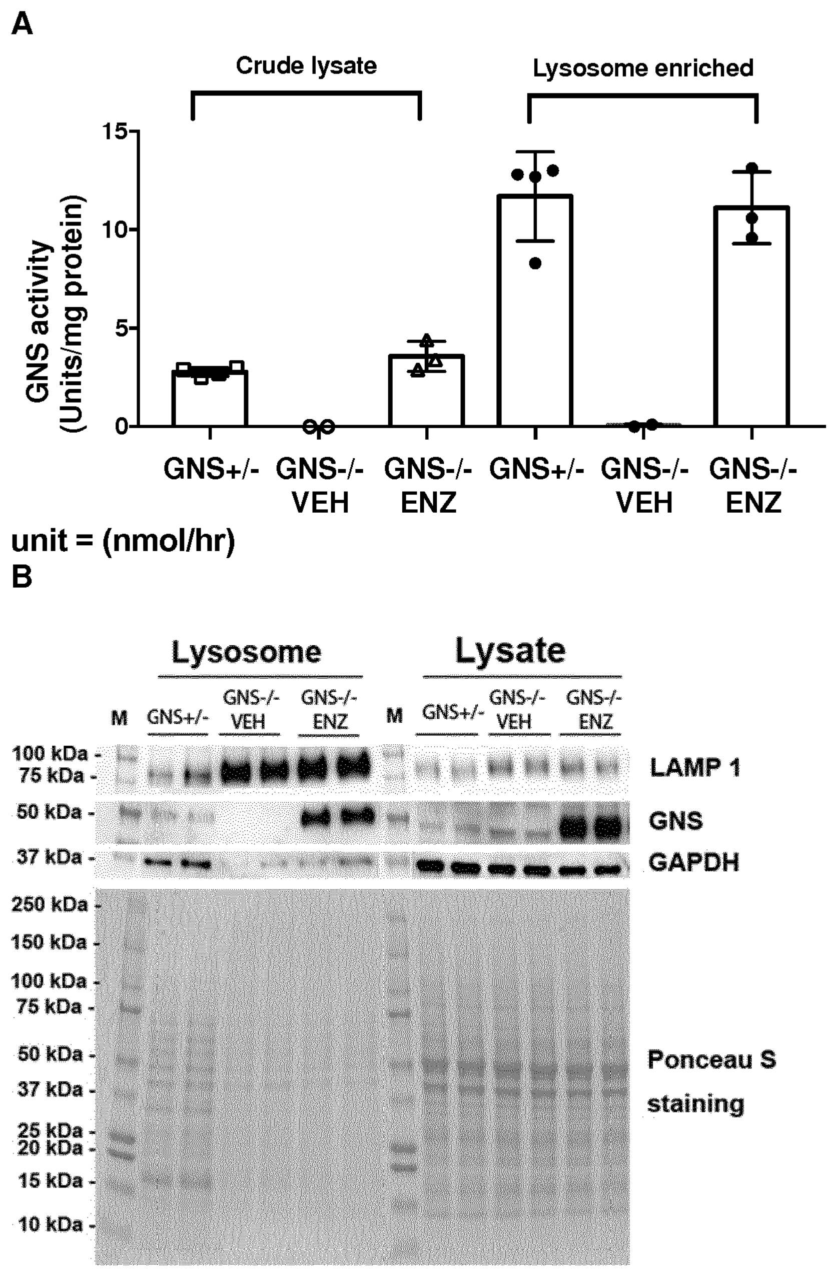
A
Crude lysate
Lysosome enriched
GNS activity
(Units/mg protein)
15
10
5
0
GNS+/-
GNS-/- VEH
GNS-/- ENZ
GNS+/-
GNS-/- VEH
GNS-/- ENZ
unit = (nmol/hr)
B
Lysosome
Lysate
M
GNS+/-
GNS-/- VEH
GNS-/- ENZ
100 kDa
75 kDa
50 kDa
37 kDa
250 kDa
150 kDa
25 kDa
20 kDa
15 kDa
10 kDa
LAMP 1
GNS
GAPDH
Ponceau S staining

FIG. 18

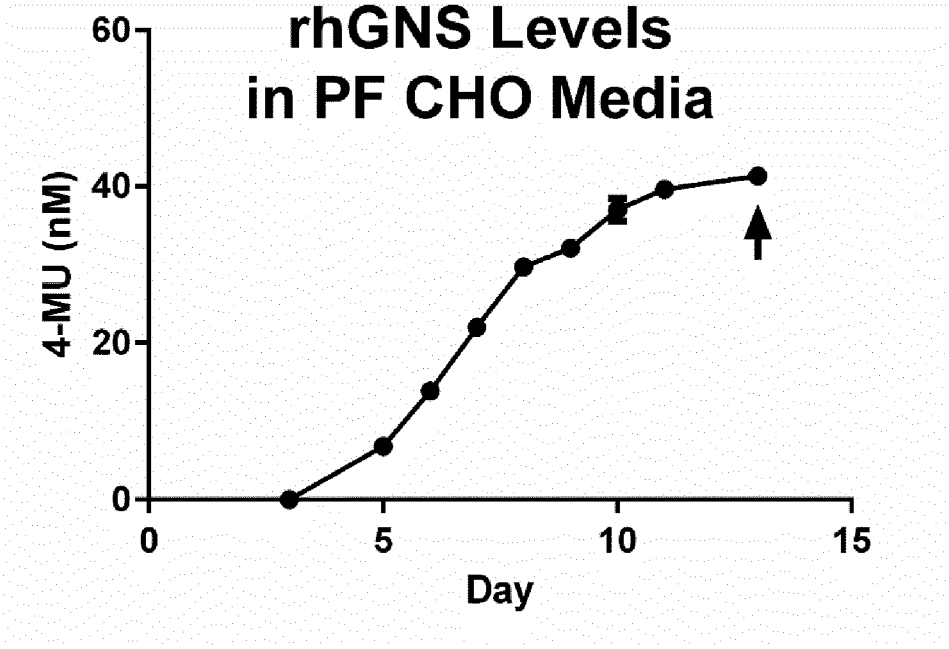
rhGNS Levels
in PF CHO Media
60
40
20
0
4-MU (nM)
0
5
10
15
Day

FIG. 19

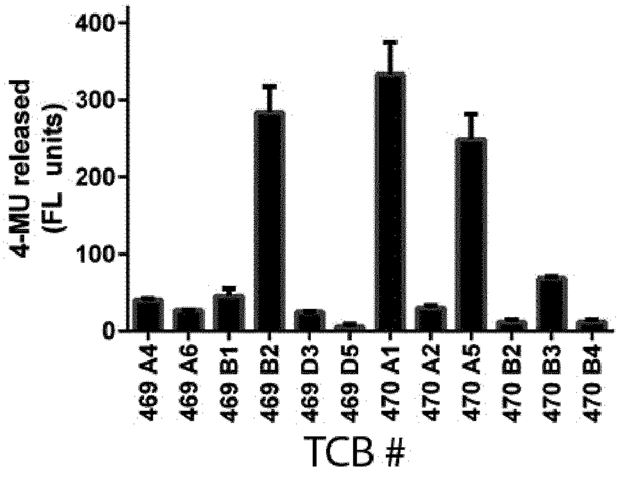
400
300
200
100
0
4-MU released
(FL units)
469 A4
469 A6
469 B1
469 B2
469 D3
469 D5
470 A1
470 A2
470 A5
470 B2
470 B3
470 B4
TCB #

FIG. 20

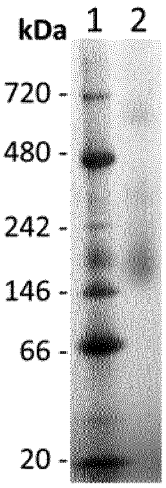
kDa
1
2
720 -
480 -
242 -
146 -
66 -
20 -

FIG. 21

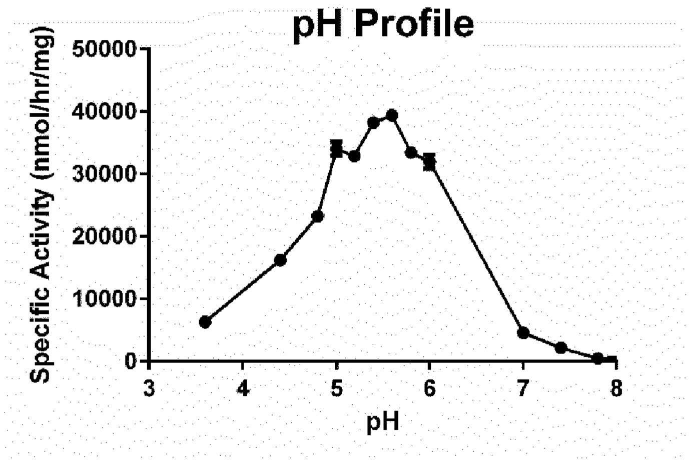
pH Profile
Specific Activity (nmol/hr/mg)
50000
40000
30000
20000
10000
0
3
4
5
6
7
8
pH

FIG. 22

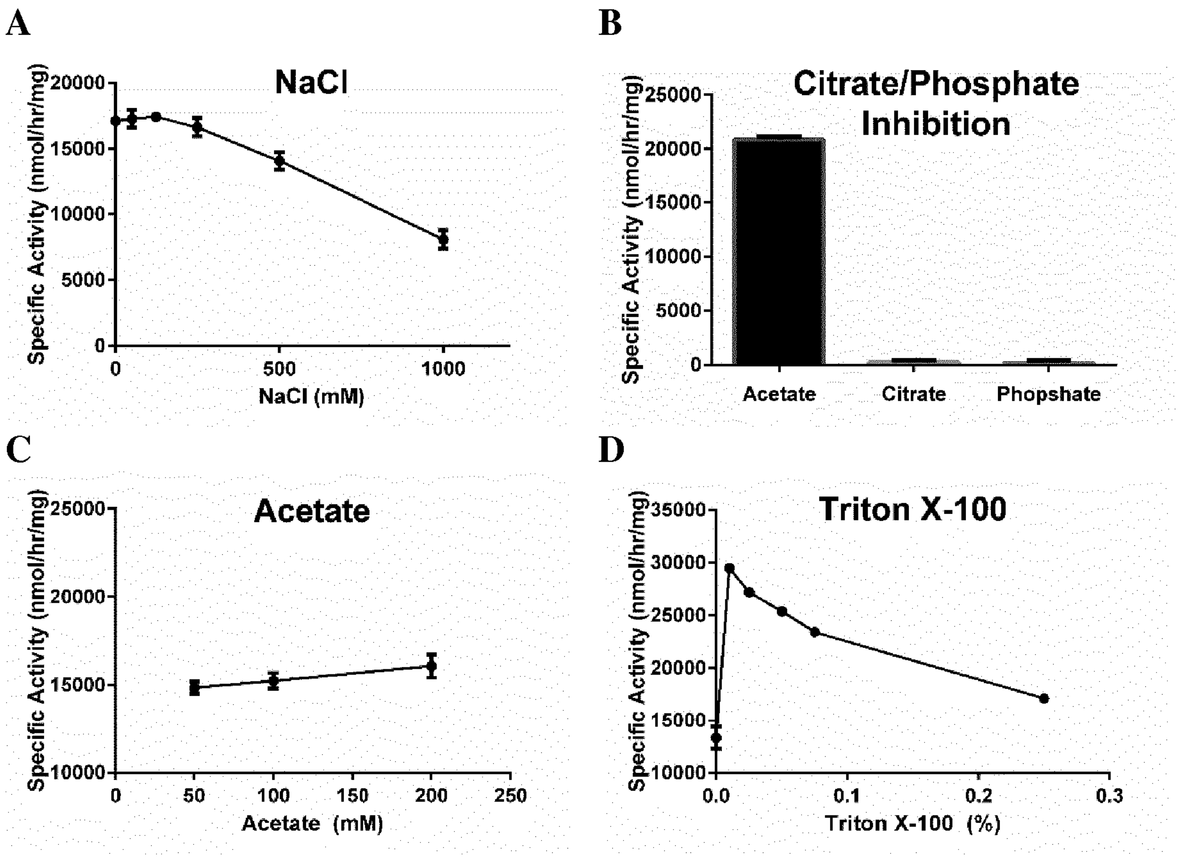
A
NaCl
Specific Activity (nmol/hr/mg)
20000
15000
10000
5000
0
0
500
1000
NaCl (mM)
B
Citrate/Phosphate Inhibition
Specific Activity (nmol/hr/mg)
25000
20000
15000
10000
5000
0
Acetate
Citrate
Phopshate
C
Acetate
Specific Activity (nmol/hr/mg)
25000
20000
15000
10000
0
50
100
150
200
250
Acetate (mM)
D
Triton X-100
Specific Activity (nmol/hr/mg)
35000
30000
25000
20000
15000
10000
0.0
0.1
0.2
0.3
Triton X-100 (%)

FIG. 23

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5440013 A, Kahn
- US 4683195 A
- US 4800159 A
- US 4754065 A
- US 4683202 A
- US 4812405 A
- US 4818700 A
- US 4929555 A
- US 5736383 A
- US 5955349 A
- US 5888768 A
- US 6258559 B
- US 62611472 B
- US 15946505 B


### Non-patent literature cited in the description


- **HRUBY.** *Life Sciences,* 1982, vol. 31, 189-199
- **HRUBY et al.** *Biochem J.,* 1990, vol. 268, 249-262
- **KAZMIERSKI et al.** *J. Am. Chem. Soc.,* 1991, vol. 113, 2275-2283
- **KAZMIERSKI ; HRUBY.** *Tetrahedron Lett.,* 1991, vol. 32 (41), 5769-5772
- **LANDIS.** Ph.D. Thesis. University of Arizona, 1989
- **MIYAKE et al.** *J. Takeda Res. Labs.,* 1989, vol. 43, 53-76
- **ZECHEL et al.** *Int. J. Pep. Protein Res.,* 1991, vol. 38 (2), 131-138
- **DHARANIPRAGADA et al.** *Int. J. Pep. Protein Res.,* 1993, vol. 42 (1), 68-77
- **DHARANIPRAGADA et al.** *Acta. Crystallogr. C.,* 1992, vol. 48, 1239-1241
- **KEMP et al.** *J. Org. Chem.,* 1985, vol. 50, 5834-5838
- **KEMP et al.** *Tetrahedron Lett.,* 1988, vol. 29, 5081-5082
- **KEMP et al.** *Tetrahedron Lett.,* 1988, vol. 29, 5057-5060
- **KEMP et al.** *Tetrahedron Lett.,* 1988, vol. 29, 4935-4938
- **KEMP et al.** *J. Org. Chem.,* 1989, vol. 54 (109), 115
- **NAGAI ; SATO.** *Tetrahedron Lett.,* 1985, vol. 26, 647-650
- **DIMAIO et al.** *J. Chem. Soc. Perkin Trans.,* 1989, 1687
- **KAHN et al.** *Tetrahedron Lett.,* 1989, vol. 30, 2317
- **CLONES et al.** *Tetrahedron Lett.,* 1988, vol. 29, 3853-3856
- **ZABROCKI et al.** *J. Am. Chem. Soc.,* 1988, vol. 110, 5875-5880
- **SAMANEN et al.** *Int. J. Protein Pep. Res.,* 1990, vol. 35 (501), 509
- **OLSON et al.** *J. Am. Chem. Sci.,* 1990, vol. 112, 323-333
- **GARVEY et al.** *J. Org. Chem.,* 1990, vol. 56, 436
- **DAHOFF et al.** Atlas of Protein Sequence and Structure. 1978, vol. 5, 345-352
- **JONES et al.** *Comput. Appl. Biosci.,* 1992, vol. 8, 275-282
- **GONNET et al.** *Science,* 1992, vol. 256, 1443-1145
- **ADACH ; HASEGAWA.** *J. Mol. Evol.,* 1996, vol. 42, 459-468
- **HENIKOFF ; HENIKOFF.** *Proc. Natl. Acad. Sci. USA,* 1992, vol. 89, 1
- **NEI.** Molecular Evolutionary Genetics. Columbia University Press, 1987
- **MÜLLER et al.** *Mol. Biol. Evol.,* 2002, vol. 19, 8-13
- PCR: The Polymerase Chain Reaction. Birkhauser Press, 1994
- Remington's Pharmaceutical Sciences. Mack Publishing Company